(19) Europäisches Patentamt
European Patent Office
Office européen des brevets

(11) Veröffentlichungsnummer: **0 294 667 B1**

# (12) EUROPÄISCHE PATENTSCHRIFT

(45) Veröffentlichungstag der Patentschrift: **17.03.93**

(51) Int. Cl.[5]: **C07C 251/32**, C07D 207/10, C07D 209/00, C07D 211/16, C07D 213/75, C07D 215/08, C07D 239/42, C07D 249/14, C07D 263/52, C07D 265/30, C07D 277/46

(21) Anmeldenummer: **88108490.9**

(22) Anmeldetag: **27.05.88**

Die Akte enthält technische Angaben, die nach dem Eingang der Anmeldung eingereicht wurden und die nicht in dieser Patentschrift enthalten sind.

## (54) (2-Cyan-2-oximinoacetyl)-aminosäure-Derivate.

(30) Priorität: **09.06.87 DE 3719227**

(43) Veröffentlichungstag der Anmeldung:
**14.12.88 Patentblatt 88/50**

(45) Bekanntmachung des Hinweises auf die Patenterteilung:
**17.03.93 Patentblatt 93/11**

(84) Benannte Vertragsstaaten:
**AT BE CH DE ES FR GB GR IT LI NL**

(56) Entgegenhaltungen:
**EP-A- 0 201 999**
**DE-A- 3 602 243**

(73) Patentinhaber: **BAYER AG**

**W-5090 Leverkusen 1 Bayerwerk(DE)**

(72) Erfinder: **Lunkenheimer, Winfried, Dr.**
**Bismarckstrasse 29**
**W-5600 Wuppertal 1(DE)**
Erfinder: **Brandes, Wilhelm, Dr.**
**Eichendorffstrasse 3**
**W-5653 Leichlingen(DE)**
Erfinder: **Hänssler, Gerd, Dr.**
**Am Arenzberg 58a**
**W-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 294 667 B1

## Beschreibung

Die vorliegende Erfindung betrifft neue (2-Cyan-2-oximino-acetyl)-aminosäure-Derivate, mehrere Verfahren zu ihrer Herstellung und ihre Verwendung als Schädlingsbekämpfungsmittel, insbesondere als Fungizide.

Es ist bereits bekannt, daß bestimmte substituierte 2-Cyan-2-methoximino-acetamide gute fungizide Wirksamkeit besitzen (vgl. z.B. EP-OS 0 201 999 und DE-OS 3 602 243). Die Wirkung dieser Verbindungen ist jedoch, insbesondere bei niedrigen Aufwandmengen und -konzentrationen, nicht immer voll befriedigend.

Es wurden neue (2-Cyan-2-oximinoacetyl)-aminosäure-Derivate der allgemeinen Formel (I),

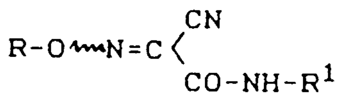

(I)

in welcher

R — für Alkyl, Cyanalkyl oder jeweils gegebenenfalls substituiertes Alkenyl, Alkinyl, Cycloalkyl, Cycloalkylalkyl oder Phenylalkyl steht,

$R^1$ — für die Gruppierungen $-B-CONR^3R^4$ steht, wobei

B — für eine gegebenenfalls substituierte, geradkettige oder verzweigte Alkylenkette steht,

$R^3$ — für substituiertes Alkyl steht mit folgenden Substituenten : Halogen, Cyano, $-COOR^I$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht und

$R^4$ — für substituiertes Alkyl steht mit folgenden Substituenten: Halogen, Cyano, $-COOR^I$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,

oder

$R^3$ — für Wasserstoff oder Alkyl steht und

$R^4$ — für substituiertes Alkyl steht mit den folgenden Substituenten: Acyl, Hydroxy, Alkylcarbonyloxy, $-S(O)_nR^{IV}$, jeweils gegebenenfalls substituiertes Heterocyclyl, Cycloalkyl und Cycloalkenyl; $R^4$ ferner für jeweils gegebenenfalls substituiertes Cycloalkenyl, Heterocyclyl oder polycyclische Carbocyclen, für substituiertes Cycloalkyl, wobei jedoch Halogen und Alkyl nicht als alleinige Substituenten vorhanden sein dürfen, oder für die Gruppierung $-OR^{IV}$ steht,

oder

$R^3$ und $R^4$ — gemeinsam mit dem Stickstoffatom, an das sie gebunden sind, für einen substituierten monocyclischen Heterocyclus oder für einen gegebenenfalls substituierten bi- oder tricyclischen Heterocyclus oder Spiroheterocyclus mit 1 bis 3 weiteren, gleichen oder verschiedenen Heteroatomen, wie Sauerstoff-, Schwefel- oder Stickstoffatome, stehen,

$R^I$ — für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ — gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ — für Wasserstoff, Alkyl, Aralkyl oder Acyl steht und

n — für die Zahlen 0, 1 oder 2 steht,

gefunden.

Die Verbindungen der Formel (I) können in verschiedenen geometrischen Isomeren vorkommmen, je nach Anordnung der Substituenten an der C = N-Gruppierung (E- bzw. Z-Isomere).

Gegebenenfalls besitzen die Verbindungen ein oder mehrere asymmetrische Kohlenstoffatomen; sie können somit auch als optische Isomere vorliegen (D- und L-Konfiguration), die in unterschiedlichen

Mengenverhältnissen anfallen können. Vorwiegend fallen sie als Racemate an.

Weiterhin wurde gefunden, daß man die neuen (2-Cyan-2-oximinoacetyl)-aminosäure-Derivate der allgemeinen Formel (I),

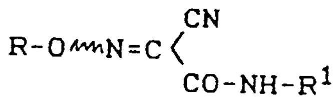

(I)

in welcher

R für Alkyl, Cyanalkyl oder jeweils gegebenenfalls substituiertes Alkenyl, Alkinyl, Cycloalkyl, Cycloalkylalkyl oder Phenylalkyl steht,

$R^1$ für die Gruppierungen -B-CON$R^3R^4$ steht, wobei

B für eine gegebenenfalls substituierte, geradkettige oder verzweigte Alkylenkette steht,

$R^3$ für substituiertes Alkyl steht mit folgenden Substituenten : Halogen, Cyano, -COO$R^{I}$, -CON$R^{II}R^{III}$, -N$R^{II}R^{III}$, -O$R^{IV}$, -S(O)$_n$$R^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht, und

$R^4$ für substituiertes Alkyl steht mit folgenden Substituenten: Halogen, Cyano, -COO$R^{I}$, -CON$R^{II}R^{III}$, -N$R^{II}R^{III}$, -O$R^{IV}$, -S(O)$_n$$R^{IV}$,Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung -O$R^{IV}$ steht,

oder

$R^3$ für Wasserstoff oder Alkyl steht und

$R^4$ für substituiertes Alkyl steht mit den folgenden Substituenten: Acyl, Hydroxy, Alkylcarbonyloxy, -S(O)$_n$$R^{IV}$, jeweils gegebenenfalls substituiertes Heterocyclyl, Cycloalkyl und Cycloalkenyl; $R^4$ ferner für jeweils gegebenenfalls substituiertes Cycloalkenyl, Heterocyclyl oder polycyclische Carbocyclen, für substituiertes Cycloalkyl, wobei jedoch Halogen und Alkyl nicht als alleinige Substituenten vorhanden sein dürfen, oder für die Gruppierung -O$R^{IV}$ steht,

oder

$R^3$ und $R^4$ gemeinsam mit dem Stickstoffatom, an das sie gebunden sind, für einen substituierten monocyclischen Heterocyclus oder für einen gegebenenfalls substituierten bi- oder tricyclischen Heterocyclus oder Spiroheterocyclus mit 1 bis 3 weiteren, gleichen oder verschiedenen Heteroatomen, wie Sauerstoff-, Schwefel-oder Stickstoffatome, stehen,

$R^{I}$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ gleich oder verschieden sind und für Wasserstoff oder gegebenenfalls substituiertes Alkyl stehen mit folgenden Substituenten: Halogen, -COO$R^{IV}$, -CON$R^{I}R^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ für Wasserstoff, Alkyl, Aralkyl oder Acyl steht und

n für die Zahlen 0, 1 oder 2 steht,

erhält, wenn man

a) 2-Cyan-2-oximino-essigsäureester der allgemeinen Formel (II),

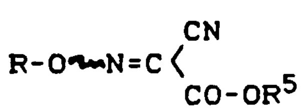

(II)

in welcher

R die oben angegebene Bedeutung hat und
$R^5$ für Alkyl steht,
mit Aminen der Formel (III),

$R^1-NH_2$ (III)

in welcher
$R^1$ die oben angegebene Bedeutung hat,
gegebenenfalls in Gegenwart eines Verdünnungsmittels und gegebenenfalls in Gegenwart einer Base umsetzt;
oder
b) carboxy-aktivierte Derivate von Carbonsäuren der allgemeinen Formel (IV),

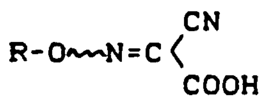
(IV)

in welcher
R die oben angegebene Bedeutung hat,
mit Aminen der Formel (III),

$R^1-NH_2$ (III)

in welcher
$R^1$ die oben angegebene Bedeutung hat,
gegebenenfalls in Gegenwart eines Katalysators und gegebenenfalls in Gegenwart eines Säurebindemittels sowie gegebenenfalls in Gegenwart eines Verdünnungsmittels umsetzt;
oder
c) 2-Cyan-2-oximino-acetamide der allgemeinen Formel (V),

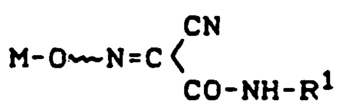
(V)

in welcher
M für Wasserstoff, ein Alkalimetallion, eine protonierte tertiäre Base oder ein quarternäres Ammoniumion steht und
$R^1$ die oben angegebene Bedeutung hat,
mit Verbindungen der Formel (VI),

R-X (VI)

in welcher
X für Halogen oder einen Sulfonyloxy-Rest steht und
R die oben angegebene Bedeutung hat,
gegebenenfalls in Gegenwart einer Base und gegebenenfalls in Gegenwart eines Verdünnungsmittels umsetzt;
oder
d) substituierte Acetyl-aminosäureester der allgemeinen Formel (VII),

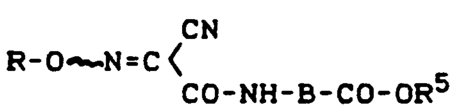
(VII)

in welcher

B, R und $R^5$ die oben angegebene Bedeutung haben,

mit Aminen der Formel (VIII),

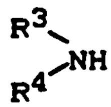

(VIII)

in welcher

$R^3$ und $R^4$ die oben angegebene Bedeutung haben,

gegebenenfalls in Gegenwart eines Verdünnungsmittels und gegebenenfalls in Gegenwart einer Base umsetzt;

oder

e) carboxy-akivierte Acetyl-aminosäuren der allgemeinen Formel (IX),

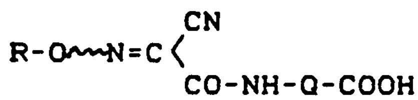

(IX)

in welcher

R die oben angegebene Bedeutung hat und

Q für A oder B steht, wobei diese die oben angegebene Bedeutung haben,

mit Verbindungen der Formel (X),

Y-H (X)

in welcher

Y für die Gruppierungen $R^2O$- oder $R^3R^4N$- steht, wobei $R^2$, $R^3$ und $R^4$ die oben angegebenen Bedeutung haben,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

f) Ammoniumsalze von 2-Cyan-2-oximino-essigsäure der allgemeinen Formel (XI),

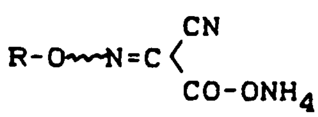

(XI)

in welcher

R die oben angegebene Bedeutung hat,

mit Aldehyden der Formel (XII),

$R^6$-CH=O (XII)

in welcher

die Gruppierung $R^6$-CH= für die oben genannten Bedeutungen von A oder B steht,

und mit Isonitrilen der Formel (XIII),

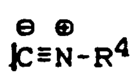

(XIII)

in welcher
R⁴ die oben angegebene Bedeutung hat,
gegebenenfalls in Gegenwart eines Verdünnungsmittels umsetzt;
oder
g) 2-Cyan-2-oximino-essigsäuren der allgemeinen Formel (IV),

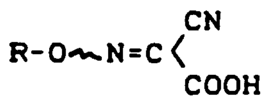

(IV)

in welcher
R die oben angegebene Bedeutung hat,
mit Isocyanaten der Formel (XIV),

$R^1$-NCO (XIV)

in welcher
$R^1$ die oben angegebene Bedeutung hat,
gegebenenfalls in Gegenwart eines Katalysators und gegebenenfalls in Gegenwart eines Verdünnungsmittels umsetzt;
oder
h) 2-Cyano-2-oximino-acetamide der allgemeinen Formel (XV),

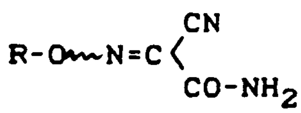

(XV)

in welcher
R die oben angegebene Bedeutung hat,
mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher
$R^1$ und X die oben angegebene Bedeutung haben,
gegebenenfalls in Gegenwart eines Verdünnungsmittels umsetzt.

Schließlich wurde gefunden, daß die neuen (2-Cyan-2-oximinoacetyl)-aminosäure-Derivate u.a. starke fungizide Eigenschaften aufweisen. Dabei zeigen überraschenderweise die erfindungsgemäßen Verbindungen eine höhere Wirkung als die aus dem Stand der Technik bekannten substituierten 2-Cyan-2-methoximino-acetamide, welche konstitutionell und/oder wirkungsmäßig naheliegende Verbindungen sind. Die erfindungsgemäßen Stoffe stellen somit eine Bereicherung der Technik dar.

Alle in der Beschreibung aufgezählten aliphatischen Reste, wie Alkyl, Alkoxy, Alkenyl u.a., einzeln oder in Zusammensetzungen, können geradkettig oder verzweigt sein, ebenso sind die Ringsysteme ein- bis mehrfach, vorzugsweise ein- bis fünffach, besonders bevorzugt ein- bis dreifach, gleich oder verschieden substituiert, auch wenn nicht ausdrücklich genannt.

Halogen bedeutet Fluor, Chlor, Brom, Jod, bevorzugt Fluor, Chlor und Brom, besonders bevorzugt Fluor und Chlor.

Die erfindungsgemäßen (2-Cyan-2-oximinoacetyl)-aminosäure-Derivate sind durch die Formel (I) allgemein definiert, wobei in der Formel (I) vorzugsweise

R für geradkettiges oder verzweigtes Alkyl mit 1 bis 4 Kohlenstoffatomen, geradkettiges oder verzweigtes Cyanalkyl mit 1 bis 4 Kohlenstoffatomen im Alkylteil, jeweils geradkettiges oder verzweigtes Alkenyl oder Alkinyl mit 2 bis 6 Kohlenstoffatomen, jeweils gegebenenfalls ein- bis fünffach, gleich oder verschieden, durch Alkyl mit 1 bis 4 Kohlenstoffatomen

substituiertes Cycloalkyl oder Cycloalkylalkyl mit jeweils 3 bis 6 Kohlenstoffatomen je Cycloalkylteil und 1 bis 4 Kohlenstoffatomen. geradkettigen oder verzweigten Alkylteil oder für jeweils gegebenenfalls ein- bis fünffach, gleich oder verschieden substituiertes Benzyl oder Phenethyl steht, wobei als Phenylsubstituenten jeweils genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen;

$R^1$ für die Gruppierungen B-CONR$^3$R$^4$ steht,

B für eine geradkettige oder verzweigte Alkylenkette mit 1 bis 4 Kohlenstoffatomen steht,

$R^3$ für ein- oder zweifach substituiertes, geradkettiges oder verzweigtes Alkyl mit 1 bis 4 Kohlenstoffatomen steht, wobei als Substituenten vorzugsweise genannt seien: Halogen, Cyano, -COOR$^{I}$, -CONR$^{II}$R$^{III}$, -NR$^{II}$R$^{III}$, -OR$^{IV}$, -S(O)$_n$R$^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen sowie Alkyl und Alkoxy mit jeweils 1 bis 4 Kohlenstoffatomen substituierten 5- oder 6-gliedriger Heterocyclus mit 1 bis 3 Heteroatomen, insbesondere Sauerstoff-, Schwefel- und Stickstoffatome, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner vorzugsweise für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten vorzugsweise genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl und Pyrrolidon; $R^3$ steht weiterhin vorzugsweise für gegebenenfalls ein- bis fünffach, gleich oder verschieden durch Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen, wie insbesondere Sauerstoff-, Schwefel- -und Stickstoffatome; als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung -OR$^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, -S(O)$_n$R$^{IV}$,gegebenenfalls ein- bis fünffach, gleich oder verschieden durch Halogen, geradkettiges oder verzweigtes Alkyl und Alkoxy mit jeweils 1 bis 4 Kohlenstoffatomen substituierten 5- oder 6-gliedriger Heterocyclus mit 1 bis 3 Heteroatomen, insbesondere Stickstoff-, Sauerstoff- und Schwefelatome, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis fünffach, gleich oder verschieden durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis fünffach, gleich oder verschieden

durch Halogen, Mercapto, Phenyl, jeweils geradkettiges oder verzweigtes Alkyl, Alkoxy, Alkylthio, Alkylsulfinyl und Alkylsulfonyl mit 1 bis 4 Kohlenstoffatomen je Alkylteil substituierten 5- oder 6-gliedrigen Heterocyclus mit 1 bis 3 gleichen oder verschiedenen Heteroatomen, insbesondere Stickstoff-, Sauerstoff- und Schwefelatome, für ein- bis fünffach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen steht, wobei gegebenenfalls Halogen und geradkettiges oder verzweigtes Alkyl mit 1 bis 4 Kohlenstoffatomen als zusätzliche Cycloalkylsubstituenten genannt seien, ferner für polycyclische Carbocyclen oder für $-OR^{IV}$ steht,

oder

$R^3$ und $R^4$ gemeinsam mit dem Stickstoffatom, an das sie gebunden sind, für einen ein- bis fünffach, gleich oder verschieden substituierten monocyclischen Heterocyclus oder für einen gegebenenfalls ein- bis fünffach, gleich oder verschieden substituierten bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl und Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen;

$R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten vorzugsweise die bei $R^{II}$ genannten Phenylsubstituenten infrage kommen; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten vorzugsweise die bei $R^{II}$ genannten Phenylsubstituenten infrage kommen; $R^{IV}$ ferner für Acyl mit 2 bis 9 Kohlenstoffatomen steht und

n für die Zahlen 0, 1 oder 2 steht.

Besonders bevorzugt sind (2-Cyan-2-oximinoacetyl)-aminosäure-Derivate der allgemeinen Formel (I), bei denen

R für Alkyl mit 1 oder 2 Kohlenstoffatomen, für Cyanmethyl oder Cyanethyl, für Allyl oder Propargyl, für jeweils gegebenenfalls ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CONR^3R^4$ steht,

B für die Gruppierung $-CH_2-$, $-CH(CH_3)-$ oder $-CH_2CH_2-$ steht,

$R^3$ für ein- oder zweifach substituiertes geradkettiges oder verzweigtes Alkyl mit 1 bis 4 Kohlenstoffatomen steht, wobei als Substituenten vorzugsweise genannt seien: Halogen, Cyano, $-COOR^I$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierten 5- oder 6-gliedrigen Heterocyclus mit 1 bis 3 gleichen oder verschiedenen Heteroatomen, insbesondere Sauerstoff-, Schwefel- und Stickstoffatome, und jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner besonders bevorzugt für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl und Pyrrolidon; $R^3$ steht weiterhin besonders bevorzugt für gegebenenfalls ein- bis dreifach, gleich oder verschieden durch Halogen, geradkettiges oder verzweigtes Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen, wie Sauerstoff-, Schwefel- oder Stickstoffatome. Als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, $-S(O)_nR^{IV}$,gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedriger Heterocyclus mit 1 bis 3 Heteroatomen, insbesondere Stickstoff-, Sauerstoff- und Schwefelatome, jeweils gegebenenfalls ein- bis dreifach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis dreifach, gleich oder verschieden durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis dreifach, gleich oder verschieden durch Halogen, Mercapto, Phenyl, jeweils geradkettiges oder verzweigtes Alkyl, Alkoxy,

Alkylthio, Alkylsulfinyl und Alkylsulfonyl mit je 1 bis 4 Kohlenstoffatomen je Alkylteil substituierten 5- oder 6-gliedrigen Heterocyclus mit 1 bis 3 gleichen oder verschiedenen Heteroatomen, insbesondere Stickstoff-, Sauerstoff- und Schwefelatome, für ein- bis dreifach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkylalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen steht, wobei gegebenenfalls Halogen und geradkettiges oder verzweigtes Alkyl mit 1 bis 4 Kohlenstoffatomen als zusätzliche Cycloalkylsubstituenten genannt seien, ferner für polycyclische Carbocyclen oder für $-OR^{IV}$ steht,

oder

$R^3$ und $R^4$ gemeinsam mit dem Stickstoffatom, an das sie gebunden sind, für einen ein- bis dreifach, gleich oder verschieden substituierten monocyclischen Heterocyclus oder für einen gegebenenfalls ein- bis dreifach, gleich oder verschieden, substituierten bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl und Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl-und Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ steht ferner für Acyl mit 2 bis 9 Kohlenstoffatomen und

n für die Zahlen 0, 1 oder 2 steht.

Ganz besonders bevorzugt sind (2-Cyan-2-oximinoacetyl)-aminosäure-Derivateder allgemeinen Formel (I), bei denen

(A)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen

im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^3$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^3$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

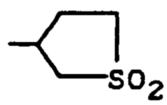 ,

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^4$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl- und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^4$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

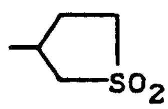 ,

$R^4$ steht weiterhin ganz besonders bevorzugt für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1- oder 2-Tetralyl.

(B)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung -B-CO-$NR^3R^4$ steht,

B für die Gruppierungen -$CH_2$-, -$CH(CH_3)$- oder -$CH_2CH_2$- steht,

$R^3$ für Wasserstoff oder geradkettiges oder verzweigtes Alkyl mit 1 bis 4 Kohlenstoffatomen steht,

$R^4$ für substituiertes Alkyl mit 1 bis 3 Kohlenstoffatomen steht, wobei als Substituenten genannt seien: Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im Alkylteil, Alkylthio mit 1 oder 2 Kohlenstoffatomen, 2-Furyl, 2-Pyridyl, 1-Morpholino, Cyclopropyl und Cyclohexyl; $R^4$ steht ferner ganz besonders bevorzugt für 1-Cyclohexenyl, für jeweils gegebenenfalls ein- bis dreifach durch Methyl substituiertes 2-Pyridyl oder 2-Pyrimidyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substitiuertes 1,2,4-Thiadiazol-5-yl, für gegebenenfalls ein- bis dreifach, gleich oder verschieden durch Mercapto und Alkyl, Alkylthio, Alkylsulfinyl und Alkylsulfonyl mit jeweils 1 bis 4 Kohlenstoffatomen substituiertes 1,3,4-Thiadiazol-5-yl, für die Gruppierung

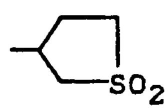 ,

für

1-Adamantyl, 2-Norbornyl, 1- oder 2-Decalyl oder 1- oder 2-Tetralyl; $R^4$ steht außerdem ganz besonders bevorzugt für ein- bis dreifach, gleich oder verschieden, substituiertes Cyclohexyl, wobei als Substituenten genannt seien: Phenyl, Cyano, Hydroxy, Alkoxy mit 1 oder 2 Kohlenstoffatomen, Alkylamino und Dialkylamino mit jeweils 1 oder 2 Kohlenstoffatomen in jedem Alkylteil, Alkylcarbamoyl und Dialkylcarbamoyl mit jeweils 1 oder 2 Kohlenstoffatomen in jedem Alkylteil, Alkoxycarbonylamino mit 1 oder 2 Kohlenstoffatomen im Alkylteil, Cyclohexyl, Cyclohexylalkyl mit 1 oder 2 Kohlenstoffatomen im Alkylteil und 1-Pyrrolidin-2-on und zusätzlich Halogen und Alkyl mit 1 oder 2 Kohlenstoffatomen.

oder

(C)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Grupierung -B-CO-$NR^3R^4$ steht,

B für die Gruppierungen -$CH_2$-, -$CH(CH_3)$- oder -$CH_2CH_2$- steht,

$R^3$ und $R^4$ gemeinsam mit dem Stickstoffatom, an das sie gebunden sind, für folgende mono-, bi- oder tricyclischen Heterocyclen oder Spiroheterocyclen stehen:

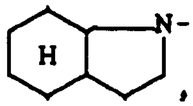 , 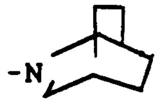 , 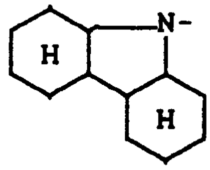 ,

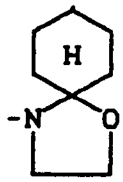 , 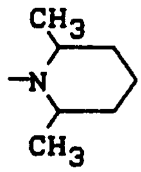 , 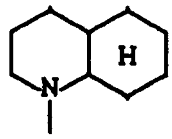 ,

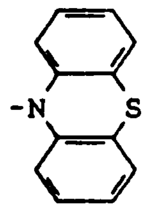 , 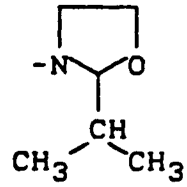 , 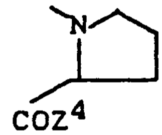

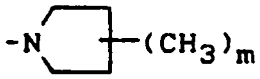 , 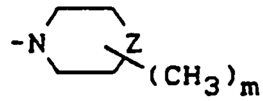

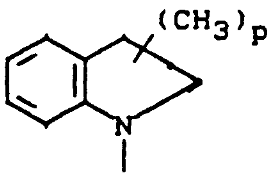 , 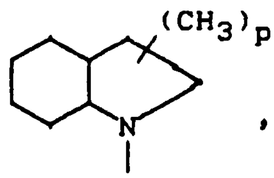 ,

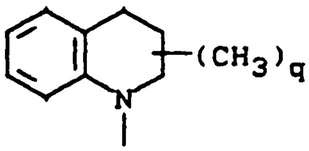 , 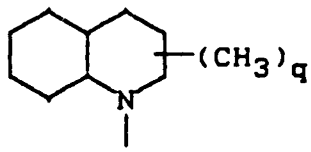 ,

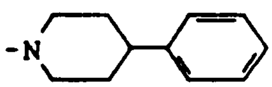 , 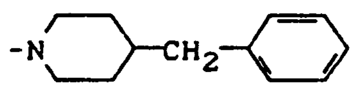 ,

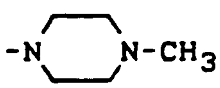 , 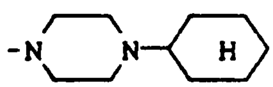 , 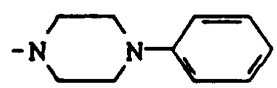 ,

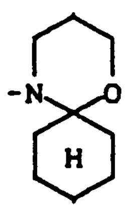 , 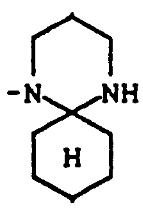 , 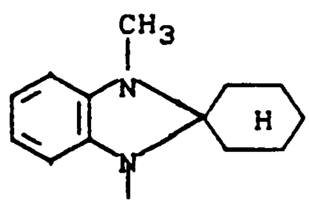 ,

wobei

- Z für Sauerstoff oder die $CH_2$-Gruppe steht,
- $Z^4$ für Hydroxy, Methoxy, Ethoxy, Amino, Methylamino, Ethylamino, Dimethylamino oder Ethylmethylamino steht,
- m für die Zahlen 1, 2, 3 oder 4 steht,
- p für die Zahlen 0, 1 oder 2 steht und
- q für die Zahlen 0, 1, 2 oder 3 steht.

Als Beispiele für erfindungsgemäße Verbindungen seien außer den bei den Herstellungsbeispielen genannten Verbindungen die in der folgenden Tabelle aufgeführten Stoffe der Formel (I) genannt:

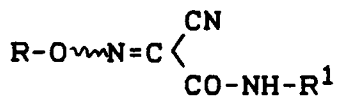 (I)

| R | $R^1$ |
|---|---|
| $CH_3$ | 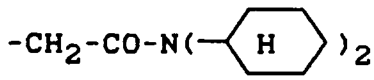 |
| $CH_3$ | 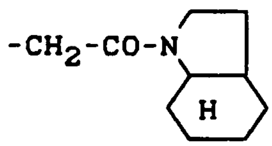 |

| R | $R^1$ |
|---|---|
| $CH_3$ | 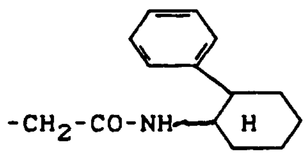 |
| $CH_3$ | 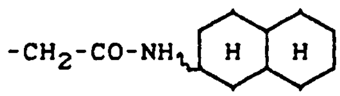 |
| $CH_3$ | 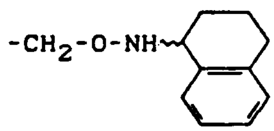 |
| $CH_3$ | 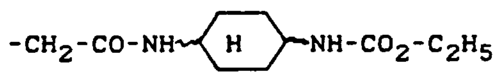 |
| $CH_3$ | 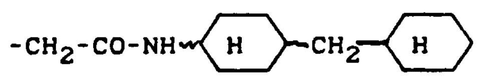 |
| $CH_3$ | 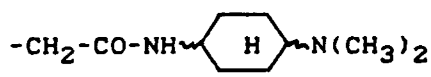 |
| $CH_3$ | 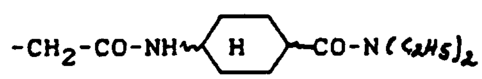 |

| R | $R^1$ |
|---|---|
| $CH_3$ | 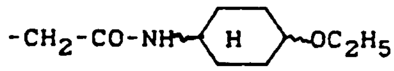 |
| $CH_3$ | 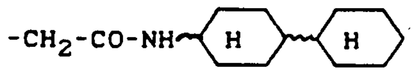 |
| $CH_3$ | 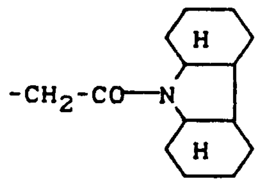 |
| $CH_3$ | $-CH_2-CO-NH-OCH_3$ |
| $CH_3$ | 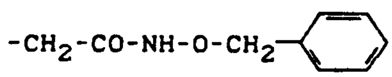 |
| $CH_3$ | 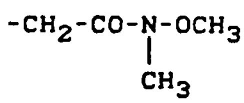 |
| $CH_3$ | 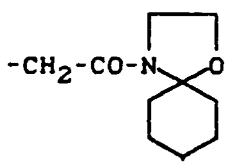 |
| $CH_3$ | 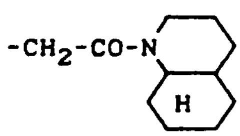 |

| R | $R^1$ |
|---|---|
| $CH_3$ | 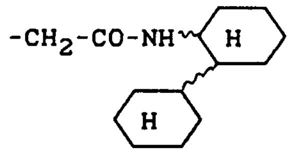 |
| $CH_3$ | 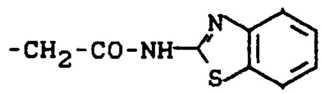 |
| $CH_3$ | 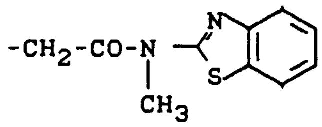 |
| $CH_3$ | 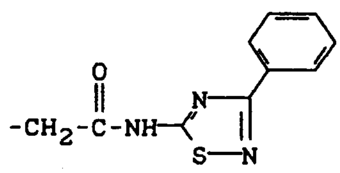 |
| $CH_3$ | 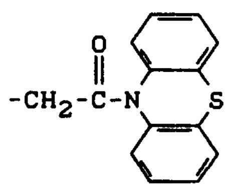 |

| R | $R^1$ |
|---|---|
| $CH_3$ | 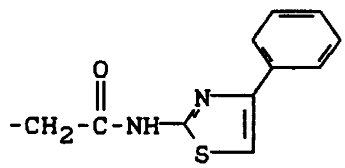 |
| $CH_3$ | 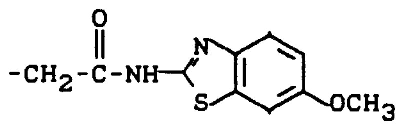 |
| $CH_3$ | 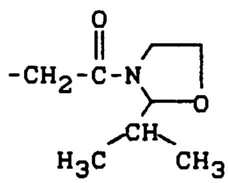 |
| $CH_3$ | 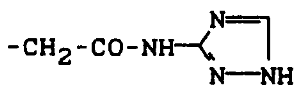 |

Verwendet man beispielsweise (E)-2-Cyan-2-methoximino-essigsäureethylesterund $N^{\alpha}$-Glycyl-prolinamin-hydrobromid als Ausgangsstoffe, so kann der Ablauf des erfindungsgemäßen Verfahrens (a) durch das folgende Formelschema wiedergegeben werden:

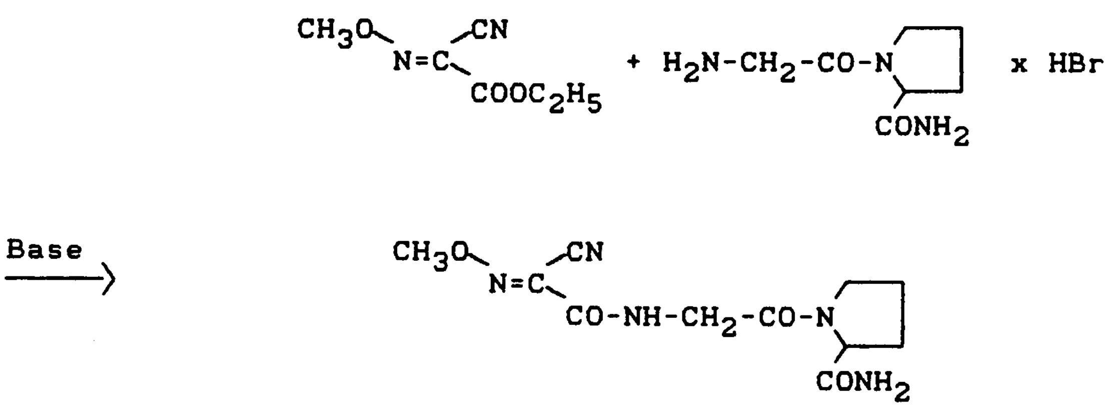


Verwendet man beispielsweise (E)-2-Cyan-2-methoximino-acetylchlorid und N-Glycyl-aminomethylcyclohexanhydrobromid als Ausgangsstoffe, so kann der Ablauf des erfindungsgemäßen Verfahren (b) durch das folgende Formelschema wiedergegeben werden:

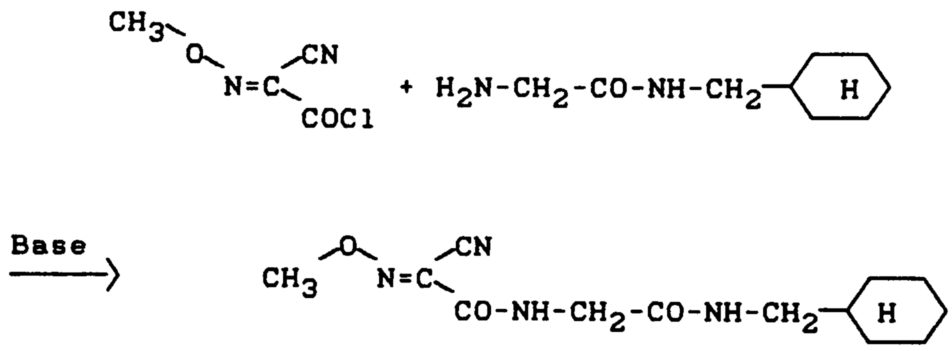


Verwendet man beispielsweise das Natriumsalz von N-(2-Cyan-2-hydroximino-acetyl)-glycincyclohexylmethylamid und Dimethylsulfat als Ausgangsstoffe, so kann der Ablauf der erfindungsgemäßen Verfahrens (c) durch das folgende Formelschema wiedergegeben werden:

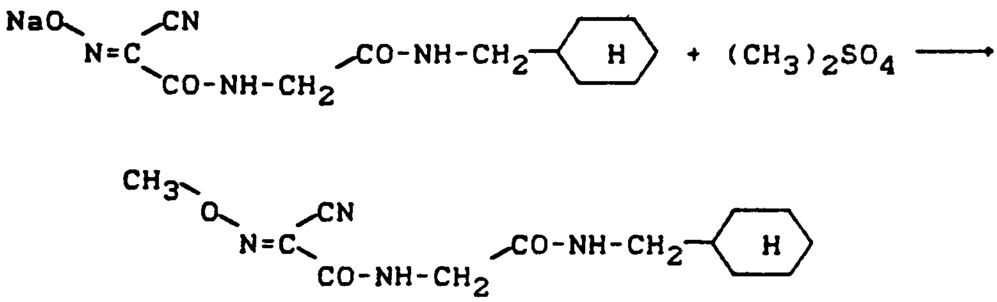

Verwendet man beispielsweise N-(2-Cyan-2-methoximino-acetyl)-glycinethylester und Ethanolamin als Ausgangsstoffe, so kann der Ablauf des erfindungsgemäßen Verfahrens (d) durch das folgende Formelschema wiedergegeben werden:

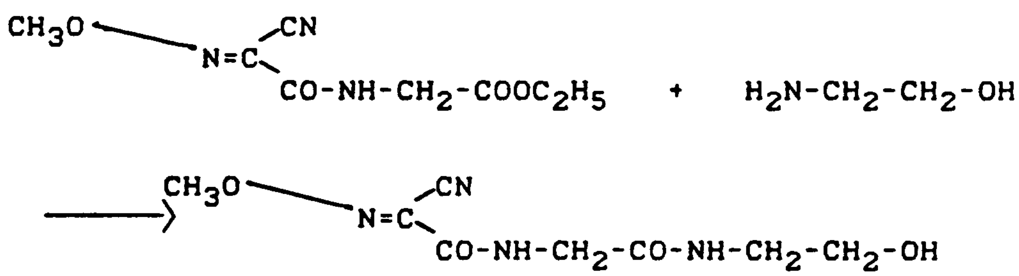

Verwendet man beispielsweise N-(2-Cyan-2-methoximinoacetyl)-glycin und 4-Ethoxycyclohexylamin als Ausgangsstoffe sowie Dicyclohexylcarbodiimid und N-Hydroxysuccinimid als Hilfsstoffe, so kann der Ablauf des erfindunsgemäßen Verfahrens (e) durch das folgende Formelschema wiedergegeben werden:

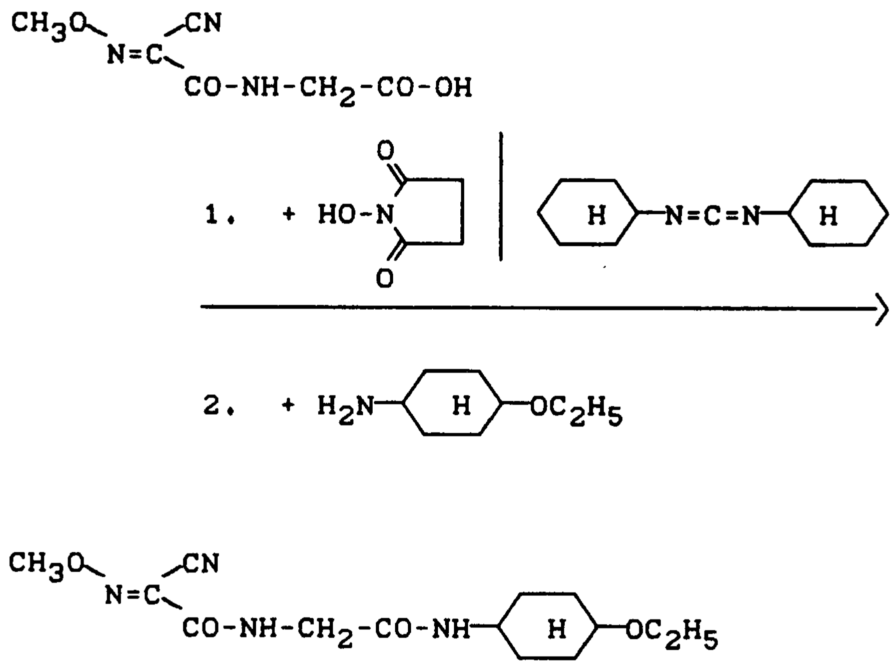


Verwendet man beispielsweise das Ammoniumsalz der 2-Cyan-2-(benzyloximino)-essigsäure, Isobutyraldehyd und Cyclohexylmethylisonitril als Ausgangsstoffe, so kann der Ablauf des erfindungsgemäßen Verfahrens (f) durch das folgende Formelschema wiedergegeben werden:

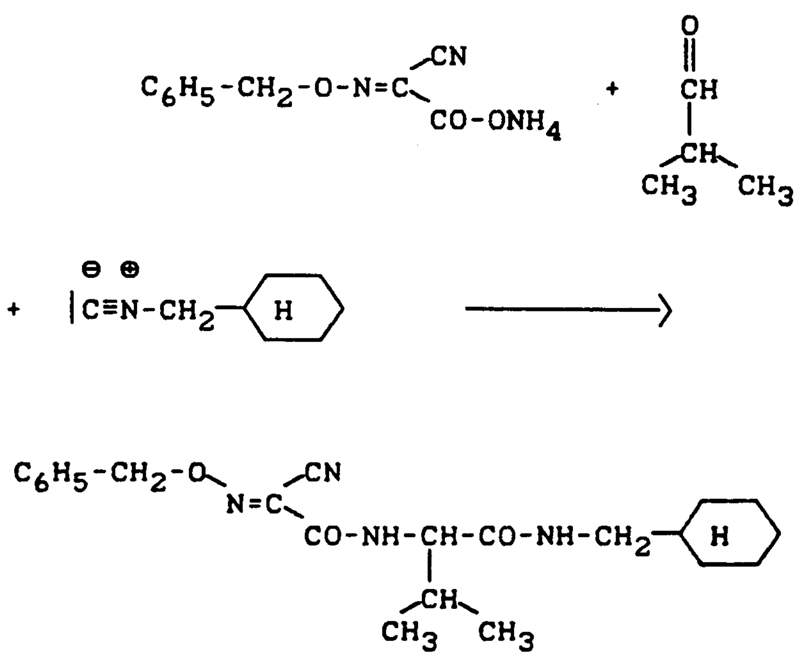

Verwendet man beispielsweise 2-Cyan-2-(methoximino)-essigsäure und Diallylaminocarbonyl-methylisocyanat als Ausgangsstoffe, so kann der Ablauf des erfindungsgemäßen Verfahrens (g) durch das folgende Formelschema wiedergegeben werden:

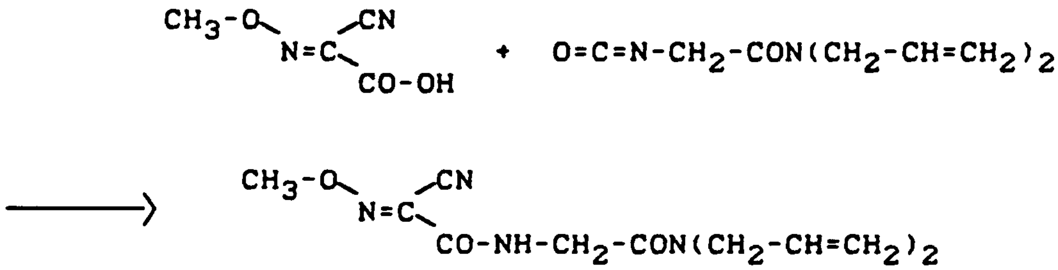

Verwendet man beispielsweise 2-Cyan-2-(methoximino)-acetamid und Bromessigsäurediallylamid als Ausgangsstoffe, so kann der Ablauf des erfindungsgemäßen Verfahrens (h) durch das folgende Formelschema wiedergegeben werden:

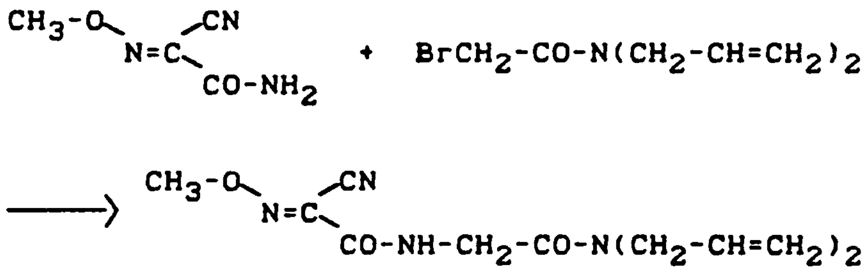

Die für die Durchführung des erfindungsgemäßen Verfahrens (a) als Ausgangsstoffe zu verwendenden 2-Cyan-2-oximino-essigsäureester sind durch die Formel (II) allgemein definiert. In dieser Formel hat R vorzugsweise die Bedeutung, die bereits im Zusammenhang mit der Beschreibung der erfindungsgemäßen Stoffe der Formel (I) für diesen Substituenten vorzugsweise genannt wurde. $R^5$ steht vorzugsweise für Methyl oder Ethyl.

Die 2-Cyan-2-oximino-essigsäureester der Formel (II) sind bekannt (vgl. z.B. J. Antibiot. 37, 557 (1984); Pesticide Science 12, 27 (1981) und EP-OS 0 201 999); bzw. sind sie Gegenstand eigener, noch nicht veröffentlichter Patentanmeldungen (vgl. die deutschen Patentanmeldung P 3 625 460 vom 28.07.86 und P 3 625 497 vom 28.07.1986) bzw. können sie nach den dort angegebenen Verfahren in allgemein bekannter Art und Weise erhalten werden.

Die außerdem für die Durchführung des erfindungsgemäßen Verfahrens (a) und ebenfalls des Verfahrens (b) als Ausgangsstoffe zu verwendenden Amine sind durch die Formel (III) allgemein definiert. In dieser Formel hat $R^1$ vorzugsweise die Bedeutung, die bereits im Zusammenhang mit der Beschreibung der erfindungsgemäßen Stoffe der Formel (I) für diesen Substituenten vorzugsweise genannt wurde.

Die Amine der Formel (III) sind bekannt (vgl. z.B. Houben-Weyl, Methoden der organischen Chemie, Band XV, Teil 1 und 2, Georg Thieme Verlag, Stuttgart 1974; bzw. R. C. Sheppard, A Specialist Periodical Report, Amino-acids, Peptides and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965 sowie EP-OS 0 201 999); bzw. sind sie Gegenstand einer eigenen, noch nicht veröffentlichten Patentanmeldung (vgl. die deutsche Patentanmeldung P 3 625 497 vom 28.07.1986) bzw. können sie nach den dort angegebenen Verfahren in allgemein bekannter Art und Weise erhalten werden.

Die für die Durchführung des erfindungsgemäßen Verfahrens (b) bzw. des erfindungsgemäßen Verfahrens (g) als Ausgangsstoffe zu verwendenden Carbonsäuren sind durch die Formel (IV) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurden.

Als carboxy-aktivierte Derivate der Carbonsäuren der Formel (IV) kommen alle carboxyaktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxysuccinimidester sowie mit Kondensationsmitteln, wie z.B. Dicyclohexylcarbodiimid oder Carbonyldiimidazol, in situ erzeugte aktivierte Formen der Carbonsäuren.

Vorzugsweise werden die den Carbonsäuren der Formel (IV) entsprechenden Säurechloride eingesetzt. Sie können hergestellt werden, indem man die Carbonsäuren der Formel (IV) oder deren Salze mit einem Halogenierungsmittel, wie beispielsweise Phosphorpentachlorid, Thionylchlorid oder Oxalylchlorid, in allgemein bekannter Art und Weise umsetzt. Bevorzugt ist der Einsatz von Oxalylchlorid zusammen mit dem Natrium- oder Kaliumsalz der Carbonsäure der Formel (IV).

Die Carbonsäuren der Formel (IV) und deren carboxyaktivierten Derivate sind bekannt (vgl. z.B. Pesticide Science 12, 27 (1981) und DE-OS 3 521 131 bzw. sind sie Gegenstand eigener, noch nicht veröffentlichter Patentanmeldungen (vgl. die deutschen Patentanmeldungen P 3 602 243 vom 25.01.1986 und P 3 625 497 vom 28.07.86) bzw. können sie nach den dort angegebenen Verfahren in allgemein bekannter Weise erhalten werden.

Die für die Durchführung des erfindungsgemäßen Verfahrens (c) als Ausgangsstoffe zu verwendenden 2-Cyan-2-oximino-acetamide sind durch die Formel (V) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde. M steht vorzugsweise für Wasserstoff oder für ein Kalium-bzw. Natriumäquivalent.

Die 2-Cyan-2-oximino-acetamide der Formel (V) sind noch nicht bekannt. Sie sind teilweise Gegenstand einer eigenen, noch nicht veröffentlichten Patentanmeldung (vgl. die deutsche Patentanmeldung P 3 702 282 vom 27.01.1987) bzw. können sie nach den dort angegebenen Verfahren erhalten werden, indem man beispielsweise Cyanessigester der Formel (XVII),

$NC\text{-}CH_2\text{-}CO\text{-}OR^7$ (XVII)

in welcher

$R^7$ für Methyl oder Ethyl steht,

mit Aminosäurederivaten der Formel (III),

$H_2N\text{-}R^1$ (III)

in welcher

$R^1$ die oben angegebene Bedeutung hat,

gegebenenfalls in Gegenwart eines Lösungsmittels, wie beispielsweise Methanol, Ethanol, Diethylether, 1,2-Dimethoxyethan, Dimethylformamid oder deren Mischungen mit Wasser bei 0 °C bis 60 °C, vorzugsweise bei Raumtemperatur umsetzt und anschließend die so erhaltenen Verbindungen der Formel (XVIII),

$NC\text{-}CH_2\text{-}CO\text{-}NH\text{-}R^1$ (XVIII)

in welcher

$R^1$ die oben angegebene Bedeutung hat,

mit salpetriger Säure oder Derivaten der salpetrigen Säure, gegebenenfalls in Gegenwart eines Verdünnungsmittels, wie beispielsweise Alkohol, und gegebenenfalls in Gegenwart einer Säure oder einer Base, wie beispielsweise Chlorwasserstoff oder Alkalimetallalkoholat, bei Temperaturen zwischen - 20 °C und 120 °C umsetzt.

Die Verbindungen der Formeln (XVII) und (III) sind bekannt; bzw. können sie in allgemein bekannter Art und Weise erhalten werden.

Die außerdem für die Durchführung des erfindungsgemäßen Verfahrens (c) als Ausgangsstoffe zu verwendenden Verbindungen sind durch die Formel (VI) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde. X steht vorzugsweise für Chlor, Brom, Methylsulfonyloxy oder p-Toluolsulfonyloxy.

Die Verbindungen der Formel (VI) sind allgemein bekannte Verbindungen der organischen Chemie; bzw. können sie in allgemein bekannter Art und Weise erhalten werden.

Die für die Durchführung des erfindungsgemäßen Verfahrens (d) als Ausgangsstoffe zu verwendenden substituierten Acetyl-aminosäureester sind durch die Formel (VII) allgemein definiert. In dieser Formel haben B und R vorzugsweise die Bedeutungen, die bereits im Zusammenhang mit der Beschreibung der erfindungsgemäßen Stoffe der Formel (I) für diese Substituenten vorzugsweise genannt wurden. $R^5$ steht vorzugsweise für Methyl oder Ethyl.

Die substituierten Acetyl-aminosäureester der allgemeinen Formel (VII) sind teilweise erfindungsgemäße Verbindungen; teilweise sind sie bekannt (vgl. EP-OS 0 201 999 und DE-OS 3 521 131), bzw. sind sie

Gegenstand eigener, noch nicht veröffentlichter Patentanmeldungen (vgl. die deutschen Patentanmeldungen P 3 602 243 vom 25.01.1986 und P 3 625 497 vom 28.07.1986); bzw. können sie nach den dort angegebenen Verfahren erhalten werden.

Die außerdem für die Durchführung des erfindungsgemäßen Verfahrens (d) als Ausgangsstoffe zu verwendenden Amine sind durch die Formel (VIII) allgemein definiert. In dieser Formel haben $R^3$ und $R^4$ vorzugsweise die Bedeutungen, die bereits im Zusammenhang mit der Beschreibung der erfindungsgemäßen Stoffe der Formel (I) für diese Substituenten vorzugsweise genannt wurden.

Die Amine der Formel (VIII) sind allgemein bekannte Verbindungen der organischen Chemie, bzw. können sie in allgemein bekannter Art und Weise erhalten werden.

Die für die Durchführung des erfindungsgemäßen Verfahrens (e) als Ausgangsstoffe zu verwendenden Acetyl-aminosäuren sind durch die Formel (IX) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde. Q steht vorzugsweise für die bereits im Zusammenhang mit der Beschreibung der erfindungsgemäßen Stoffe der Formel (I) vorzugsweise für A und B genannten Bedeutungen. Als carboxy-aktivierte Derivate kommen vorzugsweise die bei den Carbonsäuren der Formel (IV) bereits vorzugsweise genannten Derivate infrage.

Die Acetyl-aminosäuren der allgemeinen Formel (IX) sind teilweise erfindungsgemäße Verbindungen; teilweise sind sie bekannt (vgl. EP-OS 0 201 999 und DE-OS 3 521 131) bzw. sind sie Gegenstand eigener, noch nicht veröffentlichter Patentanmeldungen (vgl. die deutschen Patentanmeldungen P 3 602 243 vom 25.01.1986 und P 3 625 497 vom 28.07.1986), bzw. können sich nach den dort angegebenen Verfahren erhalten werden.

Die außerdem für die Durchführung des erfindungsgemäßen Verfahrens (e) als Ausgangsstoffe zu verwendenden Verbindungen sind durch die Formel (X) allgemein definiert.

In dieser Formel steht Y vorzugsweise für die Gruppierungen $R^2O$- und $R^3R^4N$-, wobei $R^2$, $R^3$ und $R^4$ vorzugsweise die Bedeutungen haben, die bereits im Zusammenhang mit der Beschreibung der erfindungsgemäßen Stoffe der Formel (I) für diese Substituenten vorzugsweise genannt wurden.

Die Verbindungen der Formel (X) sind allgemein bekannte Verbindungen der organischen Chemie, bzw. können sie in allgemein bekannter Art und Weise erhalten werden.

Die für die Durchführung des erfindungsgemäßen Verfahrens (f) als Ausgangsstoffe zu verwendenden Ammoniumsalze von 2-Cyan-2-oximino-essigsäuren sind durch die Formel (XI) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde.

Die Ammoniumsalze von 2-Cyan-2-oximino-essigsäuren werden erhalten, indem man die Carbonsäuren der Formel (IV) in üblicher Weise mit Ammoniak umsetzt.

Die außerdem für die Durchführung des erfindungsgemäßen Verfahrens (f) als Ausgangsstoffe zu verwendenden Aldehyde sind durch die Formel (XII) und die Isonitrile durch die Formel (XIII) allgemein definiert. In der Formel (XII) steht die Gruppierung $R^6$-CH= für die Bedeutungen von A und B, wobei diese vorzugsweise die Bedeutungen haben, die bereits im Zusammenhang mit der Beschreibung der erfindungsgemäßen Stoffe der Formel (I) für diese Substituenten vorzugsweise genannt wurden. In der Formel (XIII) hat $R^4$ vorzugsweise die Bedeutung, die bereits im Zusammenhang mit der Beschreibung der erfindungsgemäßen Stoffe der Formel (I) vorzugsweise für diesen Substituenten genannt wurde.

Die Aldehyde der Formel (XII) und die Isonitrile der Formel (XIII) sind allgemein bekannte Verbindungen der organischen Chemie, bzw. können sie in allgemein bekannter Art und Weise erhalten werden.

Die für die Durchführung des erfindungsgemäßen Verfahrens (g) als Ausgangsstoffe zu verwendenden Isocyanate sind durch die Formel (XIV) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde.

Die Isocyanate der Formel (XIV) sind allgemein bekannte Verbindungen der organischen Chemie, bzw. können sie in allgemein bekannter Art und Weise erhalten werden.

Die für die Durchführung des erfindungsgemäßen Verfahrens (h) als Ausgangsstoffe zu verwendenden 2-Cyano-2-oximino-acetamide sind durch die Formel (XV) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde.

Die 2-Cyano-2-oximino-acetamide der Formel (XV) sind bekannt (vgl. z.B. Chem. Ber. 54, 1342 (1921); DE-OS 2 623 847 und DE-OS 2 657 145); bzw. sind sie Gegenstand einer eigenen Patentanmeldung, die noch nicht veröffentlicht ist (vgl. die deutsche Patentanmeldung P 3 702 283 vom 27.01.1987) bzw. können sie nach den dort beschriebenen Verfahren erhalten werden.

Zu den weiterhin für die Durchführung des erfindungsgemäßen Verfahrens (h) als Ausgangsstoffe zu verwendenden Basen gehören alle üblichen anorganischen und organischen Basen, wie insbesondere Alkalimetallalkoholate, wie beispielsweise Kalium-tert.-butylat; Alkalimetallhydroxide und -carbonate, wie beispielsweise Natriumhydroxid, Kaliumhydroxid und Kaliumcarbonat sowie Tetraalkylammonium- bzw. Benzyltrialkylammonium-hydroxide und -alkoholate, wie beispielsweise Tetramethylammoniumhydroxid und -alkoholat, Tetrabutylammoniumhyroxid und -alkoholat oder Benzyltriethylammoniumhydroxid und -alkoholat.

Die Basen sind allgemein bekannte Verbindungen der organischen Chemie.

Die außerdem für die Durchführung des erfindungsgemäßen Verfahrens (h) als Ausgangsstoffe zu verwendenden Verbindungen sind durch die Formel (XVI) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde. X steht vorzugsweise für Chlor, Brom, Methylsulfonyloxy oder p-Toluolsulfonyloxy.

Die Verbindungen der Formel (XVI) sind allgemein bekannte Verbindungen der organischen Chemie; bzw. können sie in allgemein bekannter Art und Weise erhalten werden.

Als Verdünnungsmittel kommen für die erfindungsgemäßen Verfahren (a) und (d) Wasser sowie organische Lösungsmittel infrage. Hierzu gehören vorzugsweise Alkohole, wie Methanol, Ethanol oder Isopropanol; Ether, wie Tetrahydrofuran oder 1,2-Dimethoxyethan; Amide, wie Dimethylformamid; Nitrile, wie Acetonitril sowie tertiäre Amine, wie Pyridin.

Die erfindungsgemäßen Verfahren (a) und (d) werden gegebenenfalls in Gegenwart einer Base durchgeführt, je nachdem ob die jeweiligen Amine in Form von Säureadditionssalzen eingesetzt werden. Hierbei kommen übliche organische und anorganisch Basen infrage. Vorzugsweise genannt seien tert. Amine, wie Triethylamin; Alkoholate, wie Natriummethylat sowie Alkalicarbonate, wie Kaliumcarbonat.

Die Temperaturen können bei der Durchführung der erfindungsgemäßen Verfahren (a) und (d) in einem größeren Bereich variiert werden. Im allgemeinen abeitet man zwischen - 20 °C und der Rückflußtemperatur, vorzugsweise bei Raumtemperatur.

Bei der Durchführung der erfindungsgemäßen Verfahren (a) und (d) können alle Reaktionspartner im Überschuß eingesetzt werden; vorzugsweise arbeitet man mit äquimolaren Mengen. Die Rekationsdurchführung, Aufarbeitung und Isolierung der Rekationsprodukte der Formel (I) erfolgt in allgemein üblicher Weise.

Als Verdünnungsmittel kommen für die erfindungsgemäßen Verfahren (b) und (e) inerte organische Lösungsmittel infrage. Hierzu gehören Ketone, wie Aceton oder Ethylmethylketon; Ester, wie Ethyl- oder Methylacetat; Amide, wie Dimethylformamid; Nitrile, wie Acetonitril; Chlorkohlenwasserstoffe, wie Methylenchlorid oder Tetrachlorkohlenstoff; Kohlenwasserstoffe, wie Toluol; oder Ether, wie Tetrahydrofuran; bzw. deren Mischungen.

Als Säurebindemittel kommen für die erfindungsgemäßen Verfahren (b) und (e) übliche anorganische und organische Säurebinder infrage. Hierzu gehören vorzugsweise tertiäre Amine, wie Triethylamin, Pyridin oder N-Methylmorpholin sowie anorganische Basen, wie Natriumcarbonat oder Calciumcarbonat.

Die erfindungsgemäßen Verfahren (b) und (e) werden gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung der Verfahren (b) und (e) in einem größeren Bereich variiert werden. Im allgemeinen arbeitet man zwischen - 60 °C bis + 120 °C, vorzugsweise bei - 20 °C bis + 40 °C.

Bei der Durchführung der erfindungsgemäßen Verfahren (b) und (e) arbeitet man vorzugsweise in äquimolaren Mengen. Die Reaktionsdurchführung, Aufarbeitung und Isolierung der Reaktionsprodukte der Formel (I) erfolgt in allgemein üblicher Weise.

Als Verdünnungsmittel kommen für das erfindungsgemäße Verfahren (c) organische Lösungsmittel infrage. Hierzu gehören Alkohole, wie Methanol und Ethanol; Ketone, wie Aceton und Methylisobutylketon; Nitrile, wie Acetonitril; Ester, wie Essigester; Ether, wie Tetrahydrofuran; Amide, wie Dimethylformamid und N-Methylpyrrolidon sowie Dimethylsulfoxid.

Als Basen kommen für das erfindungsgemäße Verfahren (c) alle üblichen organischen und anorganischen Basen infrage. Hierzu gehören vorzugsweise tertiäre Amine, wie Triethylamin, Pyridin und 1,8-Diazabicyclo[5,4,0]-undec-7-en (DBU), sowie anorganische Basen, wie Natrium-, Kalium- oder Calciumcarbonat.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Bei der Durchführung des erfindungsgemäßen Verfahrens (c) setzt man pro Mol 2-Cyan-2-oximino-acetamid der Formel (V) im allgemeinen 1 bis 3 Mol, vorzugsweise 1 bis 2 Mol der Verbindung der Formel (VI) ein. Die Reaktionsdurchführung, Aufarbeitung und Isolierung der Reaktionsprodukte der Formel (I) erfolgt in allgemein üblicher Weise. Hervorzuheben ist, daß die 2-Cyan-2-oximino-acetamide der Formel (V) nach ihrer Herstellung ohne Isolierung direkt weiter umgesetzt werden können.

Als Verdünnungsmittel kommen für das erfindungsgemäße Verfahren (f) alle üblichen organischen Lösungsmittel infrage. Hierzu gehören vorzugsweise Alkohole, wie Methanol oder Ethanol; Ether, wie Diethylether; chlorierte Kohlenwasserstoffe, wie Chloroform oder Tetrachlorkohlenstoff; sowie Ketone, wie Aceton.

Die Temperaturen können bei der Durchführung des erfindungsgemäßen Verfahrens (f) in einem größeren Bereich variiert werden. Im allgemeinen arbeitet man zwischen - 20 °C bis + 40 °C, vorzugsweise bei 0 bis 20 °C.

Bei der Durchführung des erfindungsgemäßen Verfahrens (f) arbeitet man vorzugsweise in äquimolaren Mengen. Die Reaktionsdurchführung, Aufarbeitung und Isolierung der Reaktionsprodukte der Formel (I) erfolgt in allgemein üblicher Weise.

Als Verdünnungsmittel kommen für das erfindungsgemäße Verfahren (g) inerte organische Lösungsmittel infrage.

Hierzu gehören aromatische Kohlenwasserstoffe, wie Toluol; Chlorkohlenwasserstoffe, wie Methylenchlorid; Ether, wie Tetrahydrofuran oder 1,2-Dimethoxyethan; Ester, wie Essigester; und Nitrile, wie Acetonitril.

Das erfindungsgemäße Verfahren (g) wird gegebenenfalls in Gegenwart eines Katalysators durchgeführt. Beispielsweise genannt seien Triethylamin, 1,4-Diazabicyclo[2,2,2]octan (DABCO) und 1,8-Diazabicyclo[5,4,0]-undec-7-en (DBU).

Die Temperaturen können bei der Durchführung des erfindungsgemäßen Verfahrens (g) in einem größeren Bereich variiert werden. Im allgemeinen arbeitet man zwischen - 20 °C und Rückflußtemperatur, vorzugsweise bei Raumtemperatur.

Bei der Durchführung des erfindungsgemäßen Verfahrens (g) können alle Reaktionspartner in geringen Überschüssen eingesetzt werden; vorzugsweise arbeitet man mit äquimolaren Mengen. Die Reaktionsdurchführung, Aufarbeitung und Isolierung der Reaktionprodukte der Formel (I) erfolgt in allgemein üblicher Weise.

Als Verdünnungsmittel kommen für das erfindungsgemäße Verfahren (h) inerte organische Lösungsmittel infrage. Vorzugsweise verwendbar sind Ether, wie Diethylether, Diisopropylether, Tetrahydrofuran oder Dioxan; Ketone, wie Aceton oder Methylethylketon; ferner Acetonitril und Dimethylformamid.

Die Reaktionstemperatur können bei der Durchführung des erfindungsgemäßen Verfahrens (h) in einem größeren Bereich variiert werden. Bei der Salzbildung arbeitet man allgemein zwischen 0 °C und 100 °C, vorzugsweise zwischen 0 °C und 30 °C. Bei der anschließenden Alkylierung arbeitet man allgemein zwischen 0 °C und 150 °C, vorzugsweise zwischen 60 °C und 120 °C.

Bei der Durchführung des erfindungsgemäßen Verfahrens (h) setzt man vorzugsweise äquimolare Mengen ein, gegebenenfalls einen Überschuß bis zu 1 Mol an Alkylierungsmittel der Formel (XVI). Die Salze der Verbindungen der Formel (XV) können gegebenenfalls isoliert werden, es kann aber auch ohne Isolierung gearbeitet werden. In manchen Fällen erweist es sich als vorteilhaft, die Acetamide der Formel (XV) zunächst zu einem Alkalimetallsalz umzusetzen und dieses dann mit Hilfe von Tetraalkylammonium- bzw. Benzyltrialkylammoniumchloriden oder -bromiden in das entsprechende Ammoniumsalz zu überführen. Die Reaktionsdurchführung, Aufarbeitung und Isolierung der Reaktionsprodukte der Formel (I) erfolgt in allgemein üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet, vor allem als Fungizide.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fulginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Pseudocercosporella-Arten, wie beispielsweise Pseudocerco sporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Schädlingsbekämpfungsmittel können die erfindungsgemäßen Wirkstoffe mit besonders gutem Erfolg zur Bekämpfung von Phytophthora-Arten, wie beispielsweise Phytophthora infestans, an Tomaten eingesetzt werden; sowie auch zur Bekämpfung von Plasmopara-Arten, wie beispielsweise Plasmopara viticola, an Reben.

Besonders hervorzuheben ist, daß die erfindungsgemäßen Wirkstoffe nicht nur eine protektive Wirkung entfalten, sondern auch kurativ wirksam sind, also bei Anwendung nach der Kontamination mit den Sporen des Pilzes.

Ferner eignen sich die erfindungsgemäßen Wirkstoffe sehr gut zur Bekämpfung von Pyricularia-Arten wie Pyricularia oryzae am Reis.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und

Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1:

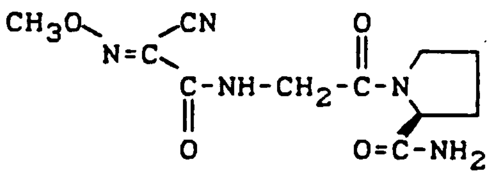

(Verfahren a)

Zu einer Lösung von 6,45 g (39,6 mMol) 96prozentigem (E)-2-Cyan-2-methoximino-essigsäureethylester und 10,0 g (39,6 mMol) $N^{\alpha}$-Glycyl-L-prolinamid-hydrobromid in 45 ml absolutem Methanol tropft man bei 0 °C 8,0 g (79,2 mMol) Triethylamin und rührt 21 Stunden bei Raumtemperatur. Man verdünnt mit 160 ml Diethylether, saugt den Niederschlag ab, dampft das Filtrat im Vakuum ein und chromatographiert den Rückstand an einer Kieselgelsäule (5 x 50 cm) mit MeOH/$CHCl_3$ (0-70 %).

Man erhält so 4,56 g einer Fraktion, die neben dem gewünschten Produkt noch Triethylaminhydrobromid enthält. Letzteres wird mit Lewatit S 100, $H^+$-Form, aus der wässrigen Lösung entfernt. Nach Eindampfen und Trocknen erhält man so 3,2 g (29 % der Theorie) $N^{\alpha}$-{N-[(E)-2-Cyan-2-methoximinoacetyl]-glycyl}-L-prolinamid vom Schmelzpunkt 90 - 94 °C.

Herstellung des Ausgangsproduktes

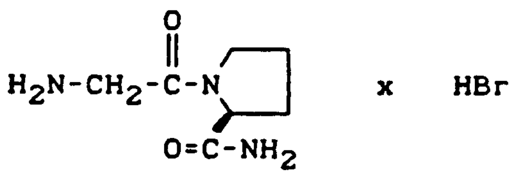

Eine Mischung aus 13 g (0,043 Mol) $N^{\alpha}$-(N-Benzyloxycarbonyl-glycyl)-L-prolinamid und 55 ml einer 33prozentigen Lösung von Bromwasserstoff in Eisessig wird 4 Stunden bei Raumtemperatur gerührt. Man versetzt mit 350 ml Ether, saugt den Niederschlag ab, wäscht ihn mit Essigester und Petrolether und trocknet ihn im Vakuum über Kaliumhydroxid.
Man erhält 10 g (92 % der Theorie) $N^{\alpha}$-Glycyl-L-prolinamid-hydrobromid vom Schmelzpunkt 120 °C - 122 °C (Zersetzung).

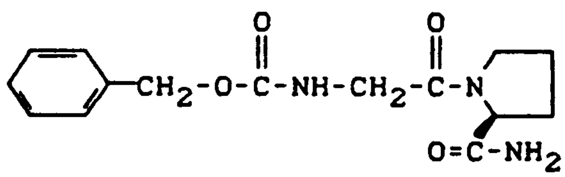

In eine Lösung von 34,2 g (0,106 Mol) $N^{\alpha}$-(N-Benzyloxycarbonyl-glycyl)-L-prolinmethylester in 150 ml Methanol leitet man bei 0 °C bis zur Sättigung Ammoniak ein und läßt die Mischung 3 Tage bei Raumtemperatur stehen. Danach wird nochmals bei 0 °C Ammoniak eingeleitet. Nach insgesamt 5 Tagen bei Raumtemperatur gibt man 500 ml absoluten Ether hinzu, worauf nach Anreiben ein Niederschlag ausfällt. Dieser wird abgesaugt, mit Ether gewaschen und getrocknet.
Man erhält 15,7 g (49 % der Theorie) $N^{\alpha}$-(N-Benzyloxycarbonyl-glycyl)-L-prolinamid vom Schmelzpunkt 142 °C bis 143 °C.

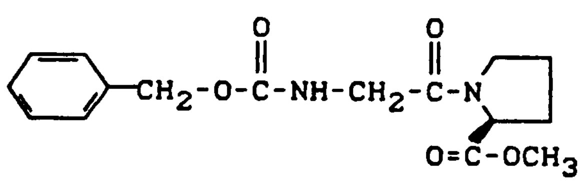

In eine Lösung von 41,9 g (0,2 Mol) N-Benzyloxycarbonylglycin und 19,8 g (0,2 Mol) N-methyl-piperidin in 250 ml trockenem Methylenchlorid tropft man bei - 20 °C 27,6 g (0,2 Mol) Chlorameisensäureisobutylester. Nach 10minütigem Rühren bei - 20 °C gibt man bei - 60 °C in einem Guß eine vorgekühlte Lösung von 33,2 g (0,2 Mol) L-Prolinmethylester-hydrochlorid und 19,8 g (0,2 mol) N-Methylpiperidin in 100 ml trockenem Methylenchlorid hinzu, rührt 2 Stunden bei - 15 °C und 3 Tage bei Raumtemperatur.
Die Reaktionslösung wird mit 1M Citronensäure (2 x 100 ml), gesättigter Natriumhydrogencarbonat-Lösung (2 x 100 ml) und Wasser (2 x 100 ml) gewaschen, über Natriumsulfat getrocknet und eingedampft.
Man erhält 57,0 g (89 % der Theorie) N-(N-Benzyloxycarbonyl-glycyl)-L-prolinmethylester als gelbliches Öl.

Beispiel 2:

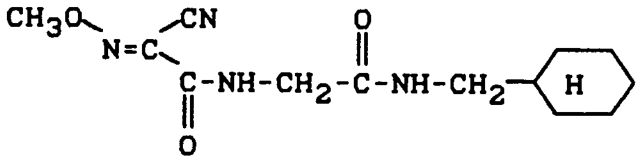

(Verfahren b)

Eine Lösung von 12,6 g (0,05 Mol) N-Glycylaminomethylcyclohexan-hydrobromid in 100 ml trockenem Dimethylformamid wird mit 11,1 g (0,11 Mol) Triethylamin bei 0 °C tropfenweise mit 7,4 g (0,05 Mol) (E)-2-Cyan-2-methoximinoacetylchlorid versetzt. Man rührt 1 Stunde bei 0 °C und 17 Stunden bei Raumtemperatur.
Das Reaktionsgemisch wird im Vakuum eingedampft, der Rückstand in 250 ml Toluol und 50 ml Methylenchlorid gelöst, die Lösung mit je 100 ml 1M Salzsäure, gesättigter Natriumhyrogencarbonat-Lösung und gesättigter Kochsalzlösung gewaschen und über Natriumsulfat getrocknet.
Man filtriert, dampft ein, kristallisiert den Rückstand aus Essigester (50 ml)/Petrolether (180 ml) und erhält 5,4 g (39 % der Theorie) N-{N-[(E)-2-Cyan-2-methoximino-acetyl]-glycyl}-aminomethylcyclohexan vom Schmelzpunkt 90 °C - 92 °C.

Herstellung des Ausgangsproduktes der Formel (IV)

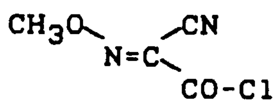

20 g (0,12 Mol) des Kaliumsalzes der (E)-2-Cyan-2-methoximinoessigsäure werden in 250 ml trockenem Ether suspendiert und nach Zugabe von einigen Tropfen Dimethylformamid bei 0 °C tropfenweise mit 76,2 g (0,6 Mol) Oxalylchlorid versetzt. Das Reaktionsgemisch wird 2 Stunden bei 0 °C gerührt, filtriert, das Filtrat im Vakuum bei Raumtemperatur eingedampft und der Rückstand durch zweimaliges Abdampfen mit Methylenchlorid vom restlichen Oxalylchlorid befreit.
Man erhält 13,5 g (77 % der Theorie) (E)-2-Cyan-2-methoximinoacetylchlorid als gelbes Öl, das sofort weiter umgesetzt wird.

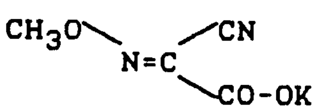

In eine Lösung von 124,8 g (0,672 Mol) 84prozentigem (E)- 2-Cyan-2-methoximino-essigsäureethylester in 500 ml Ethanol wird bei 20 °C eine Lösung von 45,1 g (0,806 Mol) Kaliumhydroxid in 500 ml Wasser getropft und das Reaktionsgemisch eine Stunde bei 40 °C gerührt. Die Lösung wird im Vakuum bei 40 °C eingedampft, der Rückstand mit Methanol 30 Minuten verrührt, abgesaugt, mit Ethanol, Acetonitril und Dichlormethan gewaschen und bei Raumtemperatur getrocknet.
Man erhält 58,6 g (53 % der Theorie) des Kaliumsalzes der (E)-2-Cyan-2-methoximino-essigsäure.

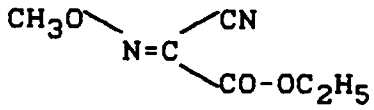

In eine Suspension von 164 g (1 Mol) (E)-2-Cyan-2-hydroximinoessigsäureethylester, Natriumsalz (G. Kinast, Liebigs Ann. Chem., 1981, 1561) und 138 g pulverisiertem Kaliumcarbonat in 1,5 l Aceton werden 161 g (1,25 Mol) Dimethylsulfat (98 %ig) in 30 Minuten getropft und das Reaktionsgemisch 3 Stunden unter Rückfluß erhitzt. Nach Abkühlen wird über Kieselgur filtriert und eingedampft. Man erhält 124,8 g (68 % der Theorie) (E)-2-Cyan-2-methoximino-essigsäure-ethylester als rotbraunes Öl von 85 % Reinheit (GC). Nach Chromatographie an der fünffachen Menge Kieselgel 60 mit Chloroform erhält man ein 93prozentiges hellgelbes Öl.

Herstellung des Ausgangsproduktes der Formel (III)

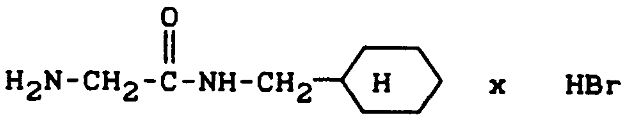

Man löst 54,8 g (0,18 Mol) N-(N-Benzyloxycarbonyl-glycyl)-aminomethylcyclohexan in 200 ml 33prozentigem Bromwasserstoff in Eisessig und rührt 3 Stunden bei Raumtemperatur. Nach Verdünnen mit 150 ml absolutem Ether wird der Niederschlag abgesaugt, mit absolutem Ether gewaschen und im Exsikkator über Kaliumhydroxid getrocknet.
Man erhält 44,8 g (99 % der Theorie) N-Glycyl-aminomethylcyclohexan-hydrobromid, das bei 205 °C-207 °C unter Zersetzung schmilzt.

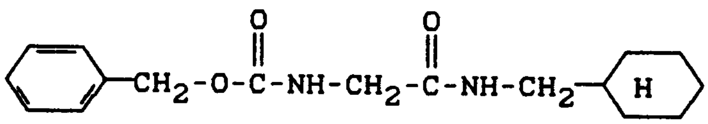

In eine Suspension von 41,9 g (0,2 Mol) N-Benzyloxycarbonyl-glycin in 250 ml Methylenchlorid tropft man bei -20 °C 19,8 g (0,2 Mol) vorgekühltes N-Methylpiperidin und 27,6 g (0,2 Mol) vorgekühlten Chlorameisensäureisobutylester, rührt 10 Minuten bei -20 °C und gibt dann nach Abkühlung auf - 60 °C in einem Guß eine Lösung von 22,7 g (0,2 Mol) Aminomethylcyclohexan in 50 ml Methylenchlorid hinzu. Man läßt die Temperatur auf - 15 °C ansteigen, rührt 2 Stunden bei - 15 °C und 17 Stunden bei Raumtemperatur. Die Reaktionslösung wird je 2 mal mit je 100 ml 1M Salzsäure, gesättigter Natriumhydrogencarbonat-Lösung und gesättigter Kochsalzlösung gewaschen, über Natriumsulfat getrocknet und im Vakuum eingedampft.
Man erhält 59,7 g (98 % der Theorie) N-(N-Benzyloxycarbonyl-glycyl)-aminomethylcyclohexan vom Schmelzpunkt 95 °C - 98 °C.

Beispiel 3:

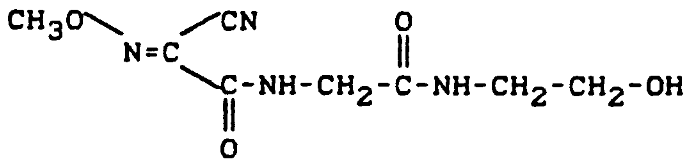

(Verfahren d)

Eine Lösung von 22,5 g (0,1 Mol) 95prozentigem N-[(E)-2-Cyan-2-methoximino-acetyl]-glycinethylester in 200 ml Isopropanol wird bei 20 °C mit 18,0 g (0,3 Mol) Ethanolamin versetzt und 15 Stunden bei Raumtemperatur gerührt. Nach Zugabe von 200 ml Ether wird der Niederschlag abgesaugt, mit Ether gewaschen und getrocknet.
Man erhält 22,4 g (98 % der Theorie) N-{N-[(E)-2-Cyan-2-methoximinoacetyl]-glycyl}-ethanolamin vom Schmelzpunkt 145 °C - 148 °C.

Beispiel 4

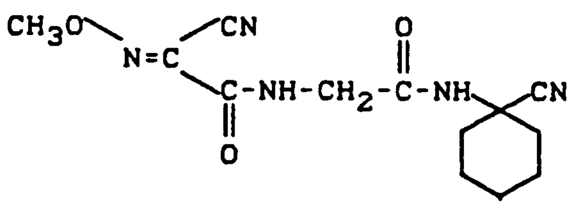

(Verfahren e)

In eine Lösung von 9,25 g (0,05 Mol) N-[(E)-2-Cyan-2-methoximino-acetyl]-glycin und 5,95 g (0,05 Mol) N-Hydroxysuccinimid in 50 ml trockenem Dimethylformamid tropft man bei 0 °C eine Lösung von 10,4 g (0,05 Mol) N,N′-Dicyclohexylcarbodiimid in 25 ml trockenem Dimethylformamid und rührt 1 Stunde bei 0 °C. Darauf tropft man bei 0 °C eine Lösung von 8,05 g (0,05 Mol) 1-Cyano-cyclohexylamin-hydrochlorid in 75 ml trockenem Dimethylformamid und 5,05 g (0,05 Mol) Triethylamin zu und rührt 20 Stunden bei Raumtemperatur.
Der Niederschlag (Dicyclohexylharnstoff) wird abgesaugt und mit Dimethylformamid gewaschen. Die vereinigten Filtrate werden eingedampft, der Rückstand wird in Methylenchlorid gelöst, die Lösung mit Natriumhydrogencarbonat-Lösung, 1M Salzsäure und Wasser gewaschen, getrocknet und eingedampft. Nach Kristallisation aus Chloroform/Petrolether erhält man 8,7 g (60 % der Theorie) 1-{$N^{\alpha}$-[(E)-2-Cyan-2-methoximino-acetyl]-glycinamido}-cyclohexylcyanid vom Schmelzpunkt 151 °C - 153 °C.

Herstellung des Ausgangsproduktes

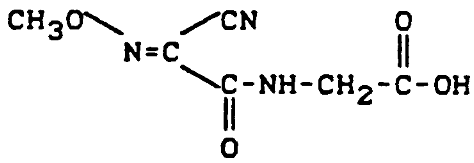

47,2 g (0,22 Mol) N-[(E)-2-Cyan-2-methoximino-acetyl]-glycinethylester und 35,6 g (0,11 Mol) Bariumhydroxidoctahydrat werden in 50 ml Wasser und 100 ml Ethanol 3 Stunden bei Raumtemperatur gerührt. Die Mischung wird im Vakuum bei 40 °C eingedampft, der Rückstand mit Ether gewaschen, in 400 ml Wasser gelöst und unter Eiskühlung mit 6,2 ml (0,11 Mol) konzentrierter Schwefelsäure versetzt. Das ausgefallene Bariumsulfat wird abfiltriert, das Filtrat im Vakuum eingedampft und der Rückstand getrocknet.
Man erhält 31 g (76 % der Theorie) N-[(E)-2-Cyan-2-methoximinoacetyl]-glycin vom Schmelzpunkt 95 °C - 100 °C.

Beispiel 5:

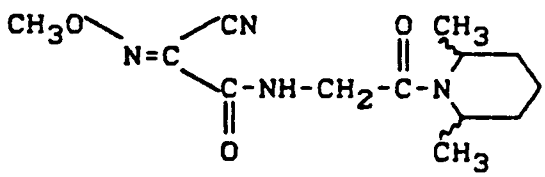

(Verfahren h)

Man suspendiert 11,6 g (0,05 Mol)Benzyltriethylammoniumchlorid in 50 ml absolutem Tetrahydrofuran, gibt 11,5 g (0,05 Mol) 26prozentige methanolische Natriummethylatlösung dazu und dampft die Mischung im Vakuum ein. Der Rückstand wird mit einer Lösung vom 6,35 g (0,05 Mol) (E)-2-Cyan-2-methioximinoacetamid in 150 ml absolutem Tetrahydrofuran sowie mit einer Lösung von 9,5 g (0,05 Mol) N-Chloracetyl-2,6-dimethyl-piperidin in 50 ml absolutem Tetrahydrofuran versetzt. Man erhitzt die Mischung 5 Stunden unter Rückfluß und rührt 16 Stunden bei Raumtemperatur. Der nach Abdestillieren des Lösungsmittels verbliebene Rückstand wird mit 200 ml Eiswasser verrieben, der Niederschlag abgesaugt, getrocknet und aus Essigester/Petrolether umkristallisiert.
Man erhält 7,05 g (50% der Theorie) N-{N-[(E)-2-Cyan-2-methoximino-acetyl]-glycyl}-2,6-dimethylpiperidin vom Schmelzpunkt 125 °C - 126 °C.

In analoger Weise und gemäß den erfindungsgemäßen Verfahren (a) bis (h) werden die nachfolgenden (2-Cyan-2-oximinoacetyl)-aminosäure-Derivate der allgemeinen Formel (I) erhalten:

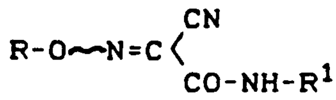 (I)

| Bsp. Nr. | R | $R^1$ | Physikal. Daten |
|---|---|---|---|
| 6 | $CH_3$- | 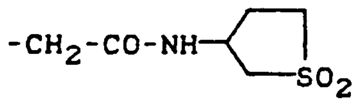 | Fp:157-158° C (E-Isomer) |
| 7 | $CH_3$ | 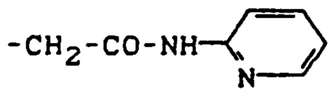 | Fp:155-156° C (E-Isomer) |
| 8 | $CH_3$- | $-CH_2-CO-NH-CH_2CH_2-O-COCH_3$ | Fp:110-114° C (E-Isomer) |
| 9 | $CH_3$- | 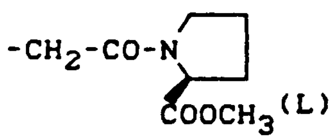 | Fp:139-141° C (E-Isomer) |
| 10 | $CH_3$- | 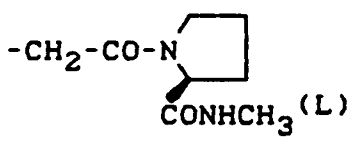 | Fp:181-183° C (E-Isomer) |

| Bsp. Nr. | R | $R^1$ | Physikal. Daten |
|---|---|---|---|
| 11 | $CH_3$- | $-CH_2-CO-$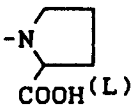 | Fp:65-72° C (E-Isomer) |
| 12 | $CH_3$- | $-CH_2-CO-$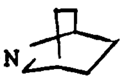 | Fp:120-122° C (E-Isomer) |
| 13 | $CH_3$- | $-CH_2-CO-NH$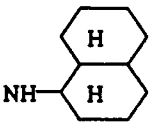 | Fp:129-131° C (E-Isomer) |
| 14 | $CH_3$ | $-CH_2-CO-NH-CH_2$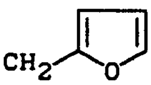 | Fp: 93- 95° C (E-Isomer) |
| 15 | $CH_3$ | $-CH_2-CO-N(CH_2-CH=CH_2)_2$ | Fp: 67- 68° C (E-Isomer) |
| 16 | $CH_3$ | $-CH_2-CO-NH$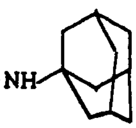 | Fp:213-214° C (E-Isomer) |
| 17 | $CH_3$ | $-CH_2-CO-N-$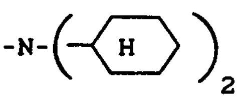2 | Fp:150° C (Zers.) (E-Isomer) |
| 18 | $CH_3$ | $-CH_2-CO-NH$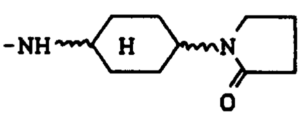 | Fp:237-39° C |

| Bsp. | R | $R^1$ | Physikal. Daten [°C] |
|---|---|---|---|
| 19 | $CH_3$ | $-CH_2-CO-NH-$ 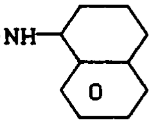 | Fp.:135-37 |
| 20 | $CH_3$ | $-CH_2-CO-NH-$ 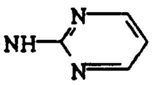 | Fp.:210-12 |
| 21 | $CH_3$ | $-CH_2-CO-NH-$ 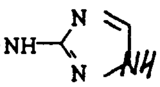 | Fp.:252-54 |
| 22 | $CH_3$ | $-CH_2-CO-NH-$ 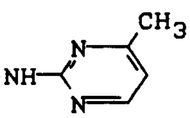 | Fp.:205-06 |
| 23 | $CH_3$ | $-CH_2-CO-NH-$ 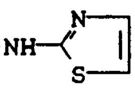 | Fp.:219-20 |
| 24 | $CH_3$ | $-CH_2-CO-N$ 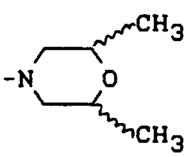 | Fp.:169-70 |
| 25 | $CH_3$ | $-CH_2-CO-NH$ 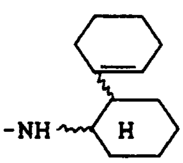 | Fp.:104-06 |

| Bsp. | R | $R^1$ | Physikal. Daten [°C] |
|---|---|---|---|
| 26 | $CH_3$ | 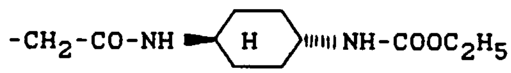 (trans) | Fp.:220-22 |
| 27 | $CH_3$ | 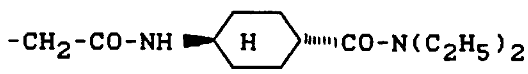 (trans) | Fp.:228-30 (Zers.) |
| 28 | $CH_3$ | 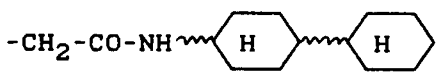 (cis/trans) | Fp.:179-81 |
| 29 | $CH_3$ | 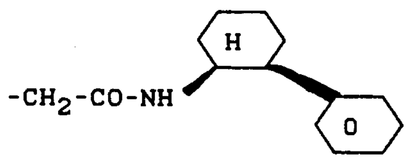 (cis) | Fp.:141-43 |
| 30 | $CH_3$ | 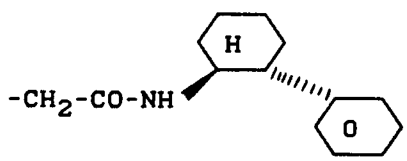 (trans) | Fp.:181-83 |
| 31 | $CH_3$ | 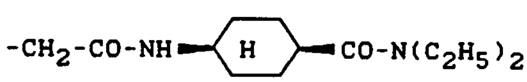 (cis) | Fp.:160-65 |

| Bsp. | R | $R^1$ | Physikal. Daten [°C] |
| --- | --- | --- | --- |
| 32 | $CH_3$ | 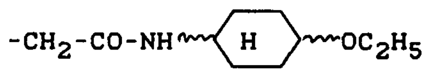 (cis/trans) | Fp.:172-74 |
| 33 | $CH_3$ | 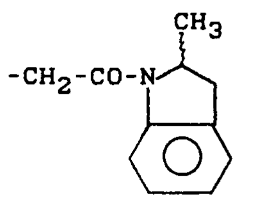 | Fp.:157-60 |
| 34 | $CH_3$ | 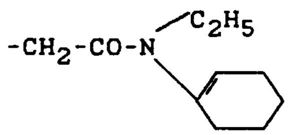 | Fp.:86-88 |
| 35 | $CH_3$ | 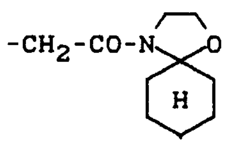 | Fp.:165-70 (E-Isomer) |
| 36 | $CH_3$ | 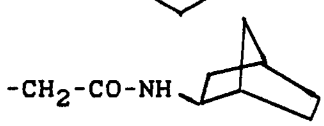 (exo) | Fp.:129-31 (E-Isomer) |
| 37 | $CH_3$ | 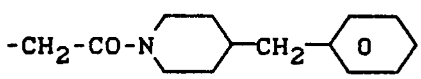 | Fp.:112-14 (E-Isomer) |
| 38 | $CH_3$ | 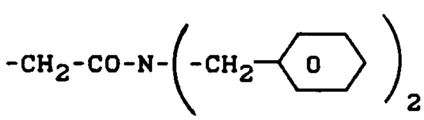 | Fp.:97-100 (E-Isomer) |

| Bsp. | R | $R^1$ | Physikal. Daten |
|---|---|---|---|
| 39 | $CH_3-$ | $-CH_2-CO-$ 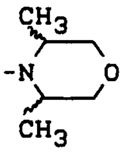 | Fp.:167-69° C (E-Isomer) |
| 40 | $CH_3$ | $-CH_2-CO-$ 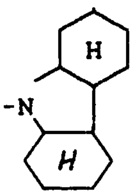 | Fp.:110-13° C (E-Isomer) |
| 41 | $CH_3$ | $-CH_2-CO-$ 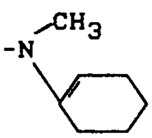 | Fp.:128-30° C (E-Isomer) |
| 42 | $CH_3$ | $-CH_2-CO-$ 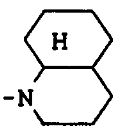 | Fp.:80-83° C (E-Isomer) |
| 43 | $CH_3$ | $-CH_2-CO-NH-O-CH_2-$ 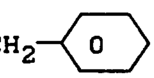 | Fp.:116-17° C (E-Isomeres) |
| 44 | $CH_3$ | $-CH_2-CO-N-(-CH_2-C{\equiv}CH)_2$ | Fp.:115-16° C (E-Isomeres) |
| 45 | $CH_3$ | $-CH_2-CO-$ 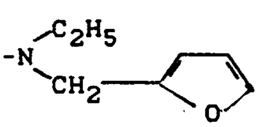 | zähfl. Öl (E-Isomeres) |

| Bsp. | R | $R^1$ | Physikal. Daten |
|---|---|---|---|
| 46 | $CH_3$ | $-CH_2-CO-N-[-(CH_2)_3-O-(CH_2)_2OCH_3]_2$ | zähfl. Öl (E-Isomeres) |
| 47 | $CH_3$ | $-CH_2-CO-N-(-CH_2CH_2OCH_3)_2$ | Fp.:55-57° C (E-Isomeres) |
| 48 | $CH_3$ | 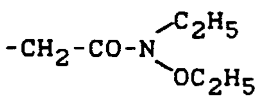 | Fp.:133-36° C (E-Isomeres) |
| 49 | $CH_3$ | 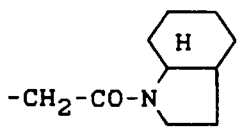 | zähfl. Öl (E-Isomeres) |

Anwendungsbeispiele

In den folgenden Anwendungsbeispielen werden die nachstehend aufgeführten Verbindungen als Vergleichssubstanzen eingesetzt:

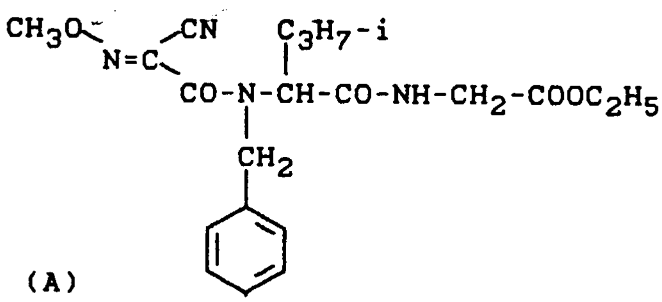

(A)

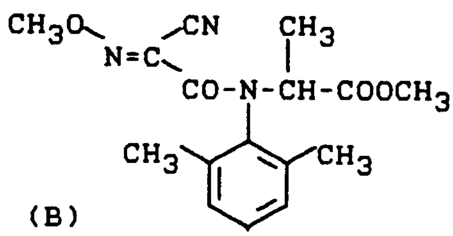

(B)

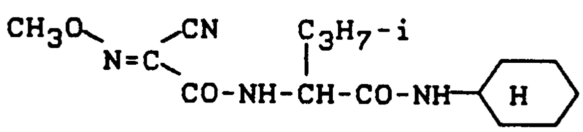

(C)

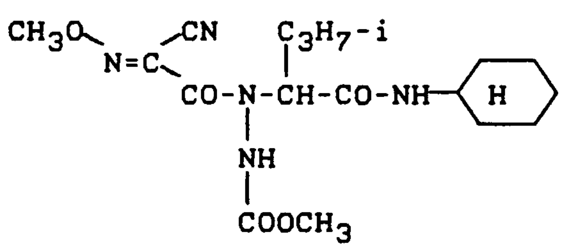

(D)

(bekannt aus DE-OS 3 521 131)

Beispiel A

Phytophthora-Test (Tomate) /kurativ

| | | |
|---|---|---|
| Lösungsmittel: | 4,7 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 ° C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß dem Herstellungsbeispielen: 7 und 9.

Beispiel B

Plasmopara-Test (Reben) / protektiv

| | | |
|---|---|---|
| Lösungsmittel: | 4,7 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 ° C und 100 % relativer Luftfeuchtigkeit. Anschließend werden die Pflanzen 5 Tage im Gewächshaus bei 22 ° C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 2 und 7.

## Patentansprüche

**Patentansprüche für folgende Vertragsstaaten : AT, BE, CH, DE, FR, GB, GR, IT, LI, NL**

**1.** (2-Cyan-2-oximinoacetyl)-aminosäure-Derivate der allgemeinen Formel (I)

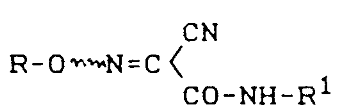

(I)

in welcher

R für Alkyl, Cyanalkyl oder jeweils gegebenenfalls substituiertes Alkenyl, Alkinyl, Cycloalkyl, Cycloalkylalkyl oder Phenylalkyl steht,

| | |
|---|---|
| $R^1$ | für die Gruppierungen -B-CONR$^3$R$^4$ steht, wobei |
| B | für eine gegebenenfalls substituierte, geradkettige oder verzweigte Alkylenkette steht, |
| $R^3$ | für substituiertes Alkyl steht mit folgenden Substituenten : Halogen, Cyano, -COOR$^I$, -CONR$^{II}$R$^{III}$,-NR$^{II}$R$^{III}$, -OR$^{IV}$, -S(O)$_n$R$^{IV}$, Acyl, jeweils gegebenenfalls substituiertes Aryl, Heterocyclyl, Cycloalkyl und Cycloalkenyl; R$^3$ ferner für jeweils gegebenenfalls substituiertes Alkenyl, Alkinyl, Cycloalkyl, Cycloalkenyl, Aryl oder Heterocyclyl steht und |
| $R^4$ | für substituiertes Alkyl steht mit folgenden Substituenten: Halogen, Cyano, -COOR$^I$, -CONR$^{II}$R$^{III}$,-NR$^{II}$R$^{III}$, -OR$^{IV}$, -S(O)$_n$R$^{IV}$, Acyl, jeweils gegebenenfalls substituiertes Aryl, Heterocyclyl, Cycloalkyl und Cycloalkenyl; R$^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung -OR$^{IV}$ steht, |
| | oder |
| $R^3$ | für Wasserstoff oder Alkyl steht und |
| $R^4$ | für substituiertes Alkyl steht mit den folgenden Substituenten: Acyl, Hydroxy, Alkylcarbonyloxy, -S(O)$_n$R$^{IV}$, jeweils gegebenenfalls substituiertes Heterocyclyl, Cycloalkyl und Cycloalkenyl; R$^4$ ferner für jeweils gegebenenfalls substituiertes Cycloalkenyl, Heterocyclyl oder polycyclische Carbocyclen, für substituiertes Cycloalkyl, wobei jedoch Halogen und Alkyl nicht als alleinige Substituenten vorhanden sein dürfen, oder für die Gruppierung -OR$^{IV}$ steht, |
| | oder |
| $R^3$ und $R^4$ | gemeinsam mit dem Stickstoffatom, an das sie gebunden sind, für einen substituierten monocyclischen Heterocyclus oder für einen gegebenenfalls substituierten bi- oder tricyclischen Heterocyclus oder Spiroheterocyclus mit 1 bis 3 weiteren, gleichen oder verschiedenen, Heteroatomen stehen, |
| $R^I$ | für Wasserstoff oder gegebenenfalls substituiertes Alkyl steht mit folgenden Substituenten: Halogen, jeweils gegebenenfalls substituiertes Aryl, Cycloalkyl und Cycloalkenyl, |
| $R^{II}$ und $R^{III}$ | gleich oder verschieden sind und für Wasserstoff oder gegebenenfalls substituiertes Alkyl stehen mit folgenden Substituenten: Halogen, -COOR$^{IV}$, -CONR$^I$R$^{IV}$, jeweils gegebenenfalls substituiertes Aryl, Cycloalkyl und Cycloalkenyl; R$^{II}$ und R$^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann, |
| $R^{IV}$ | für Wasserstoff, Alkyl, Aralkyl oder Acyl steht und |
| n | für die Zahlen 0, 1 oder 2 steht, |

deren geometrische und optische Isomeren und Isomerengemische.

**2.** (2-Cyan-2-oximinoacetyl)-aminosäure-Derivate gemäß Anspruch 1, worin in der Formel (I)

| | |
|---|---|
| R | für geradkettiges oder verzweigtes Alkyl mit 1 bis 4 Kohlenstoffatomen, geradkettiges oder verzweigtes Cyanalkyl mit 1 bis 4 Kohlenstoffatomen in Alkylteil, jeweils geradkettiges oder verzweigtes Alkenyl oder Alkinyl mit 2 bis 6 Kohlenstoffatomen, jeweils gegebenenfalls ein- bis fünffach, gleich oder verschieden, durch Alkyl mit 1 bis 4 Kohlenstoffatomensubstituiertes Cycloalkyl oder Cycloalkylalkyl mit jeweils 3 bis 6 Kohlenstoffatomen je Cycloalkylteil und 1 bis 4 Kohlenstoffatomen aus geradkettigen oder verzweigten Alkylteil oder für jeweils gegebenenfalls ein- bis fünffach, gleich oder verschieden substituiertes Benzyl oder Phenethyl steht, wobei als Phenylsubstituenten jeweils genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; |
| $R^1$ | für die Gruppierungen -A-COOR$^2$ oder -B-CONR$^3$R$^4$ steht, |
| B | für eine geradkettige oder verzweigte Alkylenkette mit 1 bis 4 Kohlenstoffatomen steht, |
| $R^3$ | für ein- oder zweifach substituiertes, geradkettiges oder verzweigtes Alkyl mit 1 bis 4 Kohlenstoffatomen steht, wobei als Substituenten genannt seien: Halogen, Cyano, -COOR$^I$,-CONR$^{II}$R$^{III}$, -NR$^{II}$R$^{III}$, -OR$^{IV}$, -S(O)$_n$R$^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, |

gleich oder verschieden, durch Halogen sowie Alkyl und Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden, durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner vorzugsweise für geradkettiges oder verzweigtes Alkenyl oder Alkinyl mit jeweils 2 mit 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden, substituiertes Cycloalkyl mit 3 bis 6 Kohlenstoffatomen, wobei als Substituenten genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen in geradkettigen oder verzweigten Alkylteil, gegebenenfalls ein- bis fünffach, gleich oder verschieden, durch Halogen und Alkyl mit 1 bis 4 Kohlenstoffatomen substituiertes Phenyl und Pyrrolidon: $R^3$ steht weiterhin vorzugsweise für gegebenenfalls ein- bis fünffach, gleich oder verschieden durch Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatom substituiertes Phenyl oder für einen gegebenenfalls ein- bis fünffach, gleich oder verschieden, substituierten 5- oder 6-gliedrigen Heterocyclus mit 1 bis 3 gleichen oder verschiedenen Heteroatomen, als Substituenten seien genannt: Halogen, Mercapto, Phenyl, geradkettiges oder verzweigtes Alkyl, Alkoxy, Alkylthio, Alkinsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, $-S(O)_nR^{IV}$, gegebenenfalls ein- bis fünffach, gleich oder verschieden durch Halogen, geradkettiges oder verzweigtes Alkyl und Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis fünffach, gleich oder verschieden durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis fünffach, gleich oder verschieden, durch Halogen, Mercapto, Phenyl, jeweils geradkettigen oder verzweigtes Alkyl, Alkoxy, Alkylthio, Alkylsulfinyl und Alkylsulfonyl mit 1 bis 4 Kohlenstoffatomen je Alkylteil substituierten 5- oder 6-gliedrigen Heterocyclus mit 1 bis 3 gleichen oder verschiedenen Heteroatomen, für ein- bis fünffach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkylalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen steht, wobei gegebenenfalls Halogen und geradkettiges oder verzweigtes Alkyl mit 1 bis 4 Kohlenstoffatomen als zusätzliche Cycloalkylsubstituenten genannt seinen, ferner für polycyclische Carbocyclen oder für $-OR^{IV}$ steht,

oder

$R^3$ und $R^4$ gemeinsam mit dem Stickstoffatom, an das sie gebunden sind, für einen ein- bis fünffach, gleich oder verschieden substituierten monocyclischen Heterocyclus oder für einen gegebenenfalls ein- bis fünffach, gleich oder verschieden substituierten bi- oder

tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.
Als Substituenten für alle Heterosysteme seien genannt:
geradkettiges oder verzweigtes Alkyl und Alkoxy mit jeweils 1 bis 4 Kohlenstoffatomen, Cycloalkyl mit 3 bis 6 Kohlenstoffatomen, die Hydroxy- oder Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl-oder Dialkylcarbamoyl mit jeweils 1 bis 4 Kohlenstoffatomen in jedem Alkylteil, sowie jeweils gegebenenfalls ein- oder zweifach, gleich oder verschieden, durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; $R^{II}$ ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen steht,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{III}$ ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen steht;

$R^{IV}$ für Wasserstoff, geradkettiges oder verzweigtes Alkyl mit 1 bis 6 Kohlenstoffatomen, gegebenenfalls ein- bis fünffach, gleich oder verschieden, substituiertes Phenylalkyl mit 1 bis 4 Kohlenstoffatomen im geradkettigen oder verzweigten Alkylteil steht, wobei als Phenylsubstituenten vorzugsweise die bei $R^{II}$ genannten Phenylsubstituenten infrage kommen; $R^{IV}$ ferner für Acyl mit 2 bis 9 Kohlenstoffatomen steht und

n für die Zahlen 0,1 odeer 2 steht,

deren geometrische und optischen Isomeren und Isomerengemische.

**3.** (2-Cyan-2-oximinoacetyl)-aminosäure-Derivate gemäß Anspruch 1, worin in der Formel (I)

R für Alkyl mit 1 oder 2 Kohlenstoffatomen, für Cyanmethyl oder Cyanethyl, für Allyl oder Propargyl, für jeweils gegebenenfalls ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden, durch Halogen und Methyl substituiertes Benzyl und Phenethyl steht,

$R^1$ für die Gruppierung $-B-CONR^3R^4$ steht,

B für die Gruppierungen $-CH_2-$, $-CH(CH_3)-$ oder $-CH_2CH_2-$ steht,

$R^3$ für ein- oder zweifach substituiertes geradkettiges oder verzweigtes Alkyl mit 1 bis 4 Kohlenstoffatomen steht, wobei als Substituenten vorzugsweise genannt seien: Halogen, Cyano, $-COOR^I$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen

Heterocyclus mit 1 bis 3 gleichen oder verschiedenen Heteroatomen, jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen, für gegebenenfalls durch geradkettiges oder verzweites Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden, durch Halogen und Alkyl mit 1 bis 4 Kohlenstoffatomen substituiertes Phenyl und Pyrrolidon; $R^3$ steht weiterhin für gegebenenfalls ein- bis dreifach, gleich oder verschieden, durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen. Als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, $-S(O)_nR^{IV}$, gegebenenfalls ein- bis dreifach, gleich oder verschieden durch Halogen, geradkettiges oder verzweigtes Alkyl und Alkoxy mit jeweils 1 bis 4 Kohlenstoffatomen substituierten 5- oder 6-gliedriger Heterocyclus mit 1 bis 3 Heteroatomen, jeweils gegebenenfalls ein- bis dreifach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis dreifach, gleich oder verschieden, durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis dreifach, gleich oder verschieden durch Halogen, Mercapto, Phenyl, jeweils geradkettiges oder verzweigtes Alkyl, Alkoxy, Alkylthio, Alkylsulfinyl und Alkylsulfonyl mit je 1 bis 4 Kohlenstoffatomen je Alkylteil substituierten 5- oder 6-gliedrigen Heterocyclus mit 1 bis 3 gleichen oder verschiedenen Heteroatomen, für ein- bis dreifach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkylalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen, wobei gegebenenfalls Halogen und geradkettiges oder verzweigtes Alkyl mit 1 bis 4 Kohlenstoffatomen als zusätzliche Cycloalkylsubstituenten genannt seien, ferner für polycyclische Carbocyclen oder für $-OR^{IV}$ steht,

oder

$R^3$ und $R^4$ gemeinsam mit dem Stickstoffatom, an das sie gebunden sind, für einen ein- bis dreifach, gleich oder verschieden substituierten monocyclischen Heterocyclus oder für einen gegebenenfalls ein- bis dreifach, gleich oder verschieden substituierten bi- oder tricyclischen Heterocyclus oder Spiroheterocyclus stehen, wobei als Heterocyclen genannt seinen: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.
Als Substituenten für alle Heterosysteme seien genannt:
geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl-oder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden, substituiertes Benzyl und Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden, durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ ferner für Acyl mit 2 bis 9 Kohlenstoffatomen steht und

n für die Zahlen 0, 1 oder 2 steht,

deren geometrische und optische Isomeren und Isomerengemische.

**4.** (2-Cyan-2-oximinoacetyl)-aminosäure-Derivate gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^3$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;
$R^3$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch

Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

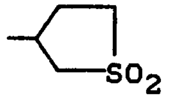 ,

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^4$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-oder Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^4$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden, durch Mercapto, geradkettiges oder verzweigtes Alkyl mit 1 bis 4 Kohlenstoffatomen, jeweils geradkettiges oder verzweigtes Alkythio, Alkylsulfinyl oder Alkylsulfonyl mit jeweils 1 bis 4 Kohlenstoffatomen substituiertes 1,3,4-Thiadiazol-5-yl oder für die Gruppierung

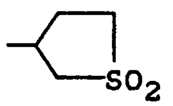 ,

$R^4$ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1-oder 2-Tetralyl.

**5.** (2-Cyan-2-oximinoacetyl)-aminosäure-Derivate gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CO-NR^3R^4$ steht,

B für die Gruppierung $-CH_2-$, $-CH(CH_3)-$ oder $-CH_2CH_2-$ steht,

$R^3$ für Wasserstoff oder geradkettiges oder verzweigtes Alkyl mit 1 bis 4 Kohlenstoffatomen steht,

$R^4$ für substituiertes Alkyl mit 1 bis 3 Kohlenstoffatomen steht, wobei als Substituenten genannt seien: Hydroxy, Alkylcarbonyloxy mit 1 bis 4 Kohlenstoffatomen in geradkettigen oder verzweigten Alkylteil, Alkylcarbonyl mit 1 bis 4 Kohlenstoffatomen im Alkylteil, Alkylthio mit 1 oder 2 Kohlenstoffatomen, 2-Furyl, 2-Pyridyl, 1-Morpholino, Cyclopropyl und Cyclohexyl; $R^4$ steht ferner für 1-Cyclohexenyl, für jeweils gegebenenfalls ein- bis dreifach durch Methyl substituiertes 2-Pyridyl oder 2-Pyrimidyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch

Phenyl substituiertes 1,2,4-Thiadiazol-5-yl, für gegebenenfalls ein- bis dreifach, gleich oder verschieden durch Mercapto und Alkyl, Alkylthio, Alkylsulfinyl und Alkylsulfonyl mit jeweils 1 bis 4 Kohlenstoffatomen substituiertes 1,3,4-Thiadiazol-5-yl, für die Gruppierung

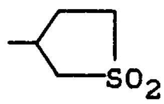 ,

für
1-Adamantyl, 2-Norbornyl, 1-oder 2-Decalyl oder 1- oder 2-Tetralyl; $R^4$ steht außerdem für ein- bis dreifach, gleich oder verschieden substituiertes Cyclohexyl, wobei als Substituenten genannt seien: Phenyl, Cyano, Hydroxy, Alkoxy mit 1 oder 2 Kohlenstoffatomen, Alkylamino und Dialkylamino mit jeweils 1 oder 2 Kohlenstoffatomen in jedem Alkylteil, Alkylcarbamoyl und Dialkylcarbamoyl mit jeweils 1 oder 2 Kohlenstoffatomen in jedem Alkylteil, Alkoxycarbonylamino mit 1 oder 2 Kohlenstoffatomen im Alkylteil, Cyclohexyl, Cyclohexylalkyl mit 1 oder 2 Kohlenstoffatomen im Alkylteil und 1-Pyrrolidin-2-on und zusätzlich Halogen und Alkyl mit 1 oder 2 Kohlenstoffatomen.

**6.** (2-Cyan-2-oximinoacetyl)-aminosäure-Derivate gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Grupierung -B-CO-$NR^3R^4$ steht,

B für die Gruppierungen $-CH_2-$, $-CH(CH_3)-$ oder $-CH_2CH_2-$ steht,

$R^3$ und $R^4$ gemeinsam mit dem Stickstoffatom, an das sie gebunden sind, für folgende mono-, bi- oder tricyclischen Heterocyclen oder Spiroheterocyclen stehen:

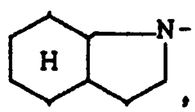 , 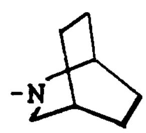 , 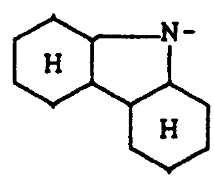 ,

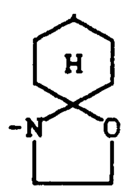 , 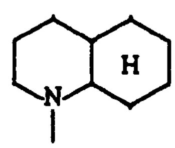 ,

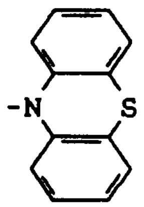 , 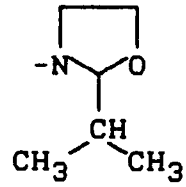 , 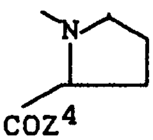

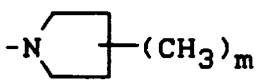 , 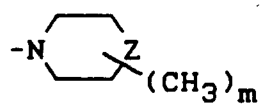

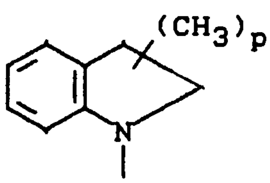 , 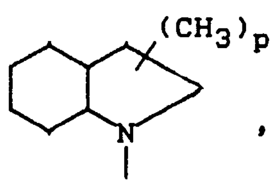 ,

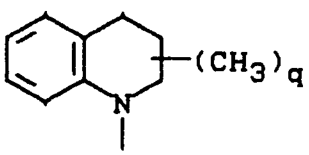 , 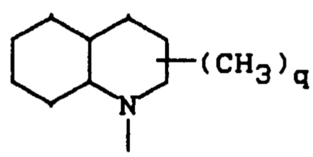 ,

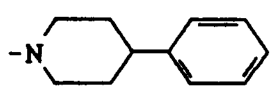 , 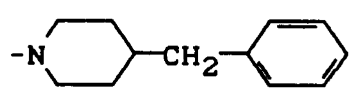 ,

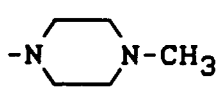 , 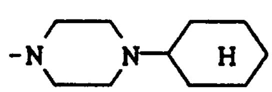 , 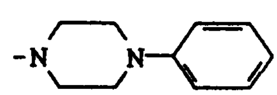 ,

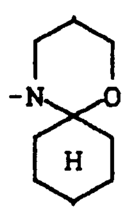 , 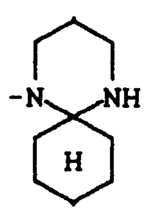 , 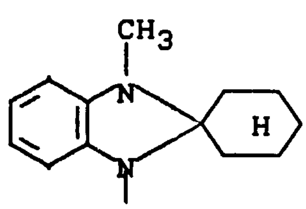 ,

wobei

| | |
|---|---|
| Z | für Sauerstoff oder die $CH_2$-Gruppe steht, |
| $Z^4$ | für Hydroxy, Methoxy, Ethoxy, Amino, Methylamino, Ethylamino, Dimethylamino oder Ethylmethylamino steht, |
| m | für die Zahlen 1, 2, 3 oder 4 steht, |
| p | für die Zahlen 0, 1 oder 2 steht und |
| q | für die Zahlen 0, 1, 2 oder 3 steht. |

**7.** Verfahren zur Herstellung von (2-Cyan-2-oximino-acetyl)-aminosäure-Derivaten der allgemeinen Formel (I),

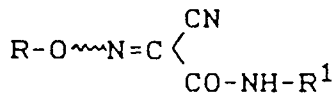

(I)

in welcher

- $R$ für Alkyl, Cyanalkyl oder jeweils gegebenenfalls substituiertes Alkenyl, Alkinyl, Cycloalkyl, Cycloalkylalkyl oder Phenylalkyl steht,
- $R^1$ für die Gruppierungen $-B-CONR^3R^4$ steht, wobei
- $B$ für eine gegebenenfalls substituierte, geradkettige oder verzweigte Alkylenkette steht,
- $R^3$ für substituiertes Alkyl steht mit folgenden Substituenten : Halogen, Cyano, $-COOR^{I}$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht und
- $R^4$ für substituiertes Alkyl steht mit folgenden Substituenten: Halogen, Cyano, $-COOR^{I}$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht, oder
- $R^3$ für Wasserstoff oder Alkyl steht und
- $R^4$ für substituiertes Alkyl steht mit den folgenden Substituenten: Acyl, Hydroxy, Alkylcarbonyloxy, $-S(O)_nR^{IV}$, jeweils gegebenenfalls substituiertes Heterocyclyl, Cycloalkyl und Cycloalkenyl; $R^4$ ferner für jeweils gegebenenfalls substituiertes Cycloalkenyl, Heterocyclyl oder polycyclische Carbocyclen, für substituiertes Cycloalkyl, wobei jedoch Halogen und Alkyl nicht als alleinige Substituenten vorhanden sein dürfen, oder für die Gruppierung $-OR^{IV}$ steht,

  oder
- $R^3$ und $R^4$ gemeinsam mit dem Stickstoffatom, an das sie gebunden sind, für einen substituierten monocyclischen Heterocyclus oder für einen gegebenenfalls substituierten bi- oder tricyclischen Heterocyclus oder Spiroheterocyclus mit 1 bis 3 weiterhin, gleichen oder verschiedenen Heteroatomen stehen,
- $R^{I}$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,
- $R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^{I}R^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,
- $R^{IV}$ für Wasserstoff, Alkyl, Aralkyl oder Acyl steht und
- $n$ für die Zahlen 0, 1 oder 2 steht,

deren geometrische und optische Isomeren und Isomerengemische, dadurch gekennzeichnet, daß man

a) 2-Cyan-2-oximino-essigsäureester der allgemeinen Formel (II),

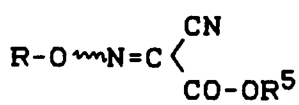

(II)

in welcher

- $R$ die oben angegebene Bedeutung hat und
- $R^5$ für Alkyl steht,

mit Aminen der Formel (III),

$R^1-NH_2$ (III)

in welcher

$R^1$ die oben angegebene Bedeutung hat,

gegebenenfalls in Gegenwart eines Verdünnungsmittels und gegebenenfalls in Gegenwart einer Base umsetzt;

oder

b) carboxy-aktivierte Derivate von Carbonsäuren der allgemeinen Formel (IV),

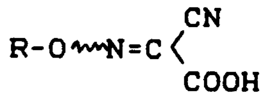 (IV)

in welcher

R die oben angegebene Bedeutung hat,

mit Aminen der Formel (III),

$R^1-NH_2$ (III)

in welcher

$R^1$ die oben angegebene Bedeutung hat,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

c) 2-Cyan-2-oximino-acetamide der allgemeinen Formel (V),

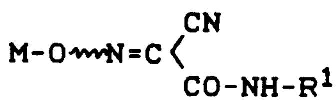 (V)

in welcher

M für Wasserstoff, ein Alkalimetallion, eine protonierte tertiäre Base oder ein quaternäres Ammoniumion steht und

$R^1$ die oben angegebene Bedeutung hat,

mit Verbindungen der Formel (VI),

R-X (VI)

in welcher

X für Halogen oder einen Sulfonyloxy-Rest steht und

R die oben angegebene Bedeutung hat,

gegebenenfalls in Gegenwart einer Base und gegebenenfalls in Gegenwart eines Verdünnungsmittels umsetzt;

oder

d) substituierte Acetyl-aminosäureester der allgemeinen Formel (VII),

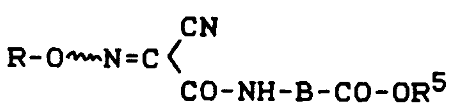 (VII)

in welcher

B, R und $R^5$ die oben angegebene Bedeutung haben,

mit Aminen der Formel (VIII),

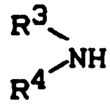

(VIII)

in welcher

$R^3$ und $R^4$ die oben angegebene Bedeutung haben,

gegebenenfalls in Gegenwart eines Verdünnungsmittels und gegebenenfalls in Gegenwart einer Base umsetzt;

oder

e) carboxy-aktivierte Acetyl-aminosäuren der allgemeinen Formel (IX),

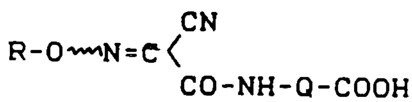

(IX)

in welcher

R die oben angegebene Bedeutung hat und

Q für A oder B steht, wobei diese die oben angegebene Bedeutung haben,

mit Verbindungen der Formel (X),

Y-H (X)

in welcher

Y für die Gruppierungen $R^2O$- oder $R^3R^4N$-steht, wobei $R^2$, $R^3$ und $R^4$ die oben angegebene Bedeutung haben,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

f) Ammoniumsalze von 2-Cyan-2-oximino-essigsäure der allgemeinen Formel (XI),

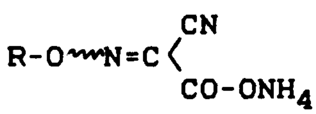

(XI)

in welcher

R die oben angegebene Bedeutung hat,

mit Aldehyden der Formel (XII),

$R^6$-CH=O (XII)

in welcher

die Gruppierung $R^6$-CH= für die oben genannten Bedeutungen von A oder B steht,

und mit Isonitrilen der Formel (XIII),

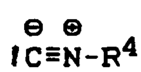

(XIII)

in welcher

$R^4$ die oben angegebene Bedeutung hat,

gegebenenfalls in Gegenwart eines Verdünnungsmittels umsetzt;

oder

g) 2-Cyan-2-oximino-essigsäuren der allgemeinen Formel (IV),

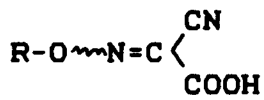

(IV)

in welcher

R die oben angegebene Bedeutung hat,

mit Isocyanaten der Formel (XIV),

$R^1$-NCO (XIV)

in welcher

$R^1$ die oben angegebene Bedeutung hat,

gegebenenfalls in Gegenwart eines Katalysators und gegebenenfalls in Gegenwart eines Verdünnungsmittels umsetzt;

oder

h) 2-Cyano-2-oximino-acetamide der allgemeinen Formel (XV),

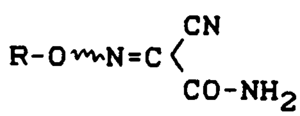

(XV)

in welcher

R die oben angegebene Bedeutung hat,

mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher

$R^1$ und X die oben angegebene Bedeutung haben,

gegebenenfalls in Gegenwart eines Verdünnungsmittels umsetzt.

**8.** Schädlingsbekämpfungsmittel, gekennzeichnet durch einen Gehalt an mindestens einem (2-Cyano-2-oximino-acetyl)-aminosäure-Derivat der Formel (I) nach den Ansprüchen 1 und 7.

**9.** Verwendung von (2-Cyano-2-oximino-acetyl)-aminosäure-Derivaten der Formel (I) nach den Ansprüchen 1 und 7 zur Bekämpfung von Schädlingen.

**10.** Verfahren zur Bekämpfung von Schädlingen, dadurch gekennzeichnet, daß man (2-Cyano-2-oximino-acetyl)-aminosäure-Derivate der Formel (I) nach den Ansprüchen 1 und 7 auf Schädlinge und/oder ihren Lebensraum einwirken läßt.

**11.** Verfahren zur Herstellung von Schädlingsbekämpfungsmitteln, dadurch gekennzeichnet, daß man (2-Cyano-2-oximino-acetyl)-aminosäure-Derivate der Formel (I) nach den Ansprüchen 1 und 7 mit Streckmitteln und/oder oberflächenaktiven Mitteln vermischt.

**Patentansprüche für folgenden Vertragsstaat : ES**

**1.** Verfahren zur Herstellung von (2-Cyan-2-oximino-acetyl)-aminosäure-Derivaten der allgemeinen Formel (I),

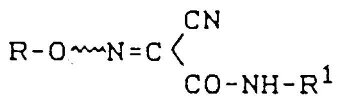

(I)

in welcher

R für Alkyl, Cyanalkyl oder jeweils gegebenenfalls substituiertes Alkenyl, Alkinyl, Cycloalkyl, Cycloalkylalkyl oder Phenylalkyl steht,

$R^1$ für die Gruppierungen $-B-CONR^3R^4$ steht, wobei

B für eine gegebenenfalls substituierte, geradkettige oder verzweigte Alkylenkette steht,

$R^3$ für substituiertes Alkyl steht mit folgenden Substituenten : Halogen, Cyano, $-COOR^I$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht und

$R^4$ für substituiertes Alkyl steht mit folgenden Substituenten: Halogen, Cyano, $-COOR^I$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,

oder

$R^3$ für Wasserstoff oder Alkyl steht und

$R^4$ für substituiertes Alkyl steht mit den folgenden Substituenten: Acyl, Hydroxy, Alkylcarbonyloxy, $-S(O)_nR^{IV}$, jeweils gegebenenfalls substituiertes Heterocyclyl, Cycloalkyl und Cycloalkenyl; $R^4$ ferner für jeweils gegebenenfalls substituiertes Cycloalkenyl, Heterocyclyl oder polycyclische Carbocyclen, für substituiertes Cycloalkyl, wobei jedoch Halogen und Alkyl nicht als alleinige Substituenten vorhanden sein dürfen, oder für die Gruppierung $-OR^{IV}$ steht,

oder

$R^3$ und $R^4$ gemeinsam mit dem Stickstoffatom, an das sie gebunden sind, für einen substituierten monocyclischen Heterocyclus oder für einen gegebenenfalls substituierten bi- oder tricyclischen Heterocyclus oder Spiroheterocyclus mit 1 bis 3 weiteren, gleichen oder verschiedenen Heteroatomen stehen,

$R^I$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ für Wasserstoff, Alkyl, Aralkyl oder Acyl steht und

n für die Zahlen 0, 1 oder 2 steht,

deren geometrische und optische Isomeren und Isomerengemische, dadurch gekennzeichnet, daß man

a) 2-Cyan-2-oximino-essigsäureester der allgemeinen Formel (II),

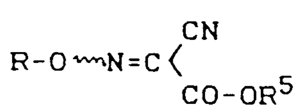

(II)

in welcher

R die oben angegebene Bedeutung hat und

$R^5$ für Alkyl steht,

mit Aminen der Formel (III),

$R^1$-$NH_2$ (III)

in welcher

$R^1$ die oben angegebene Bedeutung hat,

gegebenenfalls in Gegenwart eines Verdünnungsmittels und gegebenenfalls in Gegenwart einer Base umsetzt;

oder

b) carboxy-aktivierte Derivate von Carbonsäuren der allgemeinen Formel (IV),

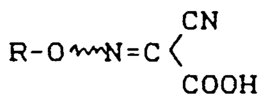

(IV)

in welcher

R die oben angegebene Bedeutung hat,

mit Aminen der Formel (III),

$R^1$-$NH_2$ (III)

in welcher

$R^1$ die oben angegebene Bedeutung hat,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

c) 2-Cyan-2-oximino-acetamide der allgemeinen Formel (V),

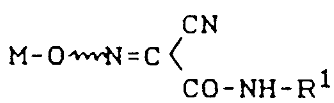

(V)

in welcher

M für Wasserstoff, ein Alkalimetallion, eine protonierte tertiäre Base oder ein quarternäres Ammoniumion steht und

$R^1$ die oben angegebene Bedeutung hat,

mit Verbindungen der Formel (VI),

R-X (VI)

in welcher

X für Halogen oder einen Sulfonyloxy-Rest steht und

R die oben angegebene Bedeutung hat,

gegebenenfalls in Gegenwart einer Base und gegebenenfalls in Gegenwart eines Verdünnungsmittels umsetzt;

oder

d) substituierte Acetyl-aminosäureester der allgemeinen Formel (VII),

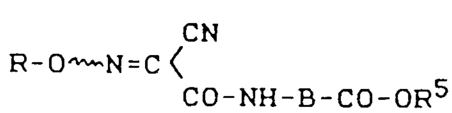

(VII)

in welcher

B, R und $R^5$ die oben angegebene Bedeutung haben,

mit Aminen der Formel (VIII),

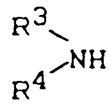

(VIII)

in welcher

$R^3$ und $R^4$ die oben angegebene Bedeutung haben,

gegebenenfalls in Gegenwart eines Verdünnungsmittels und gegebenenfalls in Gegenwart einer Base umsetzt;

oder

e) carboxy-akivierte Acetyl-aminosäuren der allgemeinen Formel (IX),

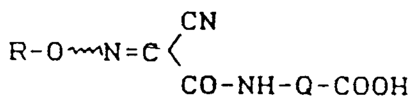

(IX)

in welcher

R die oben angegebene Bedeutung hat und

Q für A oder B steht, wobei diese die oben angegebene Bedeutung haben,

mit Verbindungen der Formel (X),

Y-H (X)

in welcher

Y für die Gruppierungen $R^2O$- oder $R^3R^4N$-steht, wobei $R^2$, $R^3$ und $R^4$ die oben angegebene Bedeutung haben,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

f) Ammoniumsalze von 2-Cyan-2-oximino-essigsäure der allgemeinen Formel (XI),

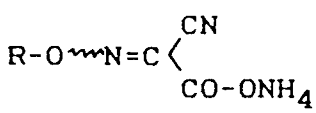

(XI)

in welcher

R die oben angegebene Bedeutung hat,

mit Aldehyden der Formel (XII),

$R^6$-CH = O (XII)

in welcher

die Gruppierung $R^6$-CH = für die oben genannten Bedeutungen von A oder B steht,

und mit Isonitrilen der Formel (XIII),

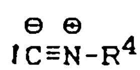

(XIII)

in welcher

$R^4$ die oben angegebene Bedeutung hat,

gegebenenfalls in Gegenwart eines Verdünnungsmittels umsetzt;

oder

g) 2-Cyan-2-oximino-essigsäuren der allgemeinen Formel (IV),

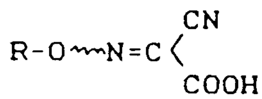 (IV)

in welcher

R die oben angegebene Bedeutung hat,

mit Isocyanaten der Formel (XIV),

$R^1$-NCO (XIV)

in welcher

$R^1$ die oben angegebene Bedeutung hat,

gegebenenfalls in Gegenwart eines Katalysators und gegebenenfalls in Gegenwart eines Verdünnungsmittels umsetzt;

oder

h) 2-Cyano-2-oximino-acetamide der allgemeinen Formel (XV),

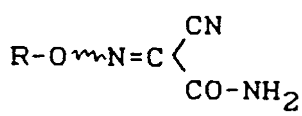 (XV)

in welcher

R die oben angegebene Bedeutung hat,

mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher

$R^1$ und X die oben angegebene Bedeutung haben,

gegebenenfalls in Gegenwart eines Verdünnungsmittels umsetzt.

**2.** Verfahren gemäß Anspruch 1, worin in der Formel (I)

R für geradkettiges oder verzweigtes Alkyl mit 1 bis 4 Kohlenstoffatomen, geradkettiges oder verzweigtes Cyanalkyl mit 1 bis 4 Kohlenstoffatomen im Alkylteil, jeweils geradkettiges oder verzweigtes Alkenyl oder Alkinyl mit 2 bis 6 Kohlenstoffatomen, jeweils gegebenenfalls ein- bis fünffach, gleich oder verschieden, durch Alkyl mit 1 bis 4 Kohlenstoffatomen substituiertes Cycloalkyl oder Cycloalkylalkyl mit jeweils 3 bis 6 Kohlenstoffatomen je Cycloalkylteil und 1 bis 4 Kohlenstoffatomen aus geradkettigen oder verzweigten Alkylteil oder für jeweils gegebenenfalls ein- bis fünffach, gleich oder verschieden substituiertes Benzyl oder Phenethyl steht, wobei als Phenylsubstituenten jeweils genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen;

$R^1$ für die Gruppierungen -A-$COOR^2$ oder -B-$CONR^3R^4$ steht,

B für eine geradkettige oder verzweigte Alkylenkette mit 1 bis 4 Kohlenstoffatomen steht,

$R^3$ für ein- oder zweifach substituiertes, geradkettiges oder verzweigtes Alkyl mit 1 bis 4 Kohlenstoffatomen steht, wobei als Substituenten genannt seien: Halogen, Cyano, -$COOR^{I}$,-$CONR^{II}R^{III}$, -$NR^{II}R^{III}$, -$OR^{IV}$, -$S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach,

gleich oder verschieden, durch Halogen sowie Alkyl und Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden, durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner vorzugsweise für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden, substituiertes Cyloalkyl mit 3 bis 6 Kohlenstoffatomen, wobei als Substituenten genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden, durch Halogen und Alkyl mit 1 bis 4 Kohlenstoffatomen substituiertes Phenyl und Pyrrolidon; $R^3$ steht weiterhin vorzugsweise für gegebenenfalls ein- bis fünffach, gleich oder verschieden durch Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden, substituierten 5- oder 6-gliedrigen Heterocyclus mit 1 bis 3 gleichen oder verschiedenen Heteroatomen, als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, $-S(O)_nR^{IV}$, gegebenenfalls ein- bis fünffach, gleich oder verschieden durch Halogen, geradkettiges oder verzweigtes Alkyl und Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis fünffach, gleich oder verschieden durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis fünffach, gleich oder verschieden, durch Halogen, Mercapto, Phenyl, jeweils geradkettiges oder verzweigtes Alkyl, Alkoxy, Alkylthio, Alkylsulfinyl und Alkylsulfonyl mit 1 bis 4 Kohlenstoffatomen je Alkylteil substituierten 5- oder 6-gliedrigen Heterocyclus mit 1 bis 3 gleichen oder verschiedenen Heteroatomen, für ein- bis fünffach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkylalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen steht, wobei gegebenenfalls Halogen und geradkettiges oder verzweigtes Alkyl mit 1 bis 4 Kohlenstoffatomen als zusätzliche Cycloalkylsubstituenten genannt seien, ferner für polycyclische Carbocyclen oder für $-OR^{IV}$ steht,

oder

$R^3$ und $R^4$ gemeinsam mit dem Stickstoffatom, an das sie gebunden sind, für einen ein- bis fünffach, gleich oder verschieden substituierten monocyclischen Heterocyclus oder für einen gegebenenfalls ein- bis fünffach, gleich oder verschieden substituierten bi- oder

tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl und Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, sowie jeweils gegebenenfals ein- oder zweifach, gleich oder verschieden, durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; $R^{II}$ ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen steht,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{III}$ ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen steht;

$R^{IV}$ für Wasserstoff, geradkettiges oder verzweigtes Alkyl mit 1 bis 6 Kohlenstoffatomen, gegebenenfalls ein- bis fünffach, gleich oder verschieden, substituiertes Phenylalkyl mit 1 bis 4 Kohlenstoffatomen im geradkettigen oder verzweigten Alkylteil steht, wobei als Phenylsubstituenten vorzugsweise die bei $R^{II}$ genannten Phenylsubstituenten infrage kommen; $R^{IV}$ ferner für Acyl mit 2 bis 9 Kohlenstoffatomen steht und

n für die Zahlen 0,1 odeer 2 steht,

deren geometrische und optischen Isomeren und Isomerengemische.

**3.** Verfahren gemäß Anspruch 1, worin in der Formel (I)

R für Alkyl mit 1 oder 2 Kohlenstoffatomen, für Cyanmethyl oder Cyanethyl, für Allyl oder Propargyl, für jeweils gegebenenfalls ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden, durch Halogen und Methyl substituiertes Benzyl und Phenethyl steht,

$R^1$ für die Gruppierung $-B-CONR^3R^4$ steht,

B für die Gruppierungen $-CH_2-$, $-CH(CH_3)-$ oder $-CH_2CH_2-$ steht,

$R^3$ für ein- oder zweifach substituiertes geradkettiges oder verzweigtes Alkyl mit 1 bis 4 Kohlenstoffatomen steht, wobei als Substituenten vorzugsweise genannt seien: Halogen, Cyano, $-COOR^I$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen

Heterocyclus mit 1 bis 3 gleichen oder verschiedenen Heteroatomen, jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden, durch Halogen und Alkyl mit 1 bis 4 Kohlenstoffatomen substituiertes Phenyl und Pyrrolidon; $R^3$ steht weiterhin für gegebenenfalls ein- bis dreifach, gleich oder verschieden, durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen. Als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

$R^3$ für Wasserstoff oder geradkettiges oder verzweigtes Alkyl mit 1 bis 4 Kohlenstoffatomen steht und

$R^4$ für ein- oder zweifach substituiertes, geradkettiges oder verzweigtes Alkyl mit 1 bis 4 Kohlenstoffatomen steht mit den folgenden Substituenten: Acyl mit 2 bis 9 Kohlenstoffatomen, Hydroxy, Alkylcarbonyloxy mit 1 bis 4 Kohlenstoffatomen im geradkettigen oder verzweigten Alkylteil, $-S(O)_nR^{IV}$, gegebenenfalls ein- bis dreifach, gleich oder verschieden durch Halogen, geradkettiges oder verzweigtes Alkyl und Alkoxy mit jeweils 1 bis 4 Kohlenstoffatomen substituierten 5- oder 6-gliedriger Heterocyclus mit 1 bis 3 Heteroatomen, jeweils gegebenenfalls ein- bis dreifach, gleich oder verschieden durch Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen;

$R^4$ ferner für gegebenenfalls ein- bis dreifach, gleich oder verschieden, durch geradkettiges oder verzweigtes Alkyl mit 1 bis 4 Kohlenstoffatomen substituiertes Cycloalkenyl mit 5 bis 7 Kohlenstoffatomen, für einen gegebenenfalls ein- bis dreifach, gleich oder verschieden durch Halogen, Mercapto, Phenyl, jeweils geradkettiges oder verzweigtes Alkyl, Alkoxy, Alkylthio, Alkylsulfinyl und Alkylsulfonyl mit je 1 bis 4 Kohlenstoffatomen je Alkylteil substituierten 5- oder 6-gliedrigen Heterocyclus mit 1 bis 3 gleichen oder verschiedenen Heteroatomen, für ein- bis dreifach, gleich oder verschieden durch Phenyl, Cyan, Hydroxy, Alkoxy, Alkylamino, Dialkylamino, Alkylcarbamoyl, Dialkylcarbamoyl, Alkoxycarbonylamino mit jeweils 1 bis 4 Kohlenstoffatomen je Alkylteil, Cycloalkyl und Cycloalkylalkyl mit jeweils 5 bis 7 Kohlenstoffatomen im Cycloalkylteil und 1 bis 4 Kohlenstoffatomen im Alkylteil substituiertes Cycloalkyl mit 3 bis 6 Kohlenstoffatomen, wobei gegebenenfalls Halogen und geradkettiges oder verzweigtes Alkyl mit 1 bis 4 Kohlenstoffatomen als zusätzliche Cycloalkylsubstituenten genannt seien, ferner für polycyclische Carbocyclen oder für $-OR^{IV}$ steht,

oder

$R^3$ und $R^4$ gemeinsam mit dem Stickstoffatom, an das sie gebunden sind, für einen ein- bis dreifach, gleich oder verschieden substituierten monocyclischen Heterocyclus oder für einen gegebenenfalls ein- bis dreifach, gleich oder verschieden substituierten bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden, substituiertes Benzyl und Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden, durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ ferner für Acyl mit 2 bis 9 Kohlenstoffatomen steht und

n für die Zahlen 0, 1 oder 2 steht,

deren geometrische und optische Isomeren und Isomerengemische.

**4.** Verfahren gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^3$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl- und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^3$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch

Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

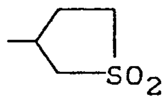 ,

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^4$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^4$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden, durch Mercapto, geradkettiges oder verzweigtes Alkyl mit 1 bis 4 Kohlenstoffatomen, jeweils geradkettiges oder verzweigtes Alkythio, Alkylsulfinyl oder Alkylsulfonyl mit jeweils 1 bis 4 Kohlenstoffatomen substituiertes 1,3,4-Thiadiazol-5-yl oder für die Gruppierung

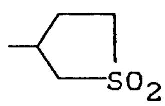 ,

$R^4$ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1-oder 2-Tetralyl.

**5.** Verfahren gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für Wasserstoff oder geradkettiges oder verzweigtes Alkyl mit 1 bis 4 Kohlenstoffatomen steht,

$R^4$ für substituiertes Alkyl mit 1 bis 3 Kohlenstoffatomen steht, wobei als Substituenten genannt seien: Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im Alkylteil, Alkylthio mit 1 oder 2 Kohlenstoffatomen, 2-Furyl, 2-Pyridyl, 1-Morpholino, Cyclopropyl und Cyclohexyl; $R^4$ steht ferner für 1-Cyclohexenyl, für jeweils gegebenenfalls ein- bis dreifach durch Methyl substituiertes 2-Pyridyl oder 2-Pyrimidyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch

Phenyl substituiertes 1,2,4-Thiadiazol-5-yl, für gegebenenfalls ein- bis dreifach, gleich oder verschieden durch Mercapto und Alkyl, Alkylthio, Alkylsulfinyl und Alkylsulfonyl mit jeweils 1 bis 4 Kohlenstoffatomen substituiertes 1,3,4-Thiadiazol-5-yl, für die Gruppierung

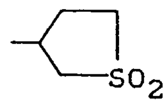 ,

für

1-Adamantyl, 2-Norbornyl, 1- oder 2-Decalyl oder 1- oder 2-Tetralyl; $R^4$ steht außerdem für ein- bis dreifach, gleich oder verschieden substituiertes Cyclohexyl, wobei als Substituenten genannt seien: Phenyl, Cyano, Hydroxy, Alkoxy mit 1 oder 2 Kohlenstoffatomen, Alkylamino und Dialkylamino mit jeweils 1 oder 2 Kohlenstoffatomen in jedem Alkylteil, Alkylcarbamoyl und Dialkylcarbamoyl mit jeweils 1 oder 2 Kohlenstoffatomen in jedem Alkylteil, Alkoxycarbonylamino mit 1 oder 2 Kohlenstoffatomen im Alkylteil, Cyclohexyl, Cyclohexylalkyl mit 1 oder 2 Kohlenstoffatomen im Alkylteil und 1-Pyrrolidin-2-on und zusätzlich Halogen und Alkyl mit 1 oder 2 Kohlenstoffatomen.

**6.** Verfahren gemäß Anspruch 1, worin in der Formel (I)

R für Methyl, Ethyl, Cyanmethyl, Cyanethyl, Allyl, Propargyl, für jeweils ein- bis dreifach durch Methyl substituiertes Cyclopropyl, Cyclohexyl, Cyclopropylmethyl oder Cyclohexylmethyl oder für jeweils gegebenenfalls ein- bis dreifach, gleich oder verschieden durch Halogen und Methyl substituiertes Benzyl oder Phenethyl steht,

$R^1$ für die Grupierung $-B-CO-NR^3R^4$ steht,

B für die Gruppierungen $-CH_2-$, $-CH(CH_3)-$ oder $-CH_2CH_2-$ steht,

$R^3$ und $R^4$ gemeinsam mit dem Stickstoffatom, an das sie gebunden sind, für folgende mono-, bi- oder tricyclischen Heterocyclen oder Spiroheterocyclen stehen:

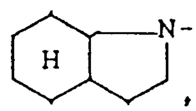 , 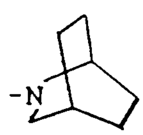 , 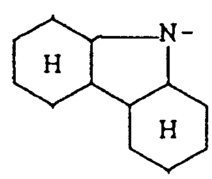 ,

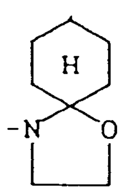 , 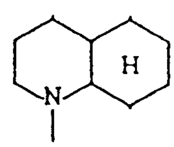 ,

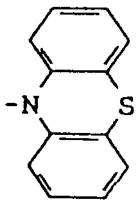 , 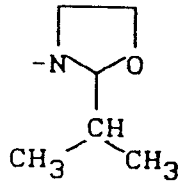 , 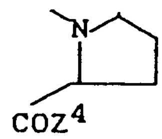

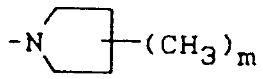 , 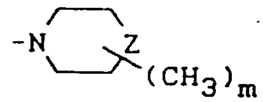

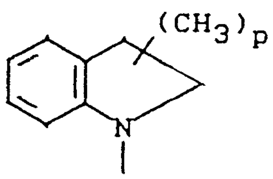 , 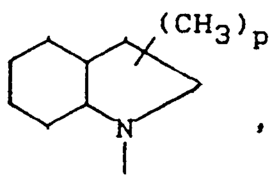 ,

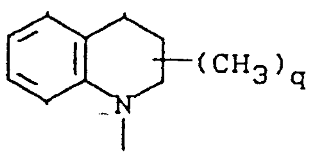 , 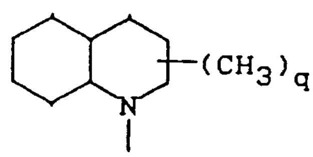 ,

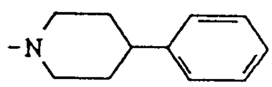 , 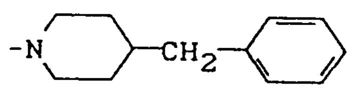 ,

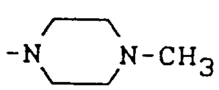 , 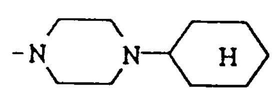 , 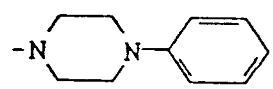 ,

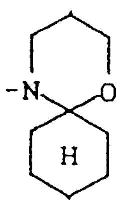 , 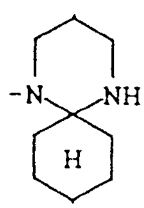 , 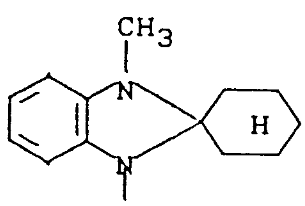 ,

wobei

Z für Sauerstoff oder die $CH_2$-Gruppe steht,

**7.** Schädlingsbekämpfungsmittel, gekennzeichnet durch einen Gehalt an mindestens einem (2-Cyano-2-oximino-acetyl)-aminosäure-Derivat der Formel (I) nach den Ansprüchen 1 und 6.

**8.** Verwendung von (2-Cyano-2-oximino-acetyl)-aminosäure-Derivaten der Formel (I) nach den Ansprüchen 1 und 6 zur Bekämpfung von Schädlingen.

**9.** Verfahren zur Bekämpfung von Schädlingen, dadurch gekennzeichnet, daß man (2-Cyano-2-oximino-acetyl)-aminosäure-Derivate der Formel (I) nach den Ansprüchen 1 und 6 auf Schädlinge und/oder ihren Lebensraum einwirken läßt.

**10.** Verfahren zur Herstellung von Schädlingsbekämpfungsmitteln, dadurch gekennzeichnet, daß man (2-Cyano-2-oximino-acetyl)-aminosäure-Derivate der Formel (I) nach den Ansprüchen 1 und 6 mit Streckmitteln und/oder oberflächenaktiven Mitteln vermischt`

## Claims

**Claims for the following Contracting States : AT, BE, CH, DE, FR, GB, GR, IT, LI, NL**

**1.** (2-Cyano-2-oximinoacetyl)-amino acid derivatives of the general formula (I)

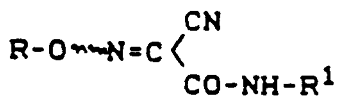

(I)

in which

- R represents alkyl, cyanoalkyl or in each case optionally substituted alkenyl, alkinyl, cycloalkyl, cycloalkylalkyl or phenylalkyl,
- $R^1$ represents the -B-$CONR^3R^4$ group, where
- B represents an optionally substituted, straight-chain or branched alkylene chain,
- $R^3$ represents substituted alkyl having the following substituents: halogen, cyano, -$COOR^I$, -$CONR^{II}R^{III}$, -$NR^{II}R^{III}$, -$OR^{IV}$, -$S(O)_nR^{IV}$, acyl, in each case optionally substituted aryl, heterocyclyl, cycloalkyl and cycloalkenyl; $R^3$ furthermore represents in each case optionally substituted alkenyl, alkinyl, cycloalkyl, cycloalkenyl, aryl or heterocyclyl, and
- $R^4$ represents substituted alkyl having the following substituents: halogen, cyano, -$COOR^I$, -$CONR^{II}R^{III}$, -$NR^{II}R^{III}$, -$OR^{IV}$, -$S(O)_nR^{IV}$, acyl, in each case optionally substituted aryl, heterocyclyl, cycloalkyl and cycloalkenyl; $R^4$ furthermore represents in each case optionally substituted alkenyl, alkinyl, cycloalkyl, cycloalkenyl, aryl, heterocyclyl or polycyclic carbocyclic rings or the -$OR^{IV}$ group,

  or
- $R^3$ represents hydrogen or alkyl, and
- $R^4$ represents substituted alkyl having the following substituents: acyl, hydroxyl, alkylcarbonyloxy, -$S(O)_nR^{IV}$, in each case optionally substituted heterocyclyl, cycloalkyl and cycloalkenyl; $R^4$ furthermore represents in each case optionally substituted cycloalkenyl, heterocyclyl or polycyclic carbocyclic rings, represents substituted cycloalkyl where, however, halogen and alkyl may not be present as the only substituents, or represents the -$OR^{IV}$ group,

  or
- $R^3$ and $R^4$, together with the nitrogen atom to which they are bound, represent a substituted, monocyclic heterocyclic ring or an optionally substituted bi- or tricyclic heterocyclic ring or spiroheterocyclic ring having 1 to 3 further identical or different heteroatoms,
- $R^I$ represents hydrogen or optionally substituted alkyl having the following substituents: halogen, in each case optionally substituted aryl, cycloalkyl and cycloalkenyl,
- $R^{II}$ and $R^{III}$ are identical or different and represent hydrogen or optionally substituted alkyl, having the following substituents: halogen, -$COOR^{IV}$, -$CONR^IR^{IV}$, in each case optionally substituted aryl, cycloalkyl and cycloalkenyl; $R^{II}$ and $R^{III}$ furthermore represent alkenyl, alkinyl or in each case optionally substituted cycloalkyl, cycloalkenyl, aryl or heterocyclyl, or, together with the nitrogen atom to which they are bound, represent an optionally substituted heterocyclic ring which may contain further heteroatoms,
- $R^{IV}$ represents hydrogen, alkyl, aralkyl or acyl, and
- n represents the numbers 0, 1 or 2,

and the geometrical and optical isomers and isomer mixtures thereof.

**2.** (2-Cyano-2-oximinoacetyl)-amino acid derivatives according to Claim 1, in which, in the formula (I),

- R represents straight-chain or branched alkyl having 1 to 4 carbon atoms, straight-chain or branched cyanoalkyl having 1 to 4 carbon atoms in the alkyl part, in each case straight-chain or branched alkenyl or alkinyl having 2 to 6 carbon atoms, cycloalkyl or

cycloalkylalkyl each of which has 3 to 6 carbon atoms per cycloalkyl part and 1 to 4 carbon atoms in the straight-chain or branched alkyl part and each of which is optionally monosubstituted to pentasubstituted by identical or different alkyl having 1 to 4 carbon atoms, or represents benzyl or phenethyl, each of which is optionally monosubstituted to pentasubstituted by identical or different substituents, phenyl substituents which may be mentioned in each case being: halogen, alkyl and alkoxy in each case having 1 to 4 carbon atoms, halogenoalkyl and halogenoalkoxy in each case having 1 or 2 carbon atoms and 2 to 5 identical or different halogen atoms;

$R^1$ represents the -A-COOR$^2$ or -B-CONR$^3$R$^4$ groups,

B represents a straight-chain or branched alkylene chain having 1 to 4 carbon atoms,

$R^3$ represents monosubstituted or disubstituted, straight-chain or branched alkyl having 1 to 4 carbon atoms, substituents which may be mentioned being: halogen, cyano, -COOR$^I$, -CONR$^{II}$R$^{III}$, -NR$^{II}$R$^{III}$, -OR$^{IV}$, -S(O)$_n$R$^{IV}$, acyl having 2 to 9 carbon atoms, phenyl which is optionally monosubstituted to pentasubstituted by identical or different substituents from the series comprising halogen and alkyl having 1 to 4 carbon atoms, a 5- or 6-membered heterocyclic ring having 1 to 3 heteroatoms, which is optionally monosubstituted to pentasubstituted by identical or different substituents from the series comprising halogen and alkyl and alkoxy in each case having 1 to 4 carbon atoms, cycloalkyl having 3 to 6 carbon atoms and cycloalkenyl having 5 to 7 carbon atoms each of which is optionally monosubstituted to pentasubstituted by identical or different alkyl having 1 to 4 carbon atoms; R$^3$ furthermore preferably represents straight-chain or branched alkenyl or alkinyl in each case having 2 to 6 carbon atoms; cycloalkenyl having 5 to 7 carbon atoms which is optionally monosubstituted to pentasubstituted by identical or different alkyl having 1 to 4 carbon atoms, or cycloalkyl having 3 to 6 carbon atoms which is optionally monosubstituted to pentasubstituted by identical or different substituents, substituents which may be mentioned being: halogen, alkyl and alkoxy in each case having 1 to 4 carbon atoms, cyano, amino, carbamoyl, alkylamino, dialkylamino, alkylcarbamoyl and dialkylcarbamoyl in each case having 1 to 4 carbon atoms in each alkyl part, alkoxycarbonylamino having 1 to 4 carbon atoms in the alkoxy part, cycloalkyl and cycloalkylalkyl having 5 or 6 carbon atoms in the cycloalkyl part and 1 to 4 carbon atoms in the straight-chain or branched alkyl part, pyrrolidone and phenyl which is optionally monosubstituted to pentasubstituted by identical or different substituents from the series comprising halogen and alkyl having 1 to 4 carbon atoms; R$^3$ additionally preferably represents phenyl which is optionally monosubstituted to pentasubstituted by identical or different substituents from the series comprising halogen, alkyl and alkoxy in each case having 1 to 4 carbon atoms, halogenoalkyl and halogenoalkoxy in each case having 1 or 2 carbon atoms and 1 to 5 identical or different halogen atoms, or represents a 5- or 6-membered heterocyclic ring which has 1 to 3 identical or different heteroatoms, and which is optionally monosubstituted to pentasubstituted by identical or different substituents, substituents which may be mentioned being: halogen, mercapto, phenyl, straight-chain or branched alkyl, alkoxy, alkylthio, alkylsulphinyl and alkylsulphonyl having 1 to 4 carbon atoms per alkyl part;

$R^4$ represents the meanings of R$^3$, represents polycyclic carbocyclic rings or the -OR$^{IV}$ group,

or

$R^3$ represents hydrogen or straight-chain or branched alkyl having 1 to 4 carbon atoms, and

$R^4$ represents monosubstituted or disubstituted, straight-chain or branched alkyl having 1 to 4 carbon atoms, having the following substituents: acyl having 2 to 9 carbon atoms, hydroxyl, alkylcarbonyloxy having 1 to 4 carbon atoms in the straight-chain or branched alkyl part, -S(O)$_n$R$^{IV}$, a 5- or 6-membered heterocyclic ring having 1 to 3 heteroatoms, which is optionally monosubstituted to pentasubstituted by identical or different substituents from the series comprising halogen, straight-chain or branched alkyl and alkoxy in each case having 1 to 4 carbon atoms, cycloalkyl having 3 to 6 carbon atoms and cycloalkenyl having 5 to 7 carbon atoms each of which is optionally monosubstituted to pentasubstituted by identical or different alkyl having 1 to 4 carbon atoms;

$R^4$ furthermore represents cycloalkenyl having 5 to 7 carbon atoms which is optionally monosubstituted to pentasubstituted by identical or different, straight-chain or branched alkyl having 1 to 4 carbon atoms, represents a 5- or 6-membered heterocyclic ring having 1 to 3 identical or different heteroatoms, which is optionally monosubstituted to pentasubstituted by identical or different substituents from the series comprising halogen, mercapto, phenyl, in each case straight-chain or branched alkyl, alkoxy, alkylthio, alkylsulphinyl and alkylsulphonyl having 1 to 4 carbon atoms per alkyl part, represents cycloalkyl having 3 to 6 carbon atoms which is monosubstituted to pentasubstituted by identical or different substituents from the series comprising phenyl, cyano, hydroxyl, alkoxy, alkylamino, dialkylamino, alkylcarbamoyl, dialkylcarbamoyl and alkoxycarbonylamino in each case having 1 to 4 carbon atoms per alkyl part, cycloalkyl and cycloalkylalkyl in each case having 5 to 7 carbon atoms in the cycloalkyl part and 1 to 4 carbon atoms in the alkyl part, where halogen and straight-chain or branched alkyl having 1 to 4 carbon atoms may be mentioned, if appropriate, as additional cycloalkyl substituents, and furthermore represents polycyclic carbocyclic rings or $-OR^{IV}$,

or

$R^3$ and $R^4$, together with the nitrogen atom to which they are bound, represent a monocyclic heterocyclic ring which is monosubstituted to pentasubstituted by identical or different substituents, or a bi- or tricyclic heterocyclic ring or spiroheterocyclic ring which is optionally monosubstituted to pentasubstituted by identical or different substituents, heterocyclic rings which may be mentioned being: oxazolidine, pyrrolidine, imidazolidine, piperidine, piperazine, morpholine, thiomorpholine, 1,3-oxazane or 1,3-diazane; these heterocyclic rings may in each case be fused, if appropriate, to 1 or 2 benzene or cyclohexane rings, or, if appropriate, bridged by methylene or ethylene.

Substituents which may be mentioned for all heterosystems are:

straight-chain or branched alkyl and alkoxy in each case having 1 to 4 carbon atoms, cycloalkyl having 3 to 6 carbon atoms, the hydroxyl group or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkyl- or dialkylcarbamoyl in each case having 1 to 4 carbon atoms in each alkyl part, and phenyl or phenylalkyl which has 1 to 4 carbon atoms in the straight-chain or branched alkyl part and each of which is optionally monosubstituted or disubstituted by identical or different substituents from the series comprising halogen or straight-chain or branched alkyl and alkoxy in each case having 1 to 4 carbon atoms,

$R^I$ represents hydrogen or straight-chain or branched alkyl having 1 to 6 carbon atoms,

$R^{II}$ represents hydrogen, straight-chain or branched alkyl having 1 to 6 carbon atoms, phenylalkyl which has 1 to 4 carbon atoms in the straight-chain or branched alkyl part and which is optionally monosubstituted to pentasubstituted by identical or different substituents, phenyl substituents which may be mentioned being: halogen, alkyl and alkoxy in each case having 1 to 4 carbon atoms, halogenoalkyl and halogenoalkoxy in each case having 1 or 2 carbon atoms and 2 to 5 identical or different halogen atoms; $R^{II}$ furthermore represents alkoxycarbonylalkyl, carbamoylalkyl, alkylcarbamoylalkyl or dialkylcarbamoylalkyl in each case having 1 to 4 carbon atoms in each alkyl part, or cycloalkyl having 3 to 6 carbon atoms which is optionally substituted by straight-chain or branched alkyl having 1 to 4 carbon atoms,

$R^{III}$ represents hydrogen, straight-chain or branched alkyl having 1 to 6 carbon atoms, phenylalkyl which has 1 to 4 carbon atoms in the straight-chain or branched alkyl part and which is optionally monosubstituted to pentasubstituted by identical or different substituents, suitable phenyl substituents being the phenyl substituents mentioned in the case of $R^{II}$; $R^{III}$ furthermore represents alkoxycarbonylalkyl, carbamoylalkyl, alkylcarbamoylalkyl or dialkylcarbamoylalkyl in each case having 1 to 4 carbon atoms in each alkyl part, or cycloalkyl having 3 to 6 carbon atoms which is optionally substituted by straight-chain or branched alkyl having 1 to 4 carbon atoms,

$R^{IV}$ represents hydrogen, straight-chain or branched alkyl having 1 to 6 carbon atoms, phenylalkyl which has 1 to 4 carbon atoms in the straight-chain or branched alkyl part

and which is optionally monosubstituted to pentasubstituted by identical or different substituents, suitable phenyl substituents preferably being the phenyl substituents mentioned in the case of $R^{II}$; $R^{IV}$ furthermore represents acyl having 2 to 9 carbon atoms, and

n represents the numbers 0, 1 or 2,

and the geometrical and optical isomers and isomer mixtures thereof.

**3.** (2-Cyano-2-oximinoacetyl)-amino acid derivatives according to Claim 1, in which, in the formula (I),

R represents alkyl having 1 or 2 carbon atoms, cyanomethyl or cyanoethyl, allyl or propargyl, cyclopropyl, cyclohexyl, cyclopropylmethyl or cyclohexylmethyl which is in each case optionally monosubstituted, disubstituted or trisubstituted by methyl, or benzyl or phenethyl which is in each case optionally monosubstituted, disubstituted or trisubstituted by identical or different substituents from the series comprising halogen and methyl,

$R^1$ represents the -B-CONR$^3$R$^4$ group,

B represents the $-CH_2-$, $-CH(CH_3)-$ or $-CH_2CH_2-$groups,

$R^3$ represents monosubstituted or disubstituted, straight-chain or branched alkyl having 1 to 4 carbon atoms, substituents which may preferably be mentioned being: halogen, cyano, $-COOR^I$, $-CONR^{II}R^{III}$, $-NR^{II}R^{III}$, $-OR^{IV}$, $-S(O)_nR^{IV}$, acyl having 2 to 9 carbon atoms, phenyl which is optionally monosubstituted, disubstituted or trisubstituted by identical or different substituents from the series comprising halogen and alkyl having 1 to 4 carbon atoms, a 5- or 6-membered heterocyclic ring having 1 to 3 identical or different heteroatoms, which is optionally monosubstituted, disubstituted or trisubstituted by identical or different substituents from the series comprising halogen, straight-chain or branched alkyl and alkoxy in each case having 1 to 4 carbon atoms, and cycloalkyl having 3 to 6 carbon atoms and cycloalkenyl having 5 to 7 carbon atoms each of which is optionally substituted by straight-chain or branched alkyl having 1 to 4 carbon atoms; $R^3$ furthermore represents straight-chain or branched alkenyl or alkinyl in each case having 2 to 6 carbon atoms, cycloalkenyl having 5 to 7 carbon atoms which is optionally substituted by straight-chain or branched alkyl having 1 to 4 carbon atoms, optionally substituted cycloalkyl having 3 to 6 carbon atoms, substituents which may preferably be mentioned being: halogen, straight-chain or branched alkyl and alkoxy in each case having 1 to 4 carbon atoms, cyano, amino, carbamoyl, alkylamino, dialkylamino, alkylcarbamoyl and dialkylcarbamoyl in each case having 1 to 4 carbon atoms in each alkyl part, alkoxycarbonylamino having 1 to 4 carbon atoms in the straight-chain or branched alkoxy part, cycloalkyl and cycloalkylalkyl in each case having 5 or 6 carbon atoms in the cycloalkyl part and 1 to 4 carbon atoms, in the straight-chain or branched alkyl part, pyrrolidone or phenyl which is optionally monosubstituted, disubstituted or trisubstituted by identical or different substituents from the series comprising halogen and alkyl having 1 to 4 carbon atoms; $R^3$ furthermore represents phenyl which is optionally monosubstituted, disubstituted or trisubstituted by identical or different substituents from the series comprising halogen, straight-chain or branched alkyl and alkoxy in each case having 1 to 4 carbon atoms, halogenoalkyl or halogenoalkoxy in each case having 1 or 2 carbon atoms and 1 to 5 identical or different halogen atoms, or a 5- or 6-membered heterocyclic ring having 1 to 3 identical or different heteroatoms, which is optionally monosubstituted, disubstituted or trisubstituted by identical or different substituents. Substituents which may be mentioned are: halogen, mercapto, phenyl, straight-chain or branched alkyl, alkoxy, alkylthio, alkylsulphinyl and alkylsulphonyl having 1 to 4 carbon atoms per alkyl part;

$R^4$ represents the meanings of $R^3$, represents polycyclic carbocyclic rings or the $-OR^{IV}$ group,

or

$R^3$ represents hydrogen or straight-chain or branched alkyl having 1 to 4 carbon atoms, and

$R^4$ represents monosubstituted or disubstituted, straight-chain or branched alkyl having 1 to 4 carbon atoms, having the following substituents: acyl having 2 to 9 carbon atoms, hydroxyl, alkylcarbonyloxy having 1 to 4 carbon atoms in the straight-chain or

branched alkyl part, $-S(O)_nR^{IV}$, a 5- or 6-membered heterocyclic ring having 1 to 3 heteroatoms, which is optionally monosubstituted, disubstituted or trisubstituted by identical or different substituents from the series comprising halogen, straight-chain or branched alkyl and alkoxy in each case having 1 to 4 carbon atoms, or cycloalkyl having 3 to 6 carbon atoms and cycloalkenyl having 5 to 7 carbon atoms each of which is optionally monosubstituted, disubstituted or trisubstituted by identical or different alkyl having 1 to 4 carbon atoms;

$R^4$ furthermore represents cycloalkenyl having 5 to 7 carbon atoms which is optionally monosubstituted, disubstituted or trisubstituted by identical or different, straight-chain or branched alkyl having 1 to 4 carbon atoms, represents a 5- or 6-membered heterocyclic ring having 1 to 3 identical or different heteroatoms, which is optionally monosubstituted, disubstituted or trisubstituted by identical or different substituents from the series comprising halogen, mercapto, phenyl, in each case straight-chain or branched alkyl, alkoxy, alkylthio, alkylsulphinyl and alkylsulphonyl in each case having 1 to 4 carbon atoms per alkyl part, represents cycloalkyl having 3 to 6 carbon atoms which is monosubstituted, disubstituted or trisubstituted by identical or different substituents from the series comprising phenyl, cyano, hydroxyl, alkoxy, alkylamino, dialkylamino, alkylcarbamoyl, dialkylcarbamoyl, alkoxycarbonylamino in each case having 1 to 4 carbon atoms per alkyl part, cycloalkyl and cycloalkylalkyl in each case having 5 to 7 carbon atoms in the cycloalkyl part and 1 to 4 carbon atoms in the alkyl part, additional cycloalkyl substituents which may be mentioned being, if appropriate, halogen and straight-chain or branched alkyl having 1 to 4 carbon atoms, and furthermore represents polycyclic carbocyclic rings or $-OR^{IV}$,

or

$R^3$ and $R^4$, together with the nitrogen atom to which they are bound, represent a monocyclic heterocyclic ring which is monosubstituted, disubstituted or trisubstituted by identical or different substituents, or a bi- or tricyclic heterocyclic ring or spiroheterocyclic ring which is optionally monosubstituted, disubstituted or trisubstituted by identical or different substituents, heterocyclic rings which may be mentioned being: oxazolidine, pyrrolidine, imidazolidine, piperidine, piperazine, morpholine, thiomorpholine, 1,3-oxazane or 1,3-diazane; these heterocyclic rings may in each case be fused, if appropriate, to 1 or 2 benzene or cyclohexane rings, or, if appropriate, bridged by methylene or ethylene.

Substituents which may be mentioned for all heterosystems are:

straight-chain or branched alkyl or alkoxy in each case having 1 to 4 carbon atoms, cycloalkyl having 3 to 6 carbon atoms, the hydroxyl group or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkyl- or dialkylcarbamoyl in each case having 1 to 4 carbon atoms in each alkyl part, and phenyl or phenylalkyl which has 1 to 4 carbon atoms in the straight-chain or branched alkyl part and each of which is optionally monosubstituted or disubstituted by identical or different substituents from the series comprising halogen or straight-chain or branched alkyl and alkoxy in each case having 1 to 4 carbon atoms,

$R^I$ represents hydrogen or straight-chain or branched alkyl having 1 to 6 carbon atoms,

$R^{II}$ represents hydrogen, straight-chain or branched alkyl having 1 to 4 carbon atoms, or benzyl or phenethyl each of which is optionally monosubstituted, disubstituted or trisubstituted by identical or different substituents, phenyl substituents which may be mentioned in each case being: fluorine, chlorine, methyl, methoxy and trifluoromethyl;

$R^{II}$ furthermore represents alkoxycarbonylalkyl having 1 to 4 carbon atoms in the alkoxy part and 1 or 2 carbon atoms in the alkyl part, carbamoylalkyl having 1 or 2 carbon atoms in the alkyl part, alkylcarbamoylalkyl and dialkylcarbamoylalkyl in each case having 1 or 2 carbon atoms in each alkyl part, or cycloalkyl having 3 to 6 carbon atoms which is optionally monosubstituted, disubstituted or trisubstituted by identical or different substituents from the series comprising methyl and ethyl,

$R^{III}$ represents the meanings of $R^{II}$,

$R^{IV}$ represents hydrogen, straight-chain or branched alkyl having 1 to 4 carbon atoms, or benzyl or phenethyl each of which is optionally monosubstituted, disubstituted or

trisubstituted by identical or different substituents, phenyl substituents which may be mentioned in each case being: fluorine, chlorine, methyl, methoxy and trifluoromethyl; $R^{IV}$ furthermore represents acyl having 2 to 9 carbon atoms, and

n represents the numbers 0, 1 or 2,

and the geometrical and optical isomers and isomer mixtures thereof.

**4.** (2-Cyano-2-oximinoacetyl)-amino acid derivatives according to Claim 1, in which, in the formula (I),

R represents methyl, ethyl, cyanomethyl, cyanoethyl, allyl, propargyl, represents a cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents the $-B-CO-NR^3R^4$ group

B represents the $-CH_2-$, $-CH(CH_3)-$ or $-CH_2CH_2-$groups

$R^3$ represents monosubstituted or disubstituted alkyl having 1 to 3 carbon atoms, substituents which may be mentioned being: chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio in each case having 1 or 2 carbon atoms, 2-furyl, 2-pyridyl, 1-morpholino, cyclopropyl, cyclohexyl and phenyl which is optionally monosubstituted, disubstituted or trisubstituted by identical or different substituents from the series comprising chlorine and methyl; $R^3$ furthermore very particularly preferably represents allyl or propargyl, 1-cyclohexenyl, or cyclohexyl which is monosubstituted, disubstituted or trisubstituted by identical or different substituents, substituents which may be mentioned being: chlorine, methyl, ethyl, methoxy, cyano, amino, alkyl- and dialkylamino in each case having 1 or 2 carbon atoms in each alkyl part, carbamoyl, alkyl- and dialkylcarbamoyl in each case having 1 or 2 carbon atoms in each alkyl part, cyclohexyl, cyclohexylalkyl having 1 or 2 carbon atoms in the alkyl part and 1-pyrrolidin-2-one;

$R^3$ additionally represents phenyl which is optionally monosubstituted, disubstituted or trisubstituted by identical or different substituents from the series comprising chlorine and methyl, represents 2-pyridyl or 2-pyrimidinyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto, straight-chain or branched alkyl having 1 to 4 carbon atoms, in each case straight-chain or branched alkylthio, alkylsulphinyl and alkylsulphonyl in each case having 1 to 4 carbon atoms, or represents the

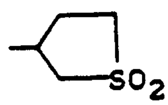

group,

$R^4$ represents monosubstituted or disubstituted alkyl having 1 to 3 carbon atoms, substituents which may be mentioned being: chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio in each case having 1 or 2 carbon atoms, or 2-furyl, 2-pyridyl, 1-morpholino, cyclopropyl, cyclohexyl and phenyl which is optionally monosubstituted, disubstituted or trisubstituted by identical or different substituents from the series comprising chlorine and methyl; $R^4$ furthermore very particularly preferably represents allyl or propargyl, 1-cyclohexenyl, or cyclohexyl which is monosubstituted, disubstituted or trisubstituted by identical or different substituents, substituents which may be mentioned being: chlorine, methyl, ethyl, methoxy, cyano, amino, alkyl- or dialkylamino in each case having 1 or 2 carbon atoms in each alkyl part, carbamoyl, alkyl- and dialkylcarbamoyl in each case having 1 or 2 carbon atoms in each alkyl part, cyclohexyl, cyclohexylalkyl having 1 or 2 carbon atoms in the alkyl part, and 1-pyrrolidin-2-one;

$R^4$ additionally represents phenyl which is optionally monosubstituted, disubstituted or trisubstituted by identical or different substituents from the series comprising chlorine and methyl, represents 2-pyridyl or 2-pyrimidinyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto, straight-chain or branched alkyl having 1 to 4 carbon atoms in each case straight-chain or branched alkylthio, alkylsulphinyl or alkylsulphonyl in each case having 1 to 4 carbon atoms, or represents the

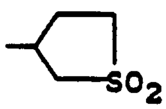

group,

$R^4$ furthermore represents hydroxyl, alkoxy having 1 or 2 carbon atoms, benzyloxy which is optionally monosubstituted, disubstituted or trisubstituted by identical or different substituents from the series comprising chlorine and methyl, represents 1-adamantyl, 2-norbornyl, 1- or 2-decalyl or 1- or 2-tetralyl.

**5.** (2-Cyano-2-oximinoacetyl)-amino acid derivatives according to Claim 1, in which, in the formula (I)

R represents methyl, ethyl, cyanomethyl, cyanoethyl, allyl, propargyl, represents cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents the $-B-CO-NR^3R^4$ group,

B represents the $-CH_2-$, $-CH(CH_3)-$ or $-CH_2CH_2-$groups,

$R^3$ represents hydrogen or straight-chain or branched alkyl having 1 to 4 carbon atoms,

$R^4$ represents substituted alkyl having 1 to 3 carbon atoms, substituents which may be mentioned being: hydroxyl, alkylcarbonyloxy having 1 to 4 carbon atoms in the straight-chain or branched alkyl part, alkylcarbonyl having 1 to 4 carbon atoms in the alkyl part, alkylthio having 1 or 2 carbon atoms, 2-furyl, 2-pyridyl, 1-morpholino, cyclopropyl and cyclohexyl; $R^4$ furthermore represents 1-cyclohexenyl, represents 2-pyridyl or 2-pyrimidyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto and alkyl, alkylthio, alkylsulphinyl and alkylsulphonyl in each case having 1 to 4 carbon atoms, represents the

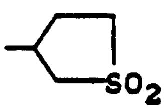

group,

represents 1-adamantyl, 2-norbornyl, 1- or 2-decalyl or 1- or 2-tetralyl; $R^4$ additionally

represents cyclohexyl which is monosubstituted, disubstituted or trisubstituted by identical or different substituents, substituents which may be mentioned being: phenyl, cyano, hydroxyl, alkoxy having 1 or 2 carbon atoms, alkylamino and dialkylamino in each case having 1 or 2 carbon atoms in each alkyl part, alkylcarbamoyl and dialkylcarbamoyl in each case having 1 or 2 carbon atoms in each alkyl part, alkoxycarbonylamino having 1 or 2 carbon atoms in the alkyl part, cyclohexyl, cyclohexylalkyl having 1 or 2 carbon atoms in the alkyl part, and 1-pyrrolidin-2-one, and additionally halogen and alkyl having 1 or 2 carbon atoms.

**6.** (2-Cyano-2-oximinoacetyl)-amino acid derivatives according to Claim 1, in which, in the formula (I),

R represents methyl, ethyl, cyanomethyl, cyanoethyl, allyl, propargyl, represents cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents the $-B-CO-NR^3R^4$ group,

B represents the $-CH_2-$, $-CH(CH_3)-$ or $-CH_2CH_2-$groups,

$R^3$ and $R^4$ , together with the nitrogen atom to which they are bound, represent the following mono-, bi- or tricyclic heterocyclic rings or spiroheterocyclic rings:

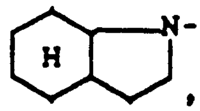 , 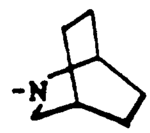 , 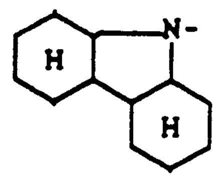 ,

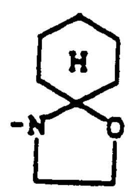 , 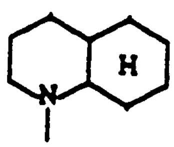 ,

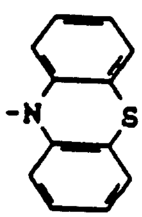 , 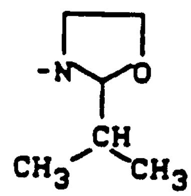 , 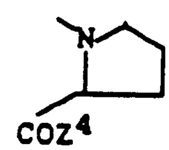

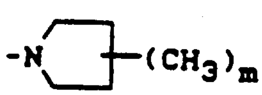 , 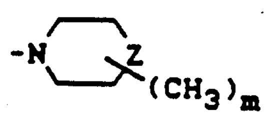

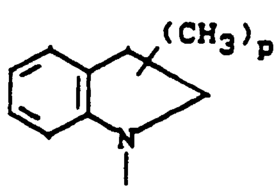 , 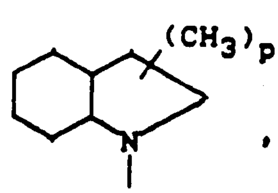 ,

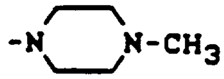 , 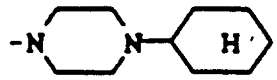 , 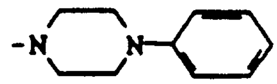 ,

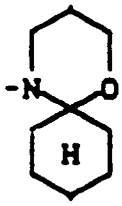 , 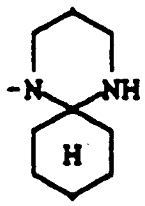 , 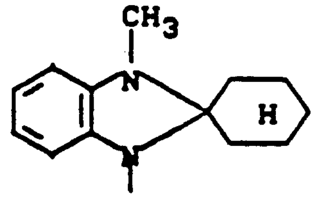 ,

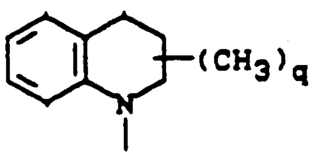 , 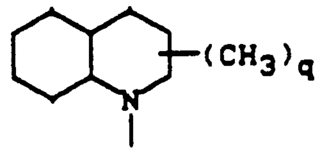 ,

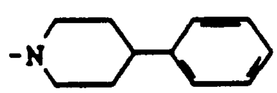 , 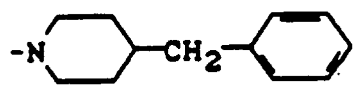 ,

where

Z represents oxygen or the $CH_2$ group,

$Z^4$ represents hydroxyl, methoxy, ethoxy, amino, methylamino, ethylamino, dimethylamino or ethylmethylamino,

m represents the numbers 1, 2, 3 or 4,

p represents the numbers 0, 1 or 2, and

q represents the numbers 0, 1, 2 or 3.

**7.** Process for the preparation of (2-cyano-2-oximinoacetyl)-amino acid derivatives of the general formula (I)

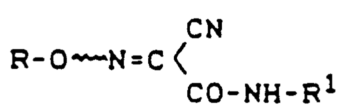 (I)

in which

R represents alkyl, cyanoalkyl or in each case optionally substituted alkenyl, alkinyl, cycloalkyl, cycloalkylalkyl or phenylalkyl,

$R^1$ represents the -B-CONR$^3$R$^4$ group, where

B represents an optionally substituted, straight-chain or branched alkylene chain,

$R^3$ represents substituted alkyl having the following substituents: halogen, cyano, -COOR$^I$, -CONR$^{II}$R$^{III}$, -NR$^{II}$R$^{III}$, -OR$^{IV}$, -S(O)$_n$R$^{IV}$, acyl, in each case optionally substituted aryl, heterocyclyl, cycloalkyl and cycloalkenyl; $R^3$ furthermore represents in each case optionally substituted alkenyl, alkinyl, cycloalkyl, cycloalkenyl, aryl or heterocyclyl, and

$R^4$ represents substituted alkyl having the following substituents: halogen, cyano, -COOR$^I$, -CONR$^{II}$R$^{III}$, -NR$^{II}$R$^{III}$, -OR$^{IV}$, -S(O)$_n$R$^{IV}$, acyl, in each case optionally substituted aryl, heterocyclyl, cycloalkyl and cycloalkenyl; $R^4$ furthermore represents in each case optionally substituted alkenyl, alkinyl, cycloalkyl, cycloalkenyl, aryl, heterocyclyl or polycyclic carbocyclic rings or the -OR$^{IV}$ group,

or

$R^3$ represents hydrogen or alkyl, and

$R^4$ represents substituted alkyl having the following substituents: acyl, hydroxyl, alkylcar-

bonyloxy, $-S(O)_nR^{IV}$, in each case optionally substituted heterocyclyl, cycloalkyl and cycloalkenyl; $R^4$ furthermore represents in each case optionally substituted cycloalkenyl, heterocyclyl or polycyclic carbocyclic rings, represents substituted cycloalkyl where, however, halogen and alkyl may not be present as the only substituents, or represents the $-OR^{IV}$ group,

or

$R^3$ and $R^4$, together with the nitrogen atom to which they are bound, represents a substituted, monocyclic heterocyclic ring or an optionally substituted bi- or tricyclic heterocyclic ring or spiroheterocyclic ring having 1 to 3 further identical or different heteroatoms,

$R^I$ represents hydrogen or optionally substituted alkyl having the following substituents: halogen, in each case optionally substituted aryl, cycloalkyl, and cycloalkenyl,

$R^{II}$ and $R^{III}$ are identical or different and represent hydrogen or optionally substituted alkyl, having the following substituents: halogen, $-COOR^{IV}$, $-CONR^IR^{IV}$, in each case optionally substituted aryl, cycloalkyl and cycloalkenyl; $R^{II}$ and $R^{III}$ furthermore represent alkenyl, alkinyl or in each case optionally substituted cycloalkyl, cycloalkenyl, aryl or heterocyclyl, or, together with the nitrogen atom to which they are bound, represent an optionally substituted heterocyclic ring which may contain further heteroatoms,

$R^{IV}$ represents hydrogen, alkyl, aralkyl or acyl, and

n represents the numbers 0, 1 or 2,

and the geometrical and optical isomers and isomer mixtures thereof, characterised in that

a) 2-cyano-2-oximino-acetates of the general formula (II)

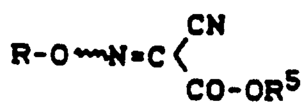

(II)

in which

R has the abovementioned meaning, and

$R^5$ represents alkyl,

are reacted with amines of the formula (III)

$R^1-NH_2$ (III)

in which

$R^1$ has the abovementioned meaning,

if appropriate in the presence of a diluent and

if appropriate in the presence of a base;

or

b) carboxyl-activated derivatives of carboxylic acids of the general formula (IV)

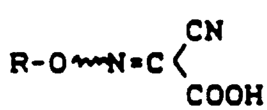

(IV)

in which

R has the abovementioned meaning,

are reacted with amines of the formula (III)

$R^1-NH_2$ (III)

in which

$R^1$ has the abovementioned meaning,

if appropriate in the presence of a catalyst and

if appropriate in the presence of an acid-binding agent and if appropriate in the presence of a

diluent;
or
c) 2-cyano-2-oximino-acetamides of the general formula (V)

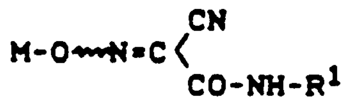 (V)

in which
M represents hydrogen, an alkali metal ion, a protonated tertiary base or a quaternary ammonium ion, and
$R^1$ has the abovementioned meaning,
are reacted with compounds of the formula (VI)

R-X (VI)

in which
X represents halogen or a sulphonyloxy radical, and
R has the abovementioned meaning,
if appropriate in the presence of a base and if appropriate in the presence of a diluent;
or
d) substituted acetyl-amino acid esters of the general formula (VII)

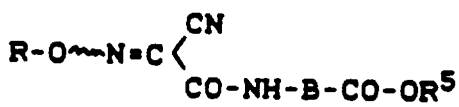 (VII)

in which
B, R and $R^5$ have the abovementioned meaning,
are reacted with amines of the formula (VIII)

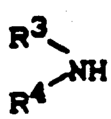 (VIII)

in which
$R^3$ and $R^4$ have the abovementioned meaning,
if appropriate in the presence of a diluent and
if appropriate in the presence of a base;
or
e) carboxyl-activated acetyl-amino acids of the general formula (IX)

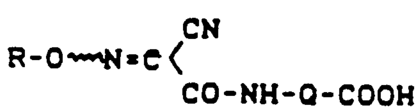 (IX)

in which
R has the abovementioned meaning, and
Q represents A or B, the latter having the abovementioned meaning,
are reacted with compounds of the formula (X)

Y-H (X)

in which

Y represents the $R^2O$- or $R^3R^4N$- groups where $R^2$, $R^3$ and $R^4$ have the abovementioned meaning,

if appropriate in the presence of a catalyst and if appropriate in the presence of an acid-binding agent and if appropriate in the presence of a diluent;

or

f) ammonium salts of 2-cyano-2-oximino-acetic acid of the general formula (XI)

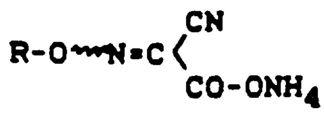 (XI)

in which

R has the abovementioned meaning,

are reacted with aldehydes of the formula (XII)

$R^6$-CH=O (XII)

in which

the $R^6$-CH= group represents the abovementioned meanings of A or B,

and with isonitriles of the formula (XIII)

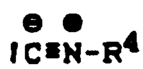 (XIII)

in which

$R^4$ has the abovementioned meaning,

if appropriate in the presence of a diluent;

or

g) 2-cyano-2-oximino-acetic acids of the general formula (IV)

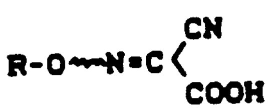 (IV)

in which

R has the abovementioned meaning,

are reacted with isocyanates of the formula (XIV)

$R^1$-NCO (XIV)

in which

$R^1$ has the abovementioned meaning, if appropriate in the presence of a catalyst and if appropriate in the presence of a diluent;

or

h) 2-cyano-2-oximino-acetamides of the general formula (XV)

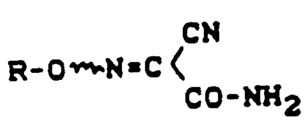 (XV)

in which

R has the abovementioned meaning,

are reacted with a base, if appropriate in the presence of a diluent, and the resultant salts are reacted directly or, if appropriate, after intermediate isolation, with compounds of the formula (XVI)

$R^1$-X (XVI)

in which

$R^1$ and X have the abovementioned meaning,

if appropriate in the presence of a diluent.

**8.** Pesticides, characterised in that they contain at least one (2-cyano-2-oximinoacetyl)-amino acid derivative of the formula (I) according to Claims 1 and 7.

**9.** Use of (2-cyano-2-oximinoacetyl)-amino acid derivatives of the formula (I) according to Claims 1 and 7 for combating pests.

**10.** Process for combating pests, characterised in that (2-cyano-2-oximinoacetyl)-amino acid derivatives of the formula (I) according to Claims 1 and 7 are allowed to act on the pests and/or on their environment.

**11.** Process for the production of pesticides, characterised in that (2-cyano-2-oximinoacetyl)-amino acid derivatives of the formula (I) according to Claims 1 and 7 are mixed with extenders and/or surface-active agents.

**Claims for the following Contracting State : ES**

**1.** Process for the preparation of (2-cyano-2-oximinoacetyl)-amino acid derivatives of the general formula (I)

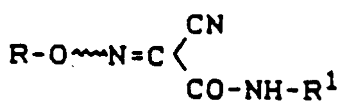

(I)

in which

| | |
|---|---|
| R | represents alkyl, cyanoalkyl or in each case optionally substituted alkenyl, alkinyl, cycloalkyl, cycloalkylalkyl or phenylalkyl, |
| $R^1$ | represents the -B-CONR$^3$R$^4$ group, where |
| B | represents an optionally substituted, straight-chain or branched alkylene chain, |
| $R^3$ | represents substituted alkyl having the following substituents: halogen, cyano, -COOR$^I$, -CONR$^{II}$R$^{III}$, -NR$^{II}$R$^{III}$, -OR$^{IV}$, -S(O)$_n$R$^{IV}$, acyl, in each case optionally substituted aryl, heterocyclyl, cycloalkyl and cycloalkenyl; $R^3$ furthermore represents in each case optionally substituted alkenyl, alkinyl, cycloalkyl, cycloalkenyl, aryl or heterocyclyl, and |
| $R^4$ | represents substituted alkyl having the following substituents: halogen, cyano, -COOR$^I$, -CONR$^{II}$R$^{III}$, -NR$^{II}$R$^{III}$, -OR$^{IV}$, -S(O)$_n$R$^{IV}$, acyl, in each case optionally substituted aryl, heterocyclyl, cycloalkyl and cycloalkenyl; $R^4$ furthermore represents in each case optionally substituted alkenyl, alkinyl, cycloalkyl, cycloalkenyl, aryl, heterocyclyl or polycyclic carbocyclic rings or the -OR$^{IV}$ group, |
| | or |
| $R^3$ | represents hydrogen or alkyl, and |
| $R^4$ | represents substituted alkyl having the following substituents: acyl, hydroxyl, alkylcarbonyloxy, -S(O)$_n$R$^{IV}$, in each case optionally substituted heterocyclyl, cycloalkyl and cycloalkenyl; $R^4$ furthermore represents in each case optionally substituted cycloalkenyl, heterocyclyl or polycyclic carbocyclic rings, represents substituted cycloalkyl where, however, halogen and alkyl may not be present as the only substituents, or represents the -OR$^{IV}$ group, |

or

$R^3$ and $R^4$, together with the nitrogen atom to which they are bound, represents a substituted, monocyclic heterocyclic ring or an optionally substituted bi- or tricyclic heterocyclic ring or spiroheterocyclic ring having 1 to 3 further identical or different heteroatoms,

$R^I$ represents hydrogen or optionally substituted alkyl having the following substituents: halogen, in each case optionally substituted aryl, cycloalkyl, and cycloalkenyl,

$R^{II}$ and $R^{III}$ are identical or different and represent hydrogen or optionally substituted alkyl, having the following substituents: halogen, $-COOR^{IV}$, $-CONR^IR^{IV}$, in each case optionally substituted aryl, cycloalkyl and cycloalkenyl; $R^{II}$ and $R^{III}$ furthermore represent alkenyl, alkinyl or in each case optionally substituted cycloalkyl, cycloalkenyl, aryl or heterocyclyl, or, together with the nitrogen atom to which they are bound, represent an optionally substituted heterocyclic ring which may contain further heteroatoms,

$R^{IV}$ represents hydrogen, alkyl, aralkyl or acyl, and

n represents the numbers 0, 1 or 2,

and the geometrical and optical isomers and isomer mixtures thereof, characterised in that

a) 2-cyano-2-oximino-acetates of the general formula (II)

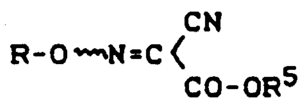

(II)

in which

R has the abovementioned meaning, and

$R^5$ represents alkyl,

are reacted with amines of the formula (III)

$R^1$-$NH_2$ (III)

in which

$R^1$ has the abovementioned meaning,

if appropriate in the presence of a diluent and if appropriate in the presence of a base;

or

b) carboxyl-activated derivatives of carboxylic acids of the general formula (IV)

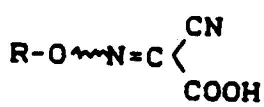

(IV)

in which

R has the abovementioned meaning,

are reacted with amines of the formula (III)

$R^1$-$NH_2$ (III)

in which

$R^1$ has the abovementioned meaning,

if appropriate in the presence of a catalyst and if appropriate in the presence of an acid-binding agent and if appropriate in the presence of a diluent;

or

c) 2-cyano-2-oximino-acetamides of the general formula (V)

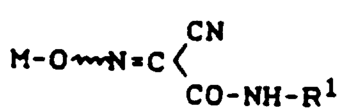

(V)

in which

M represents hydrogen, an alkali metal ion, a protonated tertiary base or a quaternary ammonium ion, and

$R^1$ has the abovementioned meaning,

are reacted with compounds of the formula (VI)

R-X (VI)

in which

X represents halogen or a sulphonyloxy radical, and

R has the abovementioned meaning,

if appropriate in the presence of a base and if appropriate in the presence of a diluent;

or

d) substituted acetyl-amino acid esters of the general formula (VII)

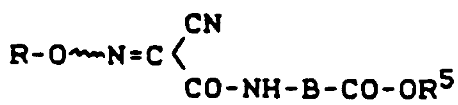

(VII)

in which

B, R and $R^5$ have the abovementioned meaning,

are reacted with amines of the formula (VIII)

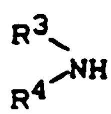

(VIII)

in which

$R^3$ and $R^4$ have the abovementioned meaning,

if appropriate in the presence of a diluent and

if appropriate in the presence of a base;

or

e) carboxyl-activated acetyl-amino acids of the general formula (IX)

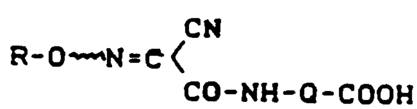

(IX)

in which

R has the abovementioned meaning, and

Q represents A or B, the latter having the abovementioned meaning,

are reacted with compounds of the formula (X)

Y-H (X)

in which

Y represents the $R^2O$- or $R^3R^4N$- groups where $R^2$, $R^3$ and $R^4$ have the abovementioned meaning,

if appropriate in the presence of a catalyst and if appropriate in the presence of an acid-binding agent and if appropriate in the presence of a diluent;

or

f) ammonium salts of 2-cyano-2-oximino-acetic acid of the general formula (XI)

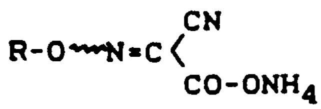

(XI)

in which

R has the abovementioned meaning,

are reacted with aldehydes of the formula (XII)

$R^6$-CH = O (XII)

in which

the $R^6$-CH = group represents the abovementioned meanings of A or B,

and with isonitriles of the formula (XIII)

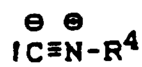

(XIII)

in which

$R^4$ has the abovementioned meaning,

if appropriate in the presence of a diluent;

or

g) 2-cyano-2-oximino-acetic acids of the general formula (IV)

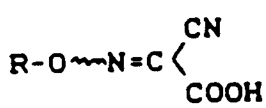

(IV)

in which

R has the abovementioned meaning,

are reacted with isocyanates of the formula (XIV)

$R^1$-NCO (XIV)

in which

$R^1$ has the abovementioned meaning,

if appropriate in the presence of a catalyst and if appropriate in the presence of a diluent;

or

h) 2-cyano-2-oximino-acetamides of the general formula (XV)

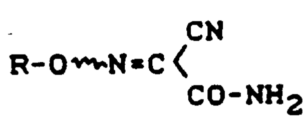

(XV)

in which

R has the abovementioned meaning,

are reacted with a base, if appropriate in the presence of a diluent, and the resultant salts are reacted directly or, if appropriate, after intermediate isolation, with compounds of the formula (XVI)

$R^1$-X (XVI)

in which
$R^1$ and X have the abovementioned meaning,
if appropriate in the presence of a diluent.

**2.** Process according to Claim 1, in which, in the formula (I),

R represents straight-chain or branched alkyl having 1 to 4 carbon atoms, straight-chain or branched cyanoalkyl having 1 to 4 carbon atoms in the alkyl part, in each case straight-chain or branched alkenyl or alkinyl having 2 to 6 carbon atoms, cycloalkyl or cycloalkylalkyl each of which has 3 to 6 carbon atoms per cycloalkyl part and 1 to 4 carbon atoms in the straight-chain or branched alkyl part and each of which is optionally monosubstituted to pentasubstituted by identical or different alkyl having 1 to 4 carbon atoms, or represents benzyl or phenethyl, each of which is optionally monosubstituted to pentasubstituted by identical or different substituents, phenyl substituents which may be mentioned in each case being: halogen, alkyl and alkoxy in each case having 1 to 4 carbon atoms, halogenoalkyl and halogenoalkoxy in each case having 1 or 2 carbon atoms and 2 to 5 identical or different halogen atoms;

$R^1$ represents the -A-COOR$^2$ or -B-CONR$^3$R$^4$ groups,

B represents a straight-chain or branched alkylene chain having 1 to 4 carbon atoms,

$R^3$ represents monosubstituted or disubstituted, straight-chain or branched alkyl having 1 to 4 carbon atoms, substituents which may be mentioned being: halogen, cyano, -COOR$^I$, -CONR$^{II}$R$^{III}$, -NR$^{II}$R$^{III}$, -OR$^{IV}$, -S(O)$_n$R$^{IV}$, acyl having 2 to 9 carbon atoms, phenyl which is optionally monosubstituted to pentasubstituted by identical or different substituents from the series comprising halogen and alkyl having 1 to 4 carbon atoms, a 5- or 6-membered heterocyclic ring having 1 to 3 heteroatoms, which is optionally monosubstituted to pentasubstituted by identical or different substituents from the series comprising halogen and alkyl and alkoxy in each case having 1 to 4 carbon atoms, cycloalkyl having 3 to 6 carbon atoms and cycloalkenyl having 5 to 7 carbon atoms each of which is optionally monosubstituted to pentasubstituted by identical or different alkyl having 1 to 4 carbon atoms; $R^3$ furthermore preferably represents straight-chain or branched alkenyl or alkinyl in each case having 2 to 6 carbon atoms; cycloalkenyl having 5 to 7 carbon atoms which is optionally monosubstituted to pentasubstituted by identical or different alkyl having 1 to 4 carbon atoms, or cycloalkyl having 3 to 6 carbon atoms which is optionally monosubstituted to pentasubstituted by identical or different substituents, substituents which may be mentioned being: halogen, alkyl and alkoxy in each case having 1 to 4 carbon atoms, cyano, amino, carbamoyl, alkylamino, dialkylamino, alkylcarbamoyl and dialkylcarbamoyl in each case having 1 to 4 carbon atoms in each alkyl part, alkoxycarbonylamino having 1 to 4 carbon atoms in the alkoxy part, cycloalkyl and cycloalkylalkyl having 5 or 6 carbon atoms in the cycloalkyl part and 1 to 4 carbon atoms in the straight-chain or branched alkyl part, pyrrolidone and phenyl which is optionally monosubstituted to pentasubstituted by identical or different substituents from the series comprising halogen and alkyl having 1 to 4 carbon atoms; $R^3$ additionally preferably represents phenyl which is optionally monosubstituted to pentasubstituted by identical or different substituents from the series comprising halogen, alkyl and alkoxy in each case having 1 to 4 carbon atoms, halogenoalkyl and halogenoalkoxy in each case having 1 or 2 carbon atoms and 1 to 5 identical or different halogen atoms, or represents a 5- or 6-membered heterocyclic ring which has 1 to 3 identical or different heteroatoms, and which is optionally monosubstituted to pentasubstituted by identical or different substituents, substituents which may be mentioned being: halogen, mercapto, phenyl, straight-chain or branched alkyl, alkoxy, alkylthio, alkylsulphinyl and alkylsulphonyl having 1 to 4 carbon atoms per alkyl part;

$R^4$ represents the meanings of $R^3$, represents polycyclic carbocyclic rings or the -OR$^{IV}$ group,

or

$R^3$ represents hydrogen or straight-chain or branched alkyl having 1 to 4 carbon atoms,

and

$R^4$ represents monosubstituted or disubstituted, straight-chain or branched alkyl having 1 to 4 carbon atoms, having the following substituents: acyl having 2 to 9 carbon atoms, hydroxyl, alkylcarbonyloxy having 1 to 4 carbon atoms in the straight-chain or branched alkyl part, $-S(O)_nR^{IV}$, a 5- or 6-membered heterocyclic ring having 1 to 3 heteroatoms, which is optionally monosubstituted to pentasubstituted by identical or different substituents from the series comprising halogen, straight-chain or branched alkyl and alkoxy in each case having 1 to 4 carbon atoms, cycloalkyl having 3 to 6 carbon atoms and cycloalkenyl having 5 to 7 carbon atoms each of which is optionally monosubstituted to pentasubstituted by identical or different alkyl having 1 to 4 carbon atoms;

$R^4$ furthermore represents cycloalkenyl having 5 to 7 carbon atoms which is optionally monosubstituted to pentasubstituted by identical or different, straight-chain or branched alkyl having 1 to 4 carbon atoms, represents a 5- or 6-membered heterocyclic ring having 1 to 3 identical or different heteroatoms, which is optionally monosubstituted to pentasubstituted by identical or different substituents from the series comprising halogen, mercapto, phenyl, in each case straight-chain or branched alkyl, alkoxy, alkylthio, alkylsulphinyl and alkylsulphonyl having 1 to 4 carbon atoms per alkyl part, represents cycloalkyl having 3 to 6 carbon atoms which is monosubstituted to pentasubstituted by identical or different substituents from the series comprising phenyl, cyano, hydroxyl, alkoxy, alkylamino, dialkylamino, alkylcarbamoyl, dialkylcarbamoyl and alkoxycarbonylamino in each case having 1 to 4 carbon atoms per alkyl part, cycloalkyl and cycloalkylalkyl in each case having 5 to 7 carbon atoms in the cycloalkyl part and 1 to 4 carbon atoms in the alkyl part, where halogen and straight-chain or branched alkyl having 1 to 4 carbon atoms may be mentioned, if appropriate, as additional cycloalkyl substituents, and furthermore represents polycyclic carbocyclic rings or $-OR^{IV}$,

or

$R^3$ and $R^4$, together with the nitrogen atom to which they are bound, represent a monocyclic heterocyclic ring which is monosubstituted to pentasubstituted by identical or different substituents, or a bi- or tricyclic heterocyclic ring or spiroheterocyclic ring which is optionally monosubstituted to pentasubstituted by identical or different substituents, heterocyclic rings which may be mentioned being: oxazolidine, pyrrolidine, imidazolidine, piperidine, piperazine, morpholine, thiomorpholine, 1,3-oxazane or 1,3-diazane; these heterocyclic rings may in each case be fused, if appropriate, to 1 or 2 benzene or cyclohexane rings, or, if appropriate, bridged by methylene or ethylene.

Substituents which may be mentioned for all heterosystems are:

straight-chain or branched alkyl and alkoxy in each case having 1 to 4 carbon atoms, cycloalkyl having 3 to 6 carbon atoms, the hydroxyl group or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkyl- or dialkylcarbamoyl in each case having 1 to 4 carbon atoms in each alkyl part, and phenyl or phenylalkyl which has 1 to 4 carbon atoms in the straight-chain or branched alkyl part and each of which is optionally monosubstituted or disubstituted by identical or different substituents from the series comprising halogen or straight-chain or branched alkyl and alkoxy in each case having 1 to 4 carbon atoms,

$R^I$ represents hydrogen or straight-chain or branched alkyl having 1 to 6 carbon atoms,

$R^{II}$ represents hydrogen, straight-chain or branched alkyl having 1 to 6 carbon atoms, phenylalkyl which has 1 to 4 carbon atoms in the straight-chain or branched alkyl part and which is optionally monosubstituted to pentasubstituted by identical or different substituents, phenyl substituents which may be mentioned being: halogen, alkyl and alkoxy in each case having 1 to 4 carbon atoms, halogenoalkyl and halogenoalkoxy in each case having 1 or 2 carbon atoms and 2 to 5 identical or different halogen atoms; $R^{II}$ furthermore represents alkoxycarbonylalkyl, carbamoylalkyl, alkylcarbamoylalkyl or dialkylcarbamoylalkyl in each case having 1 to 4 carbon atoms in each alkyl part, or cycloalkyl having 3 to 6 carbon atoms which is optionally

substituted by straight-chain or branched alkyl having 1 to 4 carbon atoms,

$R^{III}$ represents hydrogen, straight-chain or branched alkyl having 1 to 6 carbon atoms, phenylalkyl which has 1 to 4 carbon atoms in the straight-chain or branched alkyl part and which is optionally monosubstituted to pentasubstituted by identical or different substituents, suitable phenyl substituents being the phenyl substituents mentioned in the case of $R^{II}$; $R^{III}$ furthermore represents alkoxycarbonylalkyl, carbamoylalkyl, alkylcarbamoylalkyl or dialkylcarbamoylalkyl in each case having 1 to 4 carbon atoms in each alkyl part, or cycloalkyl having 3 to 6 carbon atoms which is optionally substituted by straight-chain or branched alkyl having 1 to 4 carbon atoms,

$R^{IV}$ represents hydrogen, straight-chain or branched alkyl having 1 to 6 carbon atoms, phenylalkyl which has 1 to 4 carbon atoms in the straight-chain or branched alkyl part and which is optionally monosubstituted to pentasubstituted by identical or different substituents, suitable phenyl substituents preferably being the phenyl substituents mentioned in the case of $R^{II}$; $R^{IV}$ furthermore represents acyl having 2 to 9 carbon atoms, and

n represents the numbers 0, 1 or 2,

and the geometrical and optical isomers and isomer mixtures thereof.

**3.** Process according to Claim 1, in which, in the formula (I),

R represents alkyl having 1 or 2 carbon atoms, cyanomethyl or cyanoethyl, allyl or propargyl, cyclopropyl, cyclohexyl, cyclopropylmethyl or cyclohexylmethyl which is in each case optionally monosubstituted, disubstituted or trisubstituted by methyl, or benzyl or phenethyl which is in each case optionally monosubstituted, disubstituted or trisubstituted by identical or different substituents from the series comprising halogen and methyl,

$R^1$ represents the $-B-CONR^3R^4$ group,

B represents the $-CH_2-$, $-CH(CH_3)-$ or $-CH_2CH_2-$groups,

$R^3$ represents monosubstituted or disubstituted, straight-chain or branched alkyl having 1 to 4 carbon atoms, substituents which may preferably be mentioned being: halogen, cyano, $-COOR^I$, $-CONR^{II}R^{III}$, $-NR^{II}R^{III}$, $-OR^{IV}$, $-S(O)_nR^{IV}$, acyl having 2 to 9 carbon atoms, phenyl which is optionally monosubstituted, disubstituted or trisubstituted by identical or different substituents from the series comprising halogen and alkyl having 1 to 4 carbon atoms, a 5- or 6-membered heterocyclic ring having 1 to 3 identical or different heteroatoms, which is optionally monosubstituted, disubstituted or trisubstituted by identical or different substituents from the series comprising halogen, straight-chain or branched alkyl and alkoxy in each case having 1 to 4 carbon atoms, and cycloalkyl having 3 to 6 carbon atoms and cycloalkenyl having 5 to 7 carbon atoms each of which is optionally substituted by straight-chain or branched alkyl having 1 to 4 carbon atoms; $R^3$ furthermore represents straight-chain or branched alkenyl or alkinyl in each case having 2 to 6 carbon atoms, cycloalkenyl having 5 to 7 carbon atoms which is optionally substituted by straight-chain or branched alkyl having 1 to 4 carbon atoms, optionally substituted cycloalkyl having 3 to 6 carbon atoms, substituents which may preferably be mentioned being: halogen, straight-chain or branched alkyl and alkoxy in each case having 1 to 4 carbon atoms, cyano, amino, carbamoyl, alkylamino, dialkylamino, alkylcarbamoyl and dialkylcarbamoyl in each case having 1 to 4 carbon atoms in each alkyl part, alkoxycarbonylamino having 1 to 4 carbon atoms in the straight-chain or branched alkoxy part, cycloalkyl and cycloalkylalkyl in each case having 5 or 6 carbon atoms in the cycloalkyl part and 1 to 4 carbon atoms in the straight-chain or branched alkyl part, pyrrolidone or phenyl which is optionally monosubstituted, disubstituted or trisubstituted by identical or different substituents from the series comprising halogen and alkyl having 1 to 4 carbon atoms; $R^3$ furthermore represents phenyl which is optionally monosubstituted, disubstituted or trisubstituted by identical or different substituents from the series comprising halogen, straight-chain or branched alkyl and alkoxy in each case having 1 to 4 carbon atoms, halogenoalkyl or halogenoalkoxy in each case having 1 or 2 carbon atoms and 1 to 5 identical or different halogen atoms, or a 5- or 6-membered heterocyclic ring having 1 to 3 identical or different heteroatoms, which is optionally monosubstituted, disubstituted or trisubstituted by identical or different substituents.

Substituents which may be mentioned are: halogen, mercapto, phenyl, straight-chain or branched alkyl, alkoxy, alkylthio, alkylsulphinyl and alkylsulphonyl having 1 to 4 carbon atoms per alkyl part;

$R^4$ represents the meanings of $R^3$, represents polycyclic carbocyclic rings or the $-OR^{IV}$ group,

or

$R^3$ represents hydrogen or straight-chain or branched alkyl having 1 to 4 carbon atoms, and

$R^4$ represents monosubstituted or disubstituted, straight-chain or branched alkyl having 1 to 4 carbon atoms, having the following substituents: acyl having 2 to 9 carbon atoms, hydroxyl, alkylcarbonyloxy having 1 to 4 carbon atoms in the straight-chain or branched alkyl part, $-S(O)_nR^{IV}$, a 5- or 6-membered heterocyclic ring having 1 to 3 heteroatoms, which is optionally monosubstituted, disubstituted or trisubstituted by identical or different substituents from the series comprising halogen, straight-chain or branched alkyl and alkoxy in each case having 1 to 4 carbon atoms, or cycloalkyl having 3 to 6 carbon atoms and cycloalkenyl having 5 to 7 carbon atoms each of which is optionally monosubstituted, disubstituted or trisubstituted by identical or different alkyl having 1 to 4 carbon atoms;

$R^4$ furthermore represents cycloalkenyl having 5 to 7 carbon atoms which is optionally monosubstituted, disubstituted or trisubstituted by identical or different, straight-chain or branched alkyl having 1 to 4 carbon atoms, represents a 5- or 6-membered heterocyclic ring having 1 to 3 identical or different heteroatoms, which is optionally monosubstituted, disubstituted or trisubstituted by identical or different substituents from the series comprising halogen, mercapto, phenyl, in each case straight-chain or branched alkyl, alkoxy, alkylthio, alkylsulphinyl and alkylsulphonyl in each case having 1 to 4 carbon atoms per alkyl part, represents cycloalkyl having 3 to 6 carbon atoms which is monosubstituted, disubstituted or trisubstituted by identical or different substituents from the series comprising phenyl, cyano, hydroxyl, alkoxy, alkylamino, dialkylamino, alkylcarbamoyl, dialkylcarbamoyl, alkoxycarbonylamino in each case having 1 to 4 carbon atoms per alkyl part, cycloalkyl and cycloalkylalkyl in each case having 5 to 7 carbon atoms in the cycloalkyl part and 1 to 4 carbon atoms in the alkyl part, additional cycloalkyl substituents which may be mentioned being, if appropriate, halogen and straight-chain or branched alkyl having 1 to 4 carbon atoms, and furthermore represents polycyclic carbocyclic rings or $-OR^{IV}$,

or

$R^3$ and $R^4$, together with the nitrogen atom to which they are bound, represent a monocyclic heterocyclic ring which is monosubstituted, disubstituted or trisubstituted by identical or different substituents, or a bi- or tricyclic heterocyclic ring or spiroheterocyclic ring which is optionally monosubstituted, disubstituted or trisubstituted by identical or different substituents, heterocyclic rings which may be mentioned being: oxazolidine, pyrrolidine, imidazolidine, piperidine, piperazine, morpholine, thiomorpholine, 1,3-oxazane or 1,3-diazane; these heterocyclic rings may in each case be fused, if appropriate, to 1 or 2 benzene or cyclohexane rings, or, if appropriate, bridged by methylene or ethylene.

Substituents which may be mentioned for all heterosystems are:

straight-chain or branched alkyl or alkoxy in each case having 1 to 4 carbon atoms, cycloalkyl having 3 to 6 carbon atoms, the hydroxyl group or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkyl- or dialkylcarbamoyl in each case having 1 to 4 carbon atoms in each alkyl part, and phenyl or phenylalkyl which has 1 to 4 carbon atoms in the straight-chain or branched alkyl part and each of which is optionally monosubstituted or disubstituted by identical or different substituents from the series comprising halogen or straight-chain or branched alkyl and alkoxy in each case having 1 to 4 carbon atoms,

$R^I$ represents hydrogen or straight-chain or branched alkyl having 1 to 6 carbon atoms,

$R^{II}$ represents hydrogen, straight-chain or branched alkyl having 1 to 4 carbon atoms, or benzyl or phenethyl each of which is optionally monosubstituted, disubstituted or

trisubstituted by identical or different substituents, phenyl substituents which may be mentioned in each case being: fluorine, chlorine, methyl, methoxy and trifluoromethyl; $R^{II}$ furthermore represents alkoxycarbonylalkyl having 1 to 4 carbon atoms in the alkoxy part and 1 or 2 carbon atoms in the alkyl part, carbamoylalkyl having 1 or 2 carbon atoms in the alkyl part, alkylcarbamoylalkyl and dialkylcarbamoylalkyl in each case having 1 or 2 carbon atoms in each alkyl part, or cycloalkyl having 3 to 6 carbon atoms which is optionally monosubstituted, disubstituted or trisubstituted by identical or different substituents from the series comprising methyl and ethyl,

$R^{III}$ represents the meanings of $R^{II}$,

$R^{IV}$ represents hydrogen, straight-chain or branched alkyl having 1 to 4 carbon atoms, or benzyl or phenethyl each of which is optionally monosubstituted, disubstituted or trisubstituted by identical or different substituents, phenyl substituents which may be mentioned in each case being: fluorine, chlorine, methyl, methoxy and trifluoromethyl; $R^{IV}$ furthermore represents acyl having 2 to 9 carbon atoms, and

n represents the numbers 0, 1 or 2,

and the geometrical and optical isomers and isomer mixtures thereof.

**4.** Process according to Claim 1, in which, in the formula (I),

R represents methyl, ethyl, cyanomethyl, cyanoethyl, allyl, propargyl, represents a cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents tthe $-B-CO-NR^3R^4$ group

B represents the $-CH_2-$, $-CH(CH_3)-$ or $-CH_2CH_2-$groups

$R^3$ represents monosubstituted or disubstituted alkyl having 1 to 3 carbon atoms, substituents which may be mentioned being: chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio in each case having 1 or 2 carbon atoms, 2-furyl, 2-pyridyl, 1-morpholino, cyclopropyl, cyclohexyl and phenyl which is optionally monosubstituted, disubstituted or trisubstituted by identical or different substituents from the series comprising chlorine and methyl; $R^3$ furthermore very particularly preferably represents allyl or propargyl, 1-cyclohexenyl, or cyclohexyl which is monosubstituted, disubstituted or trisubstituted by identical or different substituents, substituents which may be mentioned being: chlorine, methyl, ethyl, methoxy, cyano, amino, alkyl- and dialkylamino in each case having 1 or 2 carbon atoms in each alkyl part, carbamoyl, alkyl- and dialkylcarbamoyl in each case having 1 or 2 carbon atoms in each alkyl part, cyclohexyl, cyclohexylalkyl having 1 or 2 carbon atoms in the alkyl part and 1-pyrrolidin-2-one;

$R^3$ additionally represents phenyl which is optionally monosubstituted, disubstituted or trisubstituted by identical or different substituents from the series comprising chlorine and methyl, represents 2-pyridyl or 2-pyrimidinyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto, straight-chain or branched alkyl having 1 to 4 carbon atoms, in each case straight-chain or branched alkylthio, alkylsulphinyl and alkylsulphonyl in each case having 1 to 4 carbon atoms, or represents the

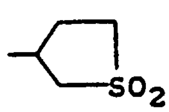

group,

$R^4$ represents monosubstituted or disubstituted alkyl having 1 to 3 carbon atoms, substituents

which may be mentioned being: chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio in each case having 1 or 2 carbon atoms, or 2-furyl, 2-pyridyl, 1-morpholino, cyclopropyl, cyclohexyl and phenyl which is optionally monosubstituted, disubstituted or trisubstituted by identical or different substituents from the series comprising chlorine and methyl; $R^4$ furthermore very particularly preferably represents allyl or propargyl, 1-cyclohexenyl, or cyclohexyl which is monosubstituted, disubstituted or trisubstituted by identical or different substituents, substituents which may be mentioned being: chlorine, methyl, ethyl, methoxy, cyano, amino, alkyl- or dialkylamino in each case having 1 or 2 carbon atoms in each alkyl part, carbamoyl, alkyl- and dialkylcarbamoyl in each case having 1 or 2 carbon atoms in each alkyl part, cyclohexyl, cyclohexylalkyl having 1 or 2 carbon atoms in the alkyl part, and 1-pyrrolidin-2-one; $R^4$ additionally represents phenyl which is optionally monosubstituted, disubstituted or trisubstituted by identical or different substituents from the series comprising chlorine and methyl, represents 2-pyridyl or 2-pyrimidinyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto, straight-chain or branched alkyl having 1 to 4 carbon atoms in each case straight-chain or branched alkylthio, alkylsulphinyl or alkylsulphonyl in each case having 1 to 4 carbon atoms, or represents the

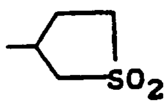

group,
$R^4$ furthermore represents hydroxyl, alkoxy having 1 or 2 carbon atoms, benzyloxy which is optionally monosubstituted, disubstituted or trisubstituted by identical or different substituents from the series comprising chlorine and methyl, represents 1-adamantyl, 2-norbornyl, 1- or 2-decalyl or 1- or 2-tetralyl.

**5.** Process according to Claim 1, in which, in the formula (I)

R represents methyl, ethyl, cyanomethyl, cyanoethyl, allyl, propargyl, represents cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents the $-B-CO-NR^3R^4$ group,

B represents the $-CH_2-$, $-CH(CH_3)-$ or $-CH_2CH_2-$groups,

$R^3$ represents hydrogen or straight-chain or branched alkyl having 1 to 4 carbon atoms,

$R^4$ represents substituted alkyl having 1 to 3 carbon atoms, substituents which may be mentioned being: hydroxyl, alkylcarbonyloxy having 1 to 4 carbon atoms in the straight-chain or branched alkyl part, alkylcarbonyl having 1 to 4 carbon atoms in the alkyl part, alkylthio having 1 or 2 carbon atoms, 2-furyl, 2-pyridyl, 1-morpholino, cyclopropyl and cyclohexyl; $R^4$ furthermore represents 1-cyclohexenyl, represents 2-pyridyl or 2-pyrimidyl each of which is optionally monosubstituted, disubstituted or trisubstituted by methyl, represents 2-thiazolyl which is optionally substituted by phenyl, represents 2-benzothiazolyl which is optionally monosubstituted, disubstituted or trisubstituted by identical or different substituents from the series comprising chlorine, methyl and methoxy, represents 1,2,4-triazol-3-yl, represents 1,2,4-thiadiazol-5-yl which is optionally substituted by phenyl, represents 1,3,4-thiadiazol-5-yl which is optionally monosubstituted, disubstituted or trisubstituted by identical or different substituents from the series comprising mercapto and alkyl, alkylthio, alkylsulphinyl and alkylsulphonyl in each case having 1 to 4 carbon atoms, represents the

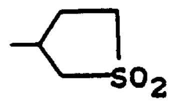

group,

represents 1-adamantyl, 2-norbornyl, 1- or 2-decalyl or 1- or 2-tetralyl; $R^4$ additionally represents cyclohexyl which is monosubstituted, disubstituted or trisubstituted by identical or different substituents, substituents which may be mentioned being: phenyl, cyano, hydroxyl, alkoxy having 1 or 2 carbon atoms, alkylamino and dialkylamino in each case having 1 or 2 carbon atoms in each alkyl part, alkylcarbamoyl and dialkylcarbamoyl in each case having 1 or 2 carbon atoms in each alkyl part, alkoxycarbonylamino having 1 or 2 carbon atoms in the alkyl part, cyclohexyl, cyclohexylalkyl having 1 or 2 carbon atoms in the alkyl part, and 1-pyrrolidin-2-one, and additionally halogen and alkyl having 1 or 2 carbon atoms.

**6.** Process according to Claim 1, in which, in the formula (I),

R represents methyl, ethyl, cyanomethyl, cyanoethyl, allyl, propargyl, represents cyclopropyl, cyclohexyl, cyclopropylmethyl or cyclohexylmethyl, each of which is monosubstituted, disubstituted or trisubstituted by methyl, or represents benzyl or phenethyl each of which is optionally monosubstituted, disubstituted or trisubstituted by identical or different substituents from the series comprising halogen and methyl,

$R^1$ represents the -B-CO-$NR^3R^4$ group,

B represents the $-CH_2-$, $-CH(CH_3)-$ or $-CH_2CH_2-$groups,

$R^3$ and $R^4$, together with the nitrogen atom to which they are bound, represent the following mono-, bi- or tricyclic heterocyclic rings or spiroheterocyclic rings:

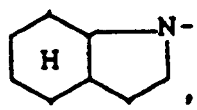,
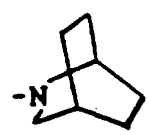,
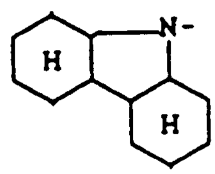,

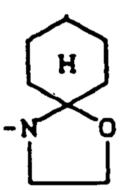,
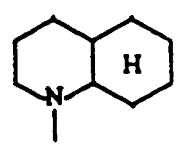,

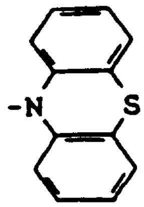 , 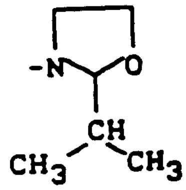 , 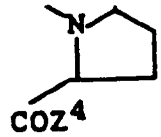

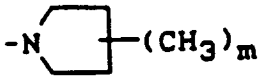 , 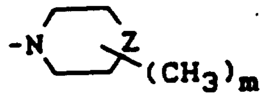

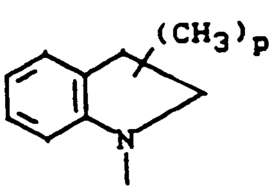 , 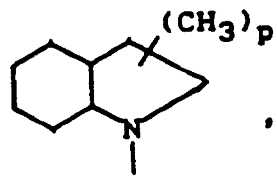 ,

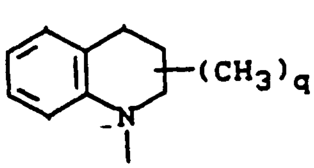 , 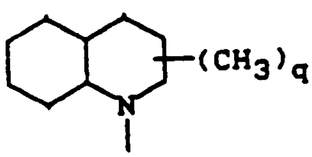 ,

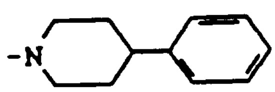 , 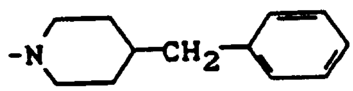 ,

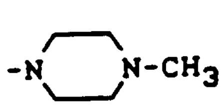 , 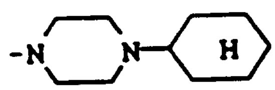 , 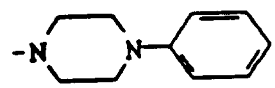 ,

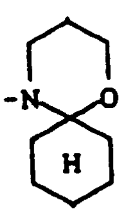 , 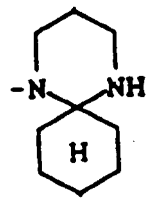 , 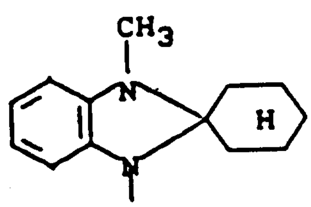 ,

where

Z represents oxygen or the $CH_2$ group,

**7.** Pesticides, characterised in that they contain at least one (2-cyano-2-oximinoacetyl)-amino acid derivative of the formula (I) according to Claims 1 and 6.

**8.** Use of (2-cyano-2-oximinoacetyl)-amino acid derivatives of the formula (I) according to Claims 1 and 6 for combating pests.

**9.** Process for combating pests, characterised in that (2-cyano-2-oximinoacetyl)-amino acid derivatives of the formula (I) according to Claims 1 and 6 are allowed to act on the pests and/or on their environment.

**10.** Process for the production of pesticides, characterised in that (2-cyano-2-oximinoacetyl)-amino acid derivatives of the formula (I) according to Claims 1 and 6 are mixed with extenders and/or surface-active agents.

## Revendications

**Revendications pour les Etats contractants suivants : AT, BE, CH, DE, FR, GB, GR, IT, LI, NL**

**1.** Dérivés de (2-cyano-2-oximinoacétyl)-aminoacides de formule générale (I)

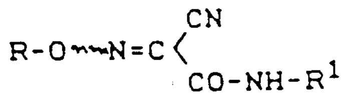

(I)

dans laquelle

R représente un groupe alkyle, cyanalkyle, ou un groupe alcényle, alcynyle, cycloalkyle, cycloalkylalkyle ou phénylalkyle, chacun d'eux éventuellement substitué,

$R^1$ représente le groupement $-B-CONR^3R^4$ dans lequel

B représente une chaîne alkylène droite ou ramifiée, éventuellement substituée,

$R^3$ représente un groupe alkyle substitué par les substituants suivants : les halogènes, les groupes cyano, $-COOR^I$, $-CONR^{II}R^{III}$, $-NR^{II}R^{III}$, $-OR^{IV}$, $-S(O)_nR^{IV}$, acyle, les groupes aryle, hétérocyclyle, cycloalkyle et cycloalcényle, chacun d'eux éventuellement substitué ; $R^3$ peut en outre représenter un groupe alcényle, alcynyle, cycloalkyle, cycloalcényle, aryle ou hétérocyclyle, chacun d'eux éventuellement substitué et

$R^4$ représente un groupe alkyle substitué par les substituants suivants : les halogènes, les groupes cyano, $-COOR^I$, $-CONR^{II}R^{III}$, $-NR^{II}R^{III}$, $-OR^{IV}$, $-S(O)_nR^{IV}$, acyle, des groupes aryle, hétérocyclyle, cycloalkyle et cycloalcényle, chacun d'eux éventuellement substitué ; $R^4$ peut en outre représenter un groupe alcényle, alcynyle, cycloalkyle, cycloalcényle, aryle, hétérocyclyle ou un carbocycle polycyclique, chacun d'eux éventuellement substitué ou le groupement $-OR^{IV}$,

ou bien

$R^3$ représente l'hydrogène ou un groupe alkyle et

$R^4$ représente un groupe alkyle substitué par les substituants suivants : les groupes acyle, hydroxy, alkylcarbonyloxy, $-S(O)_nR^{IV}$, les groupes hétérocyclyle, cycloalkyle et cycloalcényle, chacun d'eux éventuellement substitué ; $R^4$ peut en outre représenter un groupe cycloalcényle, hétérocyclyle ou un carbocycle polycyclique, chacun d'eux éventuellement substitué, un groupe cycloalkyle substitué sans toutefois que des halogènes et des groupes alkyle puissent être présents en tant qu'unique substituant, ou le groupement $OR^{IV}$,

ou bien

$R^3$et $R^4$ forment ensemble et avec l'atome d'azote auquel ils sont reliés un hétérocycle monocyclique substitué ou un hétérocycle ou spirohétérocycle bi- ou tri-cyclique éventuellement substitué contenant un à trois autres hétéroatomes identiques ou différents,

$R^I$ représente l'hydrogène ou un groupe alkyle éventuellement substitué par les substituants suivants : les halogènes, les groupes aryle, cycloalkyle et cycloalcényle, chacun d'eux éventuellement substitué,

$R^{II}$ et $R^{III}$ , ayant des significiations identiques ou différentes, représentent chacun l'hydrogène ou un groupe alkyle éventuellement substitué par les substituants suivants : les halogènes, les groupes $-COOR^{IV}$, $-CONR^IR^{IV}$, les groupes aryle, cycloaklyle et cycloalcényle, chacun d'eux éventuellement substitué ; $R^{II}$ et $R^{III}$ peuvent en outre représenter chacun un groupe alcényle, alcynyle ou un groupe cycloalkyle, cycloalcényle, aryle ou hétérocyclyle, chacun d'eux éventuellement substitué, ou former ensemble et avec l'atome d'azote auquel ils sont reliés un hétérocycle éventuellement substitué qui peut contenir d'autres hétéroatomes,

$R^{IV}$ représente l'hydrogène, un groupe alkyle, aralkyle ou acyle

n est égal à 0, 1 ou 2,

leurs isomères et mélanges d'isomères géométriques et optiques.

**2.** Dérivés de (2-cyano-2-oximino-acétyl)-aminoacides selon la revendication 1, pour lesquels, dans la formule (I),

R représente un groupe alkyle à chaîne droite ou ramifiée en $C_1$-$C_4$, un groupe cyanalkyle à chaîne droite ou ramifiée contenant 1 à 4 atomes de carbone dans la partie alkyle, un groupe alcényle ou alcynyle, chacun à chaîne droite ou ramifiée et contenant chacun 2 à 6 atomes de carbone, un groupe cycloalkyle ou cycloalkylalkyle contenant chacun 3 à 6 atomes de carbone par partie cycloalkyle et 1 à 4 atomes de carbone par partie alkyle à chaîne droite ou ramifiée, chacun d'eux portant éventuellement 1 à 5 substituants identiques ou différents choisis parmi les groupes alkyle en $C_1$-$C_4$, ou un groupe benzyle ou phénéthyle portant chacun le cas échéant 1 à 5 substituants identiques ou différents, les substituants de chacune des parties phényle étant : des halogènes, des groupes alkyle et alcoxy contenant chacun 1 à 4 atomes de carbone, des groupes halogénoalkyle et halogénoalcoxy contenant chacun 1 ou 2 atomes de carbone et 2 à 5 atomes d'halogène identiques ou différents ;

$R^1$ représente les groupements -A-$COOR^2$ ou -B-$CONR^3R^4$,

B représente une chaîne alkylène droite ou ramifiée en $C_1$-$C_4$,

$R^3$ représente un groupe alkyle à chaîne droite ou ramifiée en $C_1$-$C_4$ portant 1 ou 2 substituants, les substituants étant : les halogènes, les groupes cyano, -$COOR^I$, -$CONR^{II}R^{III}$, -$NR^{II}R^{III}$, -$OR^{IV}$, -$S(O)_nR^{IV}$, acyle en $C_2$-$C_9$, les groupes phényle portant éventuellement 1 à 5 substituants identiques ou différents choisis parmi les halogènes, les groupes alkyle en $C_1$-$C_4$ ; les hétérocycles à 5 ou 6 chaînons et 1 à 3 hétéroatomes portant éventuellement 1 à 5 substituants identiques ou différents choisis parmi les halogènes et les groupes alkyle et alcoxy contenant chacun de 1 à 4 atomes de carbone, les groupes cycloalkyle en $C_3$-$C_6$ ou cycloalcényle en $C_5$-$C_7$ contenant chacun le cas échéant 1 à 5 substituants identiques ou différents choisis parmi les groupes alkyle en $C_1$-$C_4$ ; $R^3$ peut en outre représenter avantageusement un groupe alcényle ou alcynyle à chaîne droite ou ramifiée contenant chacun 2 à 6 atomes de carbone ; un groupe cycloalcényle en $C_5$-$C_7$ portant éventuellement 1 à 5 substituants identiques ou différents choisis parmi les groupes alkyle en $C_1$-$C_4$, ou un groupe cycloalkyle en $C_3$-$C_6$ portant éventuellement 1 à 5 substituants identiques ou différents, ces substituants étant : des halogènes, des groupes alkyle et alcoxy contenant chacun 1 à 4 atomes de carbone, des groupes cyano, amino, carbamoyle, alkylamino, dialkylamino, alkylcarbamoyle et dialkylcarbamoyle contenant chacun 1 à 4 atomes de carbone dans chacune des parties alkyle, alcoxycarbonylamino contenant 1 à 4 atomes de carbone dans chacune des parties alcoxy, cycloalkyle et cycloalkylalkyle contenant 5 ou 6 atomes de carbone dans la partie cycloalkyle et 1 à 4 atomes de carbone dans la partie alkyle à chaîne droite ou ramifiée, phényle et pyrrolidone portant éventuellement 1 à 5 substituants identiques ou différents choisis parmi les halogènes et les groupes alkyle et alcoxy en $C_1$-$C_4$ ; $R^3$ peut en outre représenter avantageusement un groupe phényle portant éventuellement 1 à 5 substituants identiques ou différents choisis parmi les halogènes, les groupes alkyle et alcoxy contenant chacun 1 à 4 atomes de carbone, les groupes halogénoalkyle et halogénoalcoxy contenant chacun 1 ou 2 atomes de carbone et 1 à 5 atomes d'halogènes identiques ou différents, ou un hétérocycle à 5 ou 6 chaînons et 1 à 3 hétéroatomes identiques ou différents portant éventuellement 1 à 5 substituants identiques ou différents, ces substituants étant : des halogènes, des groupes mercapto, phényle, des groupes alkyle, alcoxy, alkylthio, alkylsulfinyle, alkylsulfonyle contenant chacun 1 à 4 atomes de carbone à chaîne droite ou ramifiée par partie alkyle ;

$R^4$ a les significations de $R^3$, représente un carbocycle polycyclique ou le groupement -$OR^{IV}$,

ou bien

$R^3$ représente l'hydrogène ou un groupe alkyle à chaîne droite ou ramifiée en $C_1$-$C_4$,

$R^4$ représente un groupe alkyle à chaîne droite ou ramifiée en $C_1$-$C_4$ portant 1 ou 2 substituants choisis parmi les suivants : les groupes acyle en $C_2$-$C_9$, hydroxy, alkylcarbonyloxy contenant de 1 à 4 atomes de carbone dans la partie alkyle à chaîne droite ou ramifiée, -$S(O)_nR^{IV}$, les hétérocycles à 5 ou 6 chaînons et 1 à 3 hétéroatomes

portant éventuellement 1 à 5 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ portant chacun le cas échéant 1 à 5 substituants identiques ou différents choisis parmi les groupes alkyle en $C_1$-$C_4$,

$R^4$ peut en outre représenter un groupe cycloalcényle en $C_5$-$C_7$ portant éventuellement 1 à 5 substituants identiques ou différents choisis parmi les groupes alkyle à chaîne droite ou ramifiée en $C_1$-$C_4$, un hétérocycle à 5 ou 6 chaînons et 1 à 3 hétéroatomes identiques ou différents portant éventuellement 1 à 5 substituants identiques ou différents choisis parmi les halogènes, les groupes mercapto, phényle, les groupes alkyle, alcoxy, alkylthio, alkylsulfinyle, alkylsulfonyle contenant 1 à 4 atomes de carbone par partie alkyle et chacun d'eux à chaîne droite ou ramifiée, un groupe cycloalkyle en $C_3$-$C_6$ portant 1 à 5 substituants identiques ou différents choisis parmi les groupes phényle, cyano, hydroxy, alcoxy, alkylamino, dialkylamino, alkylcarbamoyle, dialkylcarbamoyle, alcoxycarbonylamino contenant chacun 1 à 4 atomes de carbone par partie alkyle, cycloalkyle et cycloalkylalkyle contenant chacun 5 à 7 atomes de carbone dans la partie cycloalkyle et 1 à 4 atomes de carbone dans la partie alkyle, avec éventuellement des halogènes et des groupes alkyle à chaîne droite ou ramifiée en $C_1$-$C_4$ en tant qu'autres substituants du groupe cycloalkyle, ou un carbocycle polycyclique ou -$OR^{IV}$, ou bien

$R^3$ et $R^4$ forment ensemble et avec l'atome d'azote auquel ils sont reliés un hétérocycle monocyclique portant 1 à 5 substituants identiques ou différents ou un hétérocycle ou spirohétérocycle bi- ou tri-cyclique portant éventuellement 1 à 5 substituants identiques ou différents, ces hétérocycles pouvant consister par exemple en cycles : oxazolidine, pyrrolidine, imidazolidine, pipéridine, pipérazine, morpholine, thiomorpholine, 1,3-oxazane ou 1,3-diazane ; ces hétérocycles pouvant tous être éventuellement condensés avec 1 ou 2 cycles benzène ou cyclohexane, ou être éventuellement reliés par des ponts méthylène ou éthylène.

En tant que substituants pour tous les systèmes hétérocycles, on citera : les groupes alkyle ou alcoxy à chaîne droite ou ramifiée contenant chacun 1 à 4 atomes de carbone, les groupes cycloalkyle en $C_3$-$C_6$, les groupes hydroxy ou oxo, les groupes alcényle à chaîne droite ou ramifiée en $C_2$-$C_4$, hydroxycarbonyle, alcoxycabonyle contenant 1 à 4 atomes de carbone dans la partie alkyle à chaîne droite ou ramifiée, carbamoyle, alkyl- ou dialkylcarbamoyle contenant chacun 1 à 4 atomes de carbone dans chacune des parties alkyle, et les groupes phényle ou phénylalkyle contenant 1 à 4 atomes de carbone dans la partie alkyle à chaîne droite ou ramifiée et portant éventuellement 1 ou 2 substituants identiques ou différents choisis parmi les halogènes et les groupes alkyle et alcoxy à chaîne droite ou ramifiée contenant chacun 1 à 4 atomes de carbone,

$R^I$ représente l'hydrogène ou un groupe alkyle à chaîne droite ou ramifiée en $C_1$-$C_6$,

$R^{II}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du noyau phényle étant : des halogènes, des groupes alkyle et alcoxy contenant chacun 1 à 4 atomes de carbone, des groupes halogénoalkyle et halogénoalcoxy contenant chacun 1 ou 2 atomes de carbone et 2 à 5 atomes d'halogène identiques ou différents ; $R^{II}$ peut en outre représenter un groupe alcoxycarbonylalkyle, carbamoylalkyle, alkylcarbamoylalkyle ou dialkylcarbamoylalkyle contenant chacun 1 à 4 atomes de carbone dans chacune des parties alkyle, ou un groupe cycloalkyle en $C_3$-$C_6$ éventuellement substitué par des groupes alkyle à chaîne droite ou ramifiée en $C_1$-$C_4$,

$R^{III}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du groupe phényle étant ceux qui ont été mentionnés en référence à $R^{II}$ ; $R^{III}$ peut en outre représenter un groupe alcoxycarbonylalkyle, carbamoylalkyle, alkylcarbamoylalkyle ou dialkylcarbamoylalkyle contenant chacun 1 à 4 atomes de carbone dans chacune des parties alkyle ou un groupe cycloalkyle en $C_3$-$C_6$ éventuel-

lement substitué par des groupes alkyle à chaîne droite ou ramifiée en $C_1$-$C_4$ ;

$R^{IV}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du noyau phényle étant de préférence ceux qui ont été mentionnés en référence à $R^{II}$ ; $R^{IV}$ peut en outre représenter un groupe acyle en $C_2$-$C_9$, et

n est égal à 0, 1 ou 2,

leurs isomères et mélanges d'isomères géométriques et optiques.

**3.** Dérivés de (2-cyano-2-oximino-acétyl)-aminoacides de la revendication 1, pour lesquels, dans la formule (I) :

R représente un groupe alkyle en $C_1$ ou $C_2$, un groupe cyanométhyle ou cyanéthyle, un groupe allyle ou propargyle, un groupe cyclopropyle, cyclohexyle, cyclopropylméthyle, ou cyclohexylméthyle, portant éventuellement chacun 1 à 3 substituants méthyle, ou un groupe benzyle ou phénéthyle portant chacun, le cas échéant, 1 à 3 substituants identiques ou différents choisis parmi les halogènes et les groupes méthyle,

$R^1$ représente le groupement $-B-CONR^3R^4$,

B représente les groupements $-CH_2$, $-CH(CH_3)-$ ou $-CH_2CH_2-$,

$R^3$ représente un groupe alkyle à chaîne droite ou ramifiée en $C_1$-$C_4$ portant un ou deux substituants, ces substituants consistant de préférence en : des halogènes, des groupes cyano, $-COOR^I$, $-CONR^{II}R^{III}$, $NR^{II}R^{III}$, $-OR^{IV}$, $-S(O)_nR^{IV}$, les groupes acyle en $C_2$-$C_9$, les groupes phényle portant éventuellement 1 à 3 substituants identiques ou différents choisis parmi les halogènes et les groupes alkyle en $C_1$-$C_4$, les hétérocycles à 5 ou 6 chaînons et 1 à 3 hétéroatomes identiques ou différents portant éventuellement 1 à 3 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ chacun éventuellement substitué par des groupes alkyle à chaîne droite ou ramifiée en $C_1$-$C_4$ ; $R^3$ peut en outre représenter un groupe alcényle ou alcynyle à chaîne droite ou ramifiée contenant chacun 2 à 6 atomes de carbone, un groupe cycloalcényle en $C_5$-$C_7$ éventuellement substitué par des groupes alkyle à chaîne droite ou ramifiée en $C_1$-$C_4$, un groupe cycloalkyle en $C_3$-$C_6$ éventuellement substitué, les substituants étant de préférence : les halogènes, des groupes alkyle et alcoxy à chaîne droite ou ramifiée contenant chacun 1 à 4 atomes de carbone, des groupes cyano, amino, carbamoyle, alkylamino, dialkylamino, alkylcarbamoyle et dialkylcarbamoyle contenant chacun 1 à 4 atomes de carbone dans chacune des parties alkyle, alcoxycarbonylamino contenant 1 à 4 atomes de carbone dans la partie alcoxy à chaîne droite ou ramifiée, cycloalkyle et cycloalkylalkyle contenant chacun 5 ou 6 atomes de carbone dans la partie cycloalkyle et 1 à 4 atomes de carbone dans la partie alkyle à chaîne droite ou ramifiée, les groupes phényle et pyrrolidone portant éventuellement 1 à 3 substituants identiques ou différents choisis parmi les halogènes et les groupes alkyle en $C_1$-$C_4$ ; $R^3$ peut en outre représenter un groupe phényle portant éventuellement 1 à 3 substituants identiques ou différents choisis parmi les halogènes, les groupes alkyle ou alcoxy à chaîne droite ou ramifiée contenant chacun 1 à 4 atomes de carbone, les groupes halogénoalkyle ou halogénoalcoxy contenant chacun 1 ou 2 atomes de carbone et 1 à 5 atomes d'halogènes identiques ou différents, ou un hétérocycle à 5 ou 6 chaînons et 1 à 3 hétéroatomes identiques ou différents, portant éventuellement 1 à 3 substituants identiques ou différents, consistant en : des halogènes, des groupes mercapto, phényle, des groupes alkyle, alcoxy alkylthio, alkylsulfinyle et alkylsulfonyle, à chaîne droite ou ramifiée contenant chacun 1 à 4 atomes de carbone par partie alkyle ;

$R^4$ a les significations de $R^3$, représente un carbocycle polycyclique ou le groupement $-OR^{IV}$,

ou bien

$R^3$ représente l'hydrogène ou un groupe alkyle à chaîne droite ou ramifiée en $C_1$-$C_4$,

et

$R^4$ représente un groupe alkyle en $C_1$-$C_4$ à chaîne droite ou ramifiée portant 1 ou 2 substituants pris parmi les suivants : les groupes acyle en $C_2$-$C_9$, hydroxy, alkylcarbonyloxy contenant 1 à 4 atomes de carbone dans la partie alkyle à chaîne droite ou

ramifiée, $-S(O)_nR^{IV}$, les hétérocycles à 5 ou 6 chaînons et 1 à 3 hétéroatomes portant éventuellement 1 à 3 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ portant chacun le cas échéant 1 à 3 substituants identiques ou différents choisis parmi les groupes alkyle en $C_1$-$C_4$ ;

$R^4$ peut en outre représenter un groupe cycloalcényle en $C_5$-$C_7$ portant éventuellement 1 à 3 substituants identiques ou différents choisis parmi les groupes alkyle à chaîne droite ou ramifiée en $C_1$-$C_4$, un hétérocycle à 5 ou 6 chaînons et 1 à 3 hétéroatomes identiques ou différents, portant éventuellement 1 à 3 substituants identiques ou différents choisis parmi les halogènes, les groupes mercapto, phényle, des groupes alkyle, alcoxy, alkylthio, alkylsulfinyle et alkylsulfonyle à chaîne droite ou ramifiée contenant chacun 1 à 4 atomes de carbone par partie alkyle, un groupe cycloalkyle en $C_3$-$C_6$ portant 1 à 3 substituants identiques ou différents choisis parmi les groupes phényle, cyano, hydroxy, alcoxy, alkylamino, dialkylamino, alkylcarbamoyle, dialkylcarbamoyle, alcoxycarbonylamino contenant chacun 1 à 4 atomes de carbone par partie alkyle, cycloalkyle et cycloalkylalkyle contenant chacun 5 à 7 atomes de carbone dans la partie cycloalkyle et 1 à 4 atomes de carbone dans la partie alkyle, avec le cas échéant des halogènes et des groupes alkyles à chaîne droite ou ramifiée en $C_1$-$C_4$ en tant qu'autres substituants du groupe cycloalkyle, ou bien un carbocycle polycyclique ou un groupe $-OR^{IV}$, ou bien

$R^3$ et $R^4$ forment ensemble et avec l'atome d'azote auquel ils sont reliés un hétérocycle monocyclique portant 1 à 3 substituants identiques ou différents ou un hétérocycle ou spirohétérocycle bi- ou tri-cyclique portant éventuellement 1 à 3 substituants identiques ou différents, les hétérocycles consistant en cycles : oxazolidine, pyrrolidine, imidazolidine, pipéridine, pipérazine, morpholine, thiomorpholine, 1,3-oxazane ou 1,3-diazane ; ces hétérocycles pouvant tous être le cas échéant condensés avec 1 ou 2 cycles benzène ou cyclohexane, ou reliés éventuellement par des ponts méthylène ou éthylène.

En tant que substituants pour tous les systèmes hétérocycliques, on citera : des groupes alkyle ou alcoxy à chaîne droite ou ramifiée contenant chacun 1 à 4 atomes de carbone, des groupes cycloalkyle en $C_3$-$C_6$, des groupes hydroxy ou oxo, des groupes alcényle à chaîne droite ou ramifiée en $C_2$-$C_4$, hydroxycarbonyle, alcoxycarbonyle contenant 1 à 4 atomes de carbone dans la partie alkyle à chaîne droite ou ramifiée, carbamoyle, alkyl- ou dialkyl-carbamoyle contenant 1 à 4 atomes de carbone dans chaque partie alkyle, et les groupes phényle ou phénylalkyle contenant 1 à 4 atomes de carbone dans la partie alkyle à chaîne droite ou ramifiée et portant chacun le cas échéant 1 ou 2 substituants identiques ou différents choisis parmi les halogènes et les groupes alkyle et alcoxy à chaîne droite ou ramifiée contenant chacun 1 à 4 atomes de carbone,

$R^I$ représente l'hydrogène ou un groupe alkyle à chaîne droite ou ramifiée en $C_1$-$C_4$,

$R^{II}$ représente l'hydrogène, un groupe alkyle à chaîne droite ou ramifiée en $C_1$-$C_4$, un groupe benzyle ou phénéthyle portant chacun le cas échéant 1 à 3 substituants identiques ou différents, les substituants du noyau phényle consistant en : le fluor, le chlore, les groupes méthyle, méthoxy et trifluorométhyle ; $R^{II}$ peut en outre représenter un groupe alcoxycarbonylaklyle contenant 1 à 4 atomes de carbone dans la partie alcoxy et 1 ou 2 atomes de carbone dans la partie alkyle, un groupe carbamoylalkyle contenant 1 ou 2 atomes de carbone dans la partie alkyle, un groupe alkylcarbamoylalkyle et dialkylcarbamoylalkyle contenant chacun 1 ou 2 atomes de carbone dans chaque partie aklyle, ou un groupe cycloalkyle en $C_3$-$C_6$ portant éventuellement 1 à 3 substituants identiques ou différents choisis parmi les groupes méthyle et éthyle,

$R^{III}$ a les significations de $R^{II}$,

$R^{IV}$ représente l'hydrogène, un groupe alkyle à chaîne droite ou ramifiée en $C_1$-$C_4$, un groupe benzyle ou phénéthyle portant chacun éventuellement 1 à 3 substituants identiques ou différents, les substituants du noyau phényle étant : le fluor, le chlore, les groupes méthyle, méthoxy et trifluorométhyle ; $R^{IV}$ peut en outre représenter un groupe acyle en $C_2$-$C_9$, et

n est égal à 0, 1 ou 2,

leurs isomères et mélanges d'isomères géométriques et optiques.

**4.** Dérivés de (2-cyano-2-oximinoacétyl)-aminoacides selon la revendication 1, pour lesquels, dans la formule (I)

R représente un groupe méthyle, éthyle, cyanométhyle, cyanéthyle, allyle, propargyle, un groupe cyclopropyle, cyclohexyle, cyclopropylméthyle ou cyclohexylméthyle portant chacun 1 à 3 substituants méthyle, ou un groupe benzyle ou phénéthyle portant chacun le cas échéant 1 à 3 substituants identiques ou différents choisis parmi les halogènes et les groupes méthyle,

$R^1$ représente le groupement $-B-CO-NR^3R^4$ dans lequel

B représente les groupements $-CH_2-$, $-CH(CH_3)-$ ou $-CH_2CH_2-$,

$R^3$ représente un groupe alkyle en $C_1$-$C_3$ portant 1 ou 2 substituants consistant en le chlore, des groupes hydroxy, alkylcarbonyloxy contenant 1 à 4 atomes de carbone dans la partie alkyle à chaîne droite ou ramifiée, alkylcarbonyle contenant 1 à 4 atomes de carbone dans la partie alkyle à chaîne droite ou ramifiée, alcoxy ou alkylthio contenant 1 ou 2 atomes de carbone, phényle, 2-furyle, 2-pyridyle, 1-morpholino, cyclopropyle et cyclohexyle portant chacun éventuellement 1 à 3 substituants identiques ou différents choisis parmi le chlore et les groupes méthyle ; $R^3$ peut en outre représenter de manière particulièrement avantageuse un groupe allyle ou propargyle, un groupe 1-cyclohexényle ou un groupe cyclohexyle portant 1 à 3 substituants identiques ou différents, les substituants consistant en : le chlore, les groupes méthyle, éthyle, méthoxy, cyano, amino, alkyl- et dialkylamino contenant chacun 1 ou 2 atomes de carbone dans chaque partie alkyle, carbamoyle, alkyl- et dialkylcarbamoyle contenant chacun 1 ou 2 atomes de carbone dans chaque partie alkyle, cyclohexyle, cyclohexylalkyle contenant chacun 1 ou 2 atomes de carbone dans la partie alkyle et 1-pyrrolidine-2-one ;

$R^3$ peut en outre représenter un groupe phényle portant éventuellement 1 à 3 substituants identiques ou différents choisis parmi le chlore et les groupes méthyle, un groupe 2-pyridyle ou 2-pyrimidinyle portant chacun éventuellement 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadiazole-5-yle éventuellement substitué par un groupe phényle, un groupe 1,3,4-thiadiazole-5-yle portant éventuellement 1 à 3 substituants identiques ou différents choisis parmi les groupes mercapto, les groupes alkyle à chaîne droite ou ramifiée en $C_1$-$C_4$, les groupes alkylthio, alkylsulfinyle et alkylsulfonyle à chaîne droite ou ramifiée contenant chacun 1 à 4 atomes de carbone, ou le groupement :

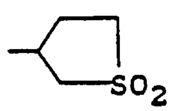 ,

$R^4$ représente un groupe alkyle en $C_1$-$C_3$ portant 1 ou 2 substituants consistant en : le chlore, les groupes hydroxy, alkylcarbonyloxy contenant chacun 1 à 4 atomes de carbone dans la partie alkyle à chaîne droite ou ramifiée, alkylcarbonyle contenant 1 à 4 atomes de carbone dans la partie alkyle à chaîne droite ou ramifiée, alcoxy et alkylthio contenant chacun 1 ou 2 atomes de carbone, les groupes phényle, 2-furyle, 2-pyridyle, 1-morpholino, cyclopropyle et cyclohexyle portant le cas échéant 1 ou 3 substituants identiques ou différents choisis parmi le chlore et les groupes méthyle ; $R^4$ peut en outre représenter de manière particulièrement avantageuse un groupe allyle ou propargyle, un groupe 1-cyclohexényle ou un groupe cyclohexyle portant 1 à 3 substituants identiques ou différents consistant en : le chlore, des groupes méthyle, éthyle, méthoxy, cyano, amino, alkyl- ou dialkylamino contenant chacun 1 ou 2 atomes de carbone dans chaque partie alkyle, les groupes carbamoyle, alkyl- et dialkylcarbamoyle contenant chacun 1 ou 2 atomes de carbone dans la partie alkyle, les groupes cyclohexyle, cyclohexylalkyle contenant 1 ou 2 atomes de carbone dans la partie alkyle, et 1-pyrrolidine-2-one ;

$R^4$ peut en outre représenter un groupe phényle portant éventuellement 1 à 3 substituants

identiques ou différents choisis parmi le chlore et les groupes méthyle, un groupe 2-pyridyle ou 2-pyrimidinyle portant chacun le cas échéant 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadiazole-5-yle éventuellement substitué par un groupe phényle, un groupe 1,3,4-thiadiazole-5-yle portant éventuellement 1 à 3 substituants identiques ou différents choisis parmi les groupes mercapto, les groupes alkyle à chaîne droite ou ramifiée en $C_1$-$C_4$, les groupes alkylthio, les groupes alkylsulfinyle ou alkylsulfonyle à chaîne droite ou ramifiée contenant 1 à 4 atomes de carbone, ou le groupement

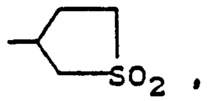

$R^4$ peut en outre représenter un groupe hydroxy, alcoxy en $C_1$-$C_2$, un groupe benzyloxy portant éventuellement 1 à 3 substituants identiques ou différents choisis parmi le chlore et les groupes méthyle, un groupe 1-adamantyle, 2-norbornyle, 1- ou 2-décalyle ou 1- ou 2-tétralyle,

**5.** Dérivés de (2-cyano-2-oximino-acétyl)-aminoacides selon la revendication 1, pour lesquels, dans la formule (I) :

R représente un groupe méthyle, éthyle, cyanométhyle, cyanéthyle, allyle, propargyle, un groupe cylcopropyle, cyclohexyle, cyclopropylméthyle ou cyclohexylméthyle portant chacun 1 à 3 substituants méthyle, ou un groupe benzyle ou phénéthyle, portant chacun 1 ou 2 substituants identiques ou différents choisis parmi les halogènes et les groupes méthyle,

$R^1$ représente le groupement $-B-CO-NR^3R^4$,

B représente les groupements $-CH_2-$, $-CH(CH_3)-$ ou $-CH_2CH_2-$,

$R^3$ représente l'hydrogène ou un groupe alkyle à chaîne droite ou ramifiée en $C_1$-$C_4$,

$R^4$ représente un groupe aklyle substitué en $C_1$-$C_3$, les substituants consistant en : des groupes hydroxy, alkylcarbonyloxy contenant 1 à 4 atomes de carbone dans la partie alkyle à chaîne droite ou ramifiée, des groupes alkylcarbonyle contenant 1 à 4 atomes de carbone dans la partie alkyle, alkylthio en $C_1$ ou $C_2$, 2-furyle, 2-pyridyle, 1-morpholino, cyclopropyle et cyclohexyle ; $R^4$ peut en outre représenter un groupe 1-cyclohexényle, un groupe 2-pyridyle ou 2-pyrimidinyle, portant chacun le cas échéant 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadizole-5-yle éventuellement substitué par un groupe phényle, un groupe 1,3,4-thiadiazole-5-yle portant éventuellement 1 à 3 substituants identiques ou différents choisis parmi les groupes mercapto, et les groupes alkyle, alkylthio, alkylsulfinyle et alkylsulfonyle contenant chacun 1 à 4 atomes de carbone, le groupement

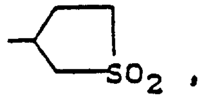

un groupe 1-adamantyle, 2-norbornyle, 1- ou 2-décalyle ou 1- ou 2-tétralyle ; $R^4$ peut en outre représenter un groupe cyclohexyle portant 1 à 3 substituants identiques ou différents, les substituants consistant en : des groupes phényle, cyano, hydroxy, alcoxy en $C_1$ ou $C_2$, alkylamino et dialkylamino contenant chacun 1 ou 2 atomes de carbone dans chaque partie alkyle, alkylcarbamoyle et dialkylcarbamoyle contenant chacun 1 ou 2 atomes de carbone dans chaque partie alkyle, alcoxycarbonylamino contenant 1 ou 2 atomes de carbone dans la partie alkyle, cyclohexyle, cyclohexylalkyle contenant 1 ou 2 atomes de carbone dans la partie alkyle, et 1-pyrrolidine-2-one, et en outre les halogènes ou les groupes alkyle en $C_1$ ou $C_2$.

**6.** Dérivés de (2-cyano-2-oximino-acétyl)-aminoacides selon la revendication 1, pour lesquels, dans la formule (I) :

R représente un groupe méthyle, éthyle, cyanométhyle, cyanéthyle, allyle, propargyle, un groupe cyclopropyle, cyclohexyle, cyclopropylméthyle ou cyclohexylméthyle portant chacun 1 à 3 substituants méthyle, ou un groupe benzyle ou phénéthyle portant chacun le cas échéant 1 à 3 substituants identiques ou différents choisis parmi les halogènes et les groupes méthyle,

$R^1$ représente le groupement $-B-CO-NR^3R^4$,

B représente les groupements $-CH_2-$, $-CH(CH_3)-$ ou $-CH_2CH_2-$,

$R^3$ et $R^4$ forment ensemble et avec l'atome d'azote auquel ils sont reliés les hétérocycles ou spirohétérocycles mono-, bi- ou tri-cycliques suivants :

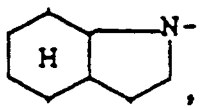
,
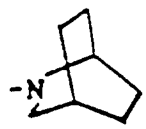
,
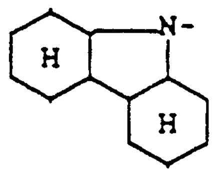
,

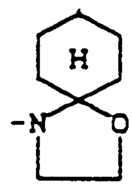
,
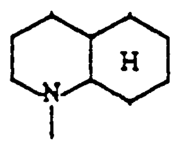
,

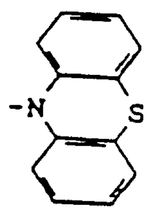
,
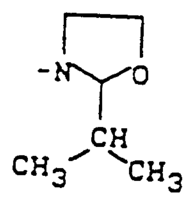
,
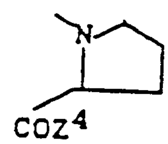

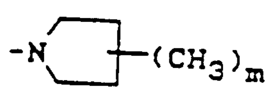
,
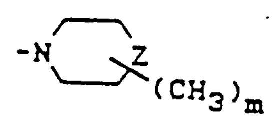

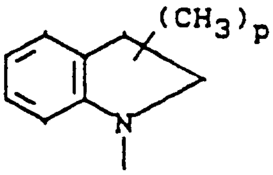 , 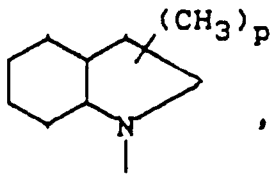 ,

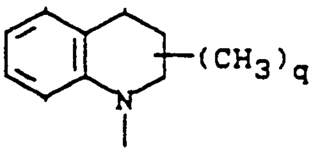 , 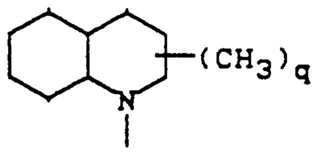 ,

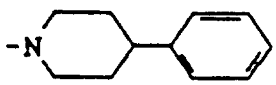 , 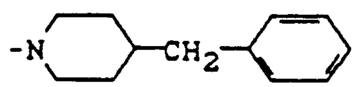 ,

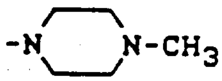 , 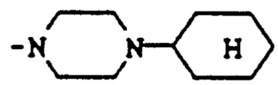 , 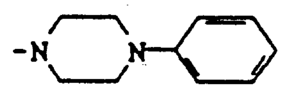 ,

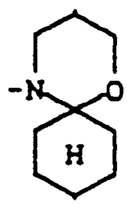 , 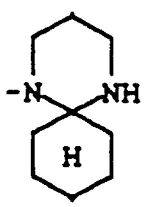 , 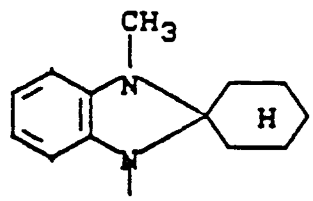 ,

dans lesquels

- Z représente l'oxygène ou le groupe $CH_2$,
- $Z^4$ représente un groupe hydroxy, méthoxy, éthoxy, amino, méthylamino, éthylamino, diméthylamino ou éthylméthylamino,
- m est égal à 1, 2, 3 ou 4,
- p est égal à 0, 1 ou 2,
- q est égal à 0, 1, 2 ou 3.

7. Procédé de préparation des dérivés de (2-cyano-2-oximino-acétyl)-aminoacides de formule générale (I) :

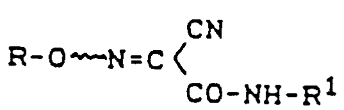 (I)

dans laquelle

- R représente un groupe alkyle, cyanalkyle, ou un groupe alcényle, alcynyle, cycloalkyle, cycloalkylalkyle ou phénylalkyle, chacun d'eux éventuellement substitué,
- $R^1$ représente le groupement $-B-CONR^3R^4$ dans lequel
- B représente une chaîne alkylène droite ou ramifiée, éventuellement substituée,
- $R^3$ représente un groupe alkyle substitué par les substituants suivants : les halogènes, les groupes cyano, $-COOR^I$, $-CONR^{II}R^{III}$, $-NR^{II}R^{III}$, $-OR^{IV}$, $-S(O)_nR^{IV}$, acyle, les groupes aryle, hétérocyclyle, cycloalkyle et cycloalcényle, chacun d'eux éventuellement substitué ; $R^3$ peut en outre représenter un groupe alcényle, alcynyle, cycloalkyle, cycloalcényle, aryle ou hétérocyclyle, chacun d'eux éventuellement substitué et

$R^4$ représente un groupe alkyle substitué par les substituants suivants : les halogènes, les groupes cyano, $-COOR^{I}$, $-CONR^{II}R^{III}$, $-NR^{II}R^{III}$, $-OR^{IV}$, $-S(O)_nR^{IV}$, acyle, des groupes aryle, hétérocyclyle, cycloalkyle et cycloalcényle, chacun d'eux éventuellement substitué ; $R^4$ peut en outre représenter un groupe alcényle, alcynyle, cycloalkyle, cycloalcényle, aryle, hétérocyclyle ou un carbocycle polycyclique, chacun d'eux éventuellement substitué ou le groupement $-OR^{IV}$,

ou bien

$R^3$ représente l'hydrogène ou un groupe alkyle et

$R^4$ représente un groupe alkyle substitué par les substituants suivants : les groupes acyle, hydroxy, alkylcarbonyloxy, $-S(O)_nR^{IV}$, les groupes hétérocyclyle, cycloalkyle et cycloalcényle, chacun d'eux éventuellement substitué ; $R^4$ peut en outre représenter un groupe cycloalcényle, hétérocyclyle ou un carbocycle polycyclique, chacun d'eux éventuellement substitué, un groupe cycloalkyle substitué sans toutefois que des halogènes et des groupes alkyle puissent être présents en tant qu'unique substituant, ou le groupement $OR^{IV}$,

ou bien

$R^3$et $R^4$ forment ensemble et avec l'atome d'azote auquel ils sont reliés un hétérocycle monocyclique substitué ou un hétérocycle ou spirohétérocycle bi- ou tri-cyclique éventuellement substitué contenant un à trois autres hétéroatomes identiques ou différents,

$R^{I}$ représente l'hydrogène ou un groupe alkyle éventuellement substitué par les substituants suivants : les halogènes, les groupes aryle, cycloalkyle et cycloalcényle, chacun d'eux éventuellement substitué,

$R^{II}$ et $R^{III}$ , ayant des significiations identiques ou différentes, représentent chacun l'hydrogène ou un groupe alkyle éventuellement substitué par les substituants suivants : les halogènes, les groupes $-COOR^{IV}$, $-CONR^{I}R^{IV}$, les groupes aryle, cycloaklyle et cycloalcényle, chacun d'eux éventuellement substitué ;

$R^{II}$ et $R^{III}$ peuvent en outre représenter chacun un groupe alcényle, alcynyle ou un groupe cycloalkyle, cycloalcényle, aryle ou hétérocyclyle, chacun d'eux éventuellement substitué, ou former ensemble et avec l'atome d'azote auquel ils sont reliés un hétérocycle éventuellement substitué qui peut contenir d'autres hétéroatomes,

$R^{IV}$ représente l'hydrogène, un groupe alkyle, aralkyle ou acyle

n est égal à 0, 1 ou 2,

de leurs isomères et mélanges d'isomères géométriques et optiques, caractérisé en ce que

a) on fait réagir des esters 2-cyano-2-oximino-acétiques de formule générale (II) :

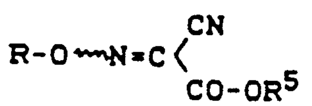

(II)

dans laquelle

R a les significations indiquées ci-dessus, et

$R^5$ représente un groupe alkyle,

avec des amines de formule (III) :

$R^1-NH_2$ (III)

dans laquelle

$R^1$ a les significations indiquées ci-dessus,

en présence d'un diluant et éventuellement en présence d'une base ;

ou bien

b) on fait réagir des dérivés à groupe carboxy activé d'acides carboxyliques de formule générale (IV) :

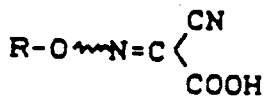

(IV)

dans laquelle

R a les significations indiquées ci-dessus,

avec des amines de formule (III) :

$R^1$-$NH_2$ (III)

dans laquelle

$R^1$ a les significations indiquées ci-dessus,

éventuellement en présence d'un catalyseur et éventuellement en présence d'un accepteur d'acide et le cas échéant en présence d'un diluant ; ou bien

c) on fait réagir des 2-cyano-2-oximino-acétamides de formule générale (V) :

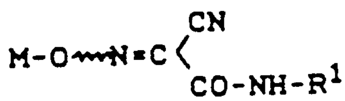

(V)

dans laquelle

M représente l'hydrogène, un ion de métal alcalin, un base tertiaire protonisée ou un ion ammonium quaternaire, et

$R^1$ a les significations indiquées ci-dessus,

avec les composés de formule (VI)

R-X (VI)

dans laquelle

X représente un halogène ou un groupe sulfonyloxy, et

R a les significations indiquées ci-dessus,

éventuellement en présence d'une base et éventuellement en présence d'un diluant ;

ou bien

d) on fait réagir des esters d'acétyl-aminoacides substitués de formule générale (VII) :

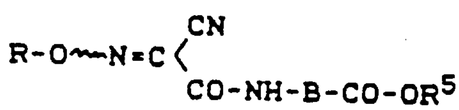

(VII)

dans laquelle B, R et $R^5$ ont les significations indiquées ci-dessus, avec des amines de formule (VIII) :

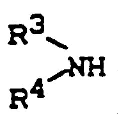

(VIII)

dans laquelle

$R^3$ et $R^4$ ont les significations indiquées ci-dessus,

éventuellement en présence d'un diluant et éventuellement en présence d'une base ; ou bien

e) on fait réagir des acétyl-aminoacides à groupe carboxy activé de formule générale (IX) :

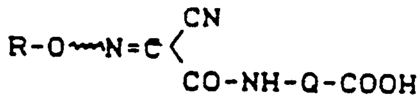 (IX)

dans laquelle

R a les significations indiquées ci-dessus, et

Q représente A ou B qui ont les significations indiquées ci-dessus,

avec des composés de formule (X) :

Y-H (X)

dans laquelle

Y représente les groupements $R^2O$- ou $R^3R^4N$-, $R^2$, $R^3$ et $R^4$ ayant les significations indiquées ci-dessus,

éventuellement en présence d'un catalyseur et éventuellement en présence d'un accepteur d'acide et éventuellement en présence d'un diluant ; ou bien

f) on fait réagir des sels d'ammonium d'acides 2-cyano-2-oximino-acétiques de formule générale (XI) :

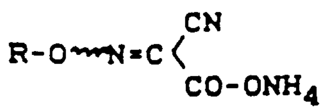 (XI)

dans laquelle

R a les significations indiquées ci-dessus,

avec des aldéhydes de formule (XII) :

$R^6$-CH=O (XII)

dans laquelle

le groupement $R^6$-CH= a les significations indiquées ci-dessus pour A ou B, et avec des isonitriles de formule (XIII) :

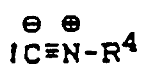 (XIII)

dans laquelle

$R^4$ a les significations indiquées ci-dessus,

éventuellement en présence d'un diluant ;

ou bien

g) on fait réagir des acides 2-cyano-2-oximino-acétiques de formule générale (IV) :

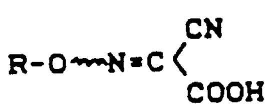 (IV)

dans laquelle

R a les significations indiquées ci-dessus,

avec des isocyanates de formule (XIV) :

$R^1$-NCO (XIV)

dans laquelle
$R^1$ a les significations indiquées ci-dessus,
éventuellement en présence d'un catalyseur et éventuellement en présence d'un diluant ;
ou bien
h) on fait réagir des 2-cyano-2-oximino-acétamides de formule générale (XV) :

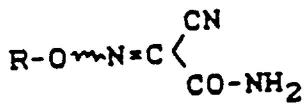

(XV)

dans laquelle
R a les significations indiquées ci-dessus,
avec une base éventuellement en présence d'un diluant puis on fait réagir les sels obtenus, directement ou après les avoir le cas échéant isolés, avec des composés de formule (XVI) :

$R^1$-X (XVI)

dans laquelle
$R^1$ et X ont les significations indiquées ci-dessus,
éventuellement en présence d'un diluant.

**8.** Produit parasiticide, caractérisé en ce qu'il contient au moins un dérivé de (2-cyano-2-oximino-acétyl)-aminoacide de formule (I) selon les revendications 1 et 7.

**9.** Utilisation des dérivés de (2-cyano-2-oximino-acétyl)-aminoacides de formule (I) selon les revendications 1 et 7 pour la lutte contre les parasites.

**10.** Procédé pour combattre les parasites, caractérisé en ce que l'on fait agir sur ces parasites et/ou leur habitat des dérivés de (2-cyano-2-oximino-acétyl)-aminoacides de formule (I) selon les revendications 1 et 7.

**11.** Procédé de préparation de produits parasiticides, caractérisé en ce que l'on mélange des dérivés de (2-cyano-2-oximino-acétyl)-aminoacides de formule (I) selon les revendications 1 et 7, avec des diluants et/ou des agents tensioactifs.

**Revendications pour l'Etat contractant suivant : ES**

**1.** Procédé de préparation des dérivés de (2-cyano-2-oximino-acétyl)-aminoacides de formule générale (I) :

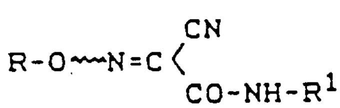

(I)

dans laquelle
R représente un groupe alkyle, cyanalkyle, ou un groupe alcényle, alcynyle, cycloalkyle, cycloalkylalkyle ou phénylalkyle, chacun d'eux éventuellement substitué,
$R^1$ représente le groupement $-B-CONR^3R^4$ dans lequel
B représente une chaîne alkylène droite ou ramifiée, éventuellement substituée,
$R^3$ représente un groupe alkyle substitué par les substituants suivants : les halogènes, les groupes cyano, $-COOR^I$, $-CONR^{II}R^{III}$, $-NR^{II}R^{III}$, $-OR^{IV}$, $-S(O)_nR^{IV}$, acyle, les groupes aryle,

hétérocyclyle, cycloalkyle et cycloalcényle, chacun d'eux éventuellement substitué ; $R^3$ peut en outre représenter un groupe alcényle, alcynyle, cycloalkyle, cycloalcényle, aryle ou hétérocyclyle, chacun d'eux éventuellement substitué et

$R^4$ représente un groupe alkyle substitué par les substituants suivants : les halogènes, les groupes cyano, $-COOR^I$, $-CONR^{II}R^{III}$, $-NR^{II}R^{III}$, $-OR^{IV}$, $-S(O)_nR^{IV}$, acyle, des groupes aryle, hétérocyclyle, cycloalkyle et cycloalcényle, chacun d'eux éventuellement substitué ; $R^4$ peut en outre représenter un groupe alcényle, alcynyle, cycloalkyle, cycloalcényle, aryle, hétérocyclyle ou un carbocycle polycyclique, chacun d'eux éventuellement substitué ou le groupement $-OR^{IV}$,

ou bien

$R^3$ représente l'hydrogène ou un groupe alkyle et

$R^4$ représente un groupe alkyle substitué par les substituants suivants : les groupes acyle, hydroxy, alkylcarbonyloxy, $-S(O)_nR^{IV}$, les groupes hétérocyclyle, cycloalkyle et cycloalcényle, chacun d'eux éventuellement substitué ; $R^4$ peut en outre représenter un groupe cycloalcényle, hétérocyclyle ou un carbocycle polycyclique, chacun d'eux éventuellement substitué, un groupe cycloalkyle substitué sans toutefois que des halogènes et des groupes alkyle puissent être présents en tant qu'unique substituant, ou le groupement $OR^{IV}$,

ou bien

$R^3$ et $R^4$ forment ensemble et avec l'atome d'azote auquel ils sont reliés un hétérocycle monocyclique substitué ou un hétérocycle ou spirohétérocycle bi- ou tri-cyclique éventuellement substitué contenant un à trois autres hétéroatomes identiques ou différents,

$R^I$ représente l'hydrogène ou un groupe alkyle éventuellement substitué par les substituants suivants : les halogènes, les groupes aryle, cycloalkyle et cycloalcényle, chacun d'eux éventuellement substitué,

$R^{II}$ et $R^{III}$, ayant des significiations identiques ou différentes, représentent chacun l'hydrogène ou un groupe alkyle éventuellement substitué par les substituants suivants : les halogènes, les groupes $-COOR^{IV}$, $-CONR^IR^{IV}$, les groupes aryle, cycloaklyle et cycloalcényle, chacun d'eux éventuellement substitué ; $R^{II}$ et $R^{III}$ peuvent en outre représenter chacun un groupe alcényle, alcynyle ou un groupe cycloalkyle, cycloalcényle, aryle ou hétérocyclyle, chacun d'eux éventuellement substitué, ou former ensemble et avec l'atome d'azote auquel ils sont reliés un hétérocycle éventuellement substitué qui peut contenir d'autres hétéroatomes,

$R^{IV}$ représente l'hydrogène, un groupe alkyle, aralkyle ou acyle

n est égal à 0, 1 ou 2,

de leurs isomères et mélanges d'isomères géométriques et optiques, caractérisé en ce que

a) on fait réagir des esters 2-cyano-2-oximino-acétiques de formule générale (II) :

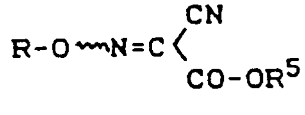

(II)

dans laquelle

R a les significations indiquées ci-dessus, et

$R^5$ représente un groupe alkyle,

avec des amines de formule (III) :

$R^1-NH_2$ (III)

dans laquelle $R^1$ a les significations indiquées ci-dessus, en présence d'un diluant et éventuellement en présence d'une base ;

ou bien

b) on fait réagir des dérivés à groupe carboxy activé d'acides carboxyliques de formule générale (IV) :

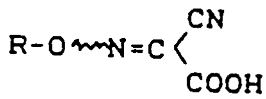 (IV)

dans laquelle
R a les significations indiquées ci-dessus,
avec des amines de formule (III) :

$R^1$-$NH_2$ (III)

dans laquelle
$R^1$ a les significations indiquées ci-dessus,
éventuellement en présence d'un catalyseur et éventuellement en présence d'un accepteur d'acide et le cas échéant en présence d'un diluant ; ou bien
c) on fait réagir des 2-cyano-2-oximino-acétamides de formule générale (V) :

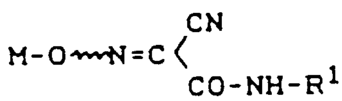 (V)

dans laquelle
M représente l'hydrogène, un ion de métal alcalin, un base tertiaire protonisée ou un ion ammonium quaternaire, et
$R^1$ a les significations indiquées ci-dessus,
avec les composés de formule (VI)

R-X (VI)

dans laquelle
X représente un halogène ou un groupe sulfonyloxy, et
R a les significations indiquées ci-dessus,
éventuellement en présence d'une base et éventuellement en présence d'un diluant ;
ou bien
d) on fait réagir des esters d'acétyl-aminoacides substitués de formule générale (VII) :

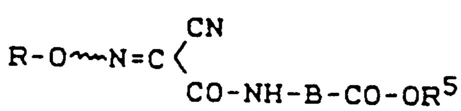 (VII)

dans laquelle B, R et $R^5$ ont les significations indiquées ci-dessus,
avec des amines de formule (VIII) :

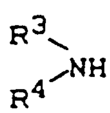 (VIII)

dans laquelle
$R^3$ et $R^4$ ont les significations indiquées ci-dessus,
éventuellement en présence d'un diluant et éventuellement en présence d'une base ; ou bien

e) on fait réagir des acétyl-aminoacides à groupe carboxy activé de formule générale (IX) :

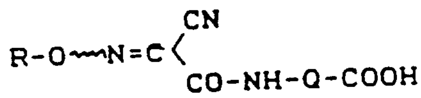

(IX)

dans laquelle

R a les significations indiquées ci-dessus, et

Q représente A ou a qui ont les significations indiquées ci-dessus,

avec des composés de formule (X) :

Y-H (X)

dans laquelle

Y représente les groupements $R^2O$- ou $R^3R^4N$-, $R^2$, $R^3$ et $R^4$ ayant les significations indiquées ci-dessus,

éventuellement en présence d'un catalyseur et éventuellement en présence d'un accepteur d'acide et éventuellement en présence d'un diluant ;

ou bien

f) on fait réagir des sels d'ammonium d'acides 2-cyano-2-oximino-acétiques de formule générale (XI) :

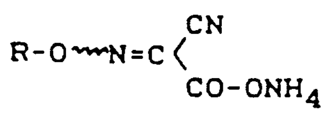

(XI)

dans laquelle

R a les significations indiquées ci-dessus,

avec des aldéhydes de formule (XII) :

$R^6$-CH = O (XII)

dans laquelle

le groupement $R^6$-CH = a les significations indiquées ci-dessus pour A ou B, et avec des isonitriles de formule (XIII) :

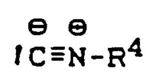

(XIII)

dans laquelle

$R^4$ a les significations indiquées ci-dessus,

éventuellement en présence d'un diluant ;

ou bien

g) on fait réagir des acides 2-cyano-2-oximino-acétiques de formule générale (IV) :

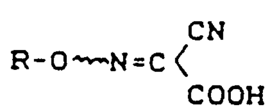

(IV)

dans laquelle

R a les significations indiquées ci-dessus,
avec des isocyanates de formule (XIV) :

$R^1$-NCO (XIV)

dans laquelle
$R^1$ a les significations indiquées ci-dessus,
éventuellement en présence d'un catalyseur et éventuellement en présence d'un diluant ;
ou bien
h) on fait réagir des 2-cyano-2-oximino-acétamides de formule générale (XV) :

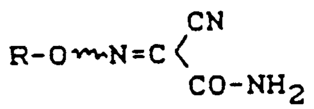

(XV)

dans laquelle
R a les significations indiquées ci-dessus,
avec une base éventuellement en présence d'un diluant puis on fait réagir les sels obtenus, directement ou après les avoir le cas échéant isolés, avec des composés de formule (XVI) :

$R^1$-X (XVI)

dans laquelle
$R^1$ et X ont les significations indiquées ci-dessus,
éventuellement en présence d'un diluant.

**2.** Procédé selon la revendication 1, pour lequel, dans la formule (I)

R représente un groupe alkyle à chaîne droite ou ramifiée en $C_1$-$C_4$, un groupe cyanalkyle à chaîne droite ou ramifiée contenant 1 à 4 atomes de carbone dans la partie alkyle, un groupe alcényle ou alcynyle, chacun à chaîne droite ou ramifiée et contenant chacun 2 à 6 atomes de carbone, un groupe cycloalkyle ou cycloalkylalkyle contenant chacun 3 à 6 atomes de carbone par partie cycloalkyle et 1 à 4 atomes de carbone par partie alkyle à chaîne droite ou ramifiée, chacun d'eux portant éventuellement 1 à 5 substituants identiques ou différents choisis parmi les groupes alkyle en $C_1$-$C_4$, ou un groupe benzyle ou phénéthyle portant chacun le cas échéant 1 à 5 substituants identiques ou différents, les substituants de chacune des parties phényle étant : des halogènes, des groupes alkyle et alcoxy contenant chacun 1 à 4 atomes de carbone, des groupes halogénoalkyle et halogénoalcoxy contenant chacun 1 ou 2 atomes de carbone et 2 à 5 atomes d'halogène identiques ou différents ;

$R^1$ représente les groupements -A-$COOR^2$ ou -B-$CONR^3R^4$,

B représente une chaîne alkylène droite ou ramifiée en $C_1$-$C_4$,

$R^3$ représente un groupe alkyle à chaîne droite ou ramifiée en $C_1$-$C_4$ portant 1 ou 2 substituants, les substituants étant : les halogènes, les groupes cyano, -$COOR^I$, -$CONR^{II}R^{III}$, -$NR^{II}R^{III}$, -$OR^{IV}$, -$S(O)_nR^{IV}$, acyle en $C_2$-$C_9$, les groupes phényle portant éventuellement 1 à 5 substituants identiques ou différents choisis parmi les halogènes, les groupes alkyle en $C_1$-$C_4$ ; les hétérocycles à 5 ou 6 chaînons et 1 à 3 hétéroatomes portant éventuellement 1 à 5 substituants identiques ou différents choisis parmi les halogènes et les groupes alkyle et alcoxy contenant chacun de 1 à 4 atomes de carbone, les groupes cycloalkyle en $C_3$-$C_6$ ou cycloalcényle en $C_5$-$C_7$ contenant chacun le cas échéant 1 à 5 substituants identiques ou différents choisis parmi les groupes alkyle en $C_1$-$C_4$ ; $R^3$ peut en outre représenter avantageusement un groupe alcényle ou alcynyle à chaîne droite ou ramifiée contenant chacun 2 à 6 atomes de carbone ; un groupe cycloalcényle en $C_5$-$C_7$ portant éventuellement 1 à 5 substituants identiques ou différents choisis parmi les groupes alkyle en $C_1$-$C_4$, ou un groupe cycloalkyle en $C_3$-$C_6$ portant éventuellement 1 à 5 substituants identiques ou différents, ces substituants étant : des halogènes, des groupes alkyle et alcoxy contenant chacun

1 à 4 atomes de carbone, des groupes cyano, amino, carbamoyle, alkylamino, dialkylamino, alkylcarbamoyle et dialkylcarbamoyle contenant chacun 1 à 4 atomes de carbone dans chacune des parties alkyle, alcoxycarbonylamino contenant 1 à 4 atomes de carbone dans chacune des parties alcoxy, cycloalkyle et cycloalkylalkyle contenant 5 ou 6 atomes de carbone dans la partie cycloalkyle et 1 à 4 atomes de carbone dans la partie alkyle à chaîne droite ou ramifiée, phényle et pyrrolidone portant éventuellement 1 à 5 substituants identiques ou différents choisis parmi les halogènes et les groupes alkyle et alcoxy en $C_1$-$C_4$ ; $R^3$ peut en outre représenter avantageusement un groupe phényle portant éventuellement 1 à 5 substituants identiques ou différents choisis parmi les halogènes, les groupes alkyle et alcoxy contenant chacun 1 à 4 atomes de carbone, les groupes halogénoalkyle et halogénoalcoxy contenant chacun 1 ou 2 atomes de carbone et 1 à 5 atomes d'halogènes identiques ou différents, ou un hétérocycle à 5 ou 6 chaînons et 1 à 3 hétéroatomes identiques ou différents portant éventuellement 1 à 5 substituants identiques ou différents, ces substituants étant : des halogènes, des groupes mercapto, phényle, des groupes alkyle, alcoxy, alkylthio, alkylsulfinyle, alkylsulfonyle contenant chacun 1 à 4 atomes de carbone à chaîne droite ou ramifiée par partie alkyle ;

$R^4$ a les significations de $R^3$, représente un carbocycle polycyclique ou le groupement $-OR^{IV}$,

ou bien

$R^3$ représente l'hydrogène ou un groupe alkyle à chaîne droite ou ramifiée en $C_1$-$C_4$,

$R^4$ représente un groupe alkyle à chaîne droite ou ramifiée en $C_1$-$C_4$ portant 1 ou 2 substituants choisis parmi les suivants : les groupes acyle en $C_2$-$C_9$, hydroxy, alkylcarbonyloxy contenant de 1 à 4 atomes de carbone dans la partie alkyle à chaîne droite ou ramifiée, $-S(O)_nR^{IV}$, les hétérocycles à 5 ou 6 chaînons et 1 à 3 hétéroatomes portant éventuellement 1 à 5 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ portant chacun le cas échéant 1 à 5 substituants identiques ou différents choisis parmi les groupes alkyle en $C_1$-$C_4$,

$R^4$ peut en outre représenter un groupe cycloalcényle en $C_5$-$C_7$ portant éventuellement 1 à 5 substituants identiques ou différents choisis parmi les groupes alkyle à chaîne droite ou ramifiée en $C_1$-$C_4$, un hétérocycle à 5 ou 6 chaînons et 1 à 3 hétéroatomes identiques ou différents portant éventuellement 1 à 5 substituants identiques ou différents choisis parmi les halogènes, les groupes mercapto, phényle, les groupes alkyle, alcoxy, alkylthio, alkylsulfinyle, alkylsulfonyle contenant 1 à 4 atomes de carbone par partie alkyle et chacun d'eux à chaîne droite ou ramifiée, un groupe cycloalkyle en $C_3$-$C_6$ portant 1 à 5 substituants identiques ou différents choisis parmi les groupes phényle, cyano, hydroxy, alcoxy, alkylamino, dialkylamino, alkylcarbamoyle, dialkylcarbamoyle, alcoxycarbonylamino contenant chacun 1 à 4 atomes de carbone par partie alkyle, cycloalkyle et cycloalkylalkyle contenant chacun 5 à 7 atomes de carbone dans la partie cycloalkyle et 1 à 4 atomes de carbone dans la partie alkyle, avec éventuellement des halogènes et des groupes alkyle à chaîne droite ou ramifiée en $C_1$-$C_4$ en tant qu'autres substituants du groupe cycloalkyle, ou un carbocycle polycyclique ou $-OR^{IV}$,

ou bien

$R^3$ et $R^4$ forment ensemble et avec l'atome d'azote auquel ils sont reliés un hétérocycle monocyclique portant 1 à 5 substituants identiques ou différents ou un hétérocycle ou spirohétérocycle bi- ou tri-cyclique portant éventuellement 1 à 5 substituants identiques ou différents, ces hétérocycles pouvant consister par exemple en cycles : oxazolidine, pyrrolidine, imidazolidine, pipéridine, pipérazine, morpholine, thiomorpholine, 1,3-oxazane ou 1,3-diazane ; ces hétérocycles pouvant tous être éventuellement condensés avec 1 ou 2 cycles benzène ou cyclohexane, ou être éventuellement reliés par des ponts méthylène ou éthylène.

En tant que substituants pour tous les systèmes hétérocycles, on citera : les groupes alkyle ou alcoxy à chaîne droite ou ramifiée contenant chacun 1 à 4 atomes de carbone, les groupes cycloalkyle en $C_3$-$C_6$, les groupes hydroxy ou oxo, les groupes alcényle à chaîne droite ou ramifiée en $C_2$-$C_4$, hydroxycarbonyle, alcoxycabonyle contenant 1 à 4 atomes de carbone dans la partie alkyle à chaîne droite ou ramifiée,

carbamoyle, alkyl- ou dialkylcarbamoyle contenant chacun 1 à 4 atomes de carbone dans chacune des parties alkyle, et les groupes phényle ou phénylalkyle contenant 1 à 4 atomes de carbone dans la partie alkyle à chaîne droite ou ramifiée et portant éventuellement 1 ou 2 substituants identiques ou différents choisis parmi les halogènes et les groupes alkyle et alcoxy à chaîne droite ou ramifiée contenant chacun 1 à 4 atomes de carbone,

$R^I$ représente l'hydrogène ou un groupe alkyle à chaîne droite ou ramifiée en $C_1$-$C_6$,

$R^{II}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du noyau phényle étant : des halogènes, des groupes alkyle et alcoxy contenant chacun 1 à 4 atomes de carbone, des groupes halogénoalkyle et halogénoalcoxy contenant chacun 1 ou 2 atomes de carbone et 2 à 5 atomes d'halogène identiques ou différents ; $R^{II}$ peut en outre représenter un groupe alcoxycarbonylalkyle, carbamoylalkyle, alkylcarbamoylalkyle ou dialkylcarbamoylalkyle contenant chacun 1 à 4 atomes de carbone dans chacune des parties alkyle, ou un groupe cycloalkyle en $C_3$-$C_6$ éventuellement substitué par des groupes alkyle à chaîne droite ou ramifiée en $C_1$-$C_4$,

$R^{III}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du groupe phényle étant ceux qui ont été mentionnés en référence à $R^{II}$ ; $R^{III}$ peut en outre représenter un groupe alcoxycarbonylalkyle, carbamoylalkyle, alkylcarbamoylalkyle ou dialkylcarbamoylalkyle contenant chacun 1 à 4 atomes de carbone dans chacune des parties alkyle ou un groupe cycloalkyle en $C_3$-$C_6$ éventuellement substitué par des groupes alkyle à chaîne droite ou ramifiée en $C_1$-$C_4$ ;

$R^{IV}$ représente l'hydrogène, un groupe alkyle à chaîne droite ou ramifiée en $C_1$-$C_6$, un groupe phénylalkyle contenant 1 à 4 atomes de carbone dans la partie alkyle à chaîne droite ou ramifiée et portant éventuellement 1 à 5 substituants identiques ou différents, les substituants du noyau phényle étant de préférence ceux qui ont été mentionnés en référence à $R^{II}$ ; $R^{IV}$ peut en outre représenter un groupe acyle en $C_2$-$C_9$, et

n est égal à 0, 1 ou 2,

leurs isomères et mélanges d'isomères géométriques et optiques.

3. Procédé selon la revendication 1, pour lequel, dans la formule (I) :

R représente un groupe alkyle en $C_1$ ou $C_2$, un groupe cyanométhyle ou cyanéthyle, un groupe allyle ou propargyle, un groupe cyclopropyle, cyclohexyle, cyclopropylméthyle, ou cyclohexylméthyle, portant éventuellement chacun 1 à 3 substituants méthyle, ou un groupe benzyle ou phénéthyle portant chacun, le cas échéant, 1 à 3 substituants identiques ou différents choisis parmi les halogènes et les groupes méthyle,

$R^1$ représente le groupement $-B-CONR^3R^4$,

B représente les groupements $-CH_2$, $-CH(CH_3)-$ ou $-CH_2CH_2-$,

$R^3$ représente un groupe alkyle à chaîne droite ou ramifiée en $C_1$-$C_4$ portant un ou deux substituants, ces substituants consistant de préférence en : des halogènes, des groupes cyano, $-COOR^I$, $-CONR^{II}R^{III}$, $NR^{II}R^{III}$, $-OR^{IV}$, $-S(O)_nR^{IV}$, les groupes acyle en $C_2$-$C_9$, les groupes phényle portant éventuellement 1 à 3 substituants identiques ou différents choisis parmi les halogènes et les groupes alkyle en $C_1$-$C_4$, les hétérocycles à 5 ou 6 chaînons et 1 à 3 hétéroatomes identiques ou différents portant éventuellement 1 à 3 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ chacun éventuellement substitué par des groupes alkyle à chaîne droite ou ramifiée en $C_1$-$C_4$ ; $R^3$ peut en outre représenter un groupe alcényle ou alcynyle à chaîne droite ou ramifiée contenant chacun 2 à 6 atomes de carbone, un groupe cycloalcényle en $C_5$-$C_7$ éventuellement substitué par des groupes alkyle à chaîne droite ou ramifiée en $C_1$-$C_4$, un groupe cycloalkyle en $C_3$-$C_6$ éventuellement substitué, les substituants étant de préférence : les halogènes, des groupes alkyle et alcoxy à chaîne droite ou ramifiée contenant chacun 1 à 4 atomes de carbone, des groupes cyano, amino, carbamoyle, alkylamino,

dialkylamino, alkylcarbamoyle et dialkylcarbamoyle contenant chacun 1 à 4 atomes de carbone dans chacune des parties alkyle, alcoxycarbonylamino contenant 1 à 4 atomes de carbone dans la partie alcoxy à chaîne droite ou ramifiée, cycloalkyle et cycloalkylalkyle contenant chacun 5 ou 6 atomes de carbone dans la partie cycloalkyle et 1 à 4 atomes de carbone dans la partie alkyle à chaîne droite ou ramifiée, les groupes phényle et pyrrolidone portant éventuellement 1 à 3 substituants identiques ou différents choisis parmi les halogènes et les groupes alkyle en $C_1$-$C_4$ ; $R^3$ peut en outre représenter un groupe phényle portant éventuellement 1 à 3 substituants identiques ou différents choisis parmi les halogènes, les groupes alkyle ou alcoxy à chaîne droite ou ramifiée contenant chacun 1 à 4 atomes de carbone, les groupes halogénoalkyle ou halogénoalcoxy contenant chacun 1 ou 2 atomes de carbone et 1 à 5 atomes d'halogènes identiques ou différents, ou un hétérocycle à 5 ou 6 chaînons et 1 à 3 hétéroatomes identiques ou différents, portant éventuellement 1 à 3 substituants identiques ou différents, consistant en : des halogènes, des groupes mercapto, phényle, des groupes alkyle, alcoxy alkylthio, alkylsulfinyle et alkylsulfonyle, à chaîne droite ou ramifiée contenant chacun 1 à 4 atomes de carbone par partie alkyle ;

$R^4$ a les significations de $R^3$, représente un carbocycle polycyclique ou le groupement $-OR^{IV}$,

ou bien

$R^3$ représente l'hydrogène ou un groupe alkyle à chaîne droite ou ramifiée en $C_1$-$C_4$,

et

$R^4$ représente un groupe alkyle en $C_1$-$C_4$ à chaîne droite ou ramifiée portant 1 ou 2 substituants pris parmi les suivants : les groupes acyle en $C_2$-$C_9$, hydroxy, alkylcarbonyloxy contenant 1 à 4 atomes de carbone dans la partie alkyle à chaîne droite ou ramifiée, $-S(O)_nR^{IV}$, les hétérocycles à 5 ou 6 chaînons et 1 à 3 hétéroatomes portant éventuellement 1 à 3 substituants identiques ou différents choisis parmi les halogènes, les groupes alkyle et alcoxy à chaîne droite ou ramifiée contenant chacun 1 à 4 atomes de carbone, les groupes cycloalkyle en $C_3$-$C_6$ et cycloalcényle en $C_5$-$C_7$ portant chacun le cas échéant 1 à 3 substituants identiques ou différents choisis parmi les groupes alkyle en $C_1$-$C_4$ ;

$R^4$ peut en outre représenter un groupe cycloalcényle en $C_5$-$C_7$ portant éventuellement 1 à 3 substituants identiques ou différents choisis parmi les groupes alkyle à chaîne droite ou ramifiée en $C_1$-$C_4$, un hétérocycle à 5 ou 6 chaînons et 1 à 3 hétéroatomes identiques ou différents, portant éventuellement 1 à 3 substituants identiques ou différents choisis parmi les halogènes, les groupes mercapto, phényle, des groupes alkyle, alcoxy, alkylthio, alkylsulfinyle et alkylsulfonyle à chaîne droite ou ramifiée contenant chacun 1 à 4 atomes de carbone par partie alkyle, un groupe cycloalkyle en $C_3$-$C_6$ portant 1 à 3 substituants identiques ou différents choisis parmi les groupes phényle, cyano, hydroxy, alcoxy, alkylamino, dialkylamino, alkylcarbamoyle, dialkylcarbamoyle, alcoxycarbonylamino contenant chacun 1 à 4 atomes de carbone par partie alkyle, cycloalkyle et cycloalkylalkyle contenant chacun 5 à 7 atomes de carbone dans la partie cycloalkyle et 1 à 4 atomes de carbone dans la partie alkyle, avec le cas échéant des halogènes et des groupes alkyles à chaîne droite ou ramifiée en $C_1$-$C_4$ en tant qu'autres substituants du groupe cycloalkyle, ou bien un carbocycle polycyclique ou un groupe $-OR^{IV}$,

ou bien

$R^3$ et $R^4$ forment ensemble et avec l'atome d'azote auquel ils sont reliés un hétérocycle monocyclique portant 1 à 3 substituants identiques ou différents ou un hétérocycle ou spirohétérocycle bi- ou tri-cyclique portant éventuellement 1 à 3 substituants identiques ou différents, les hétérocycles consistant en cycles : oxazolidine, pyrrolidine, imidazolidine, pipéridine, pipérazine, morpholine, thiomorpholine, 1,3-oxazane ou 1,3-diazane; ces hétérocycles pouvant tous être le cas échéant condensés avec 1 ou 2 cycles benzène ou cyclohexane, ou reliés éventuellement par des ponts méthylène ou éthylène.

En tant que substituants pour tous les systèmes hétérocycliques, on citera : des groupes alkyle ou alcoxy à chaîne droite ou ramifiée contenant chacun 1 à 4 atomes de carbone, des groupes cycloalkyle en $C_3$-$C_6$, des groupes hydroxy ou oxo, des groupes alcényle à chaîne droite ou ramifiée en $C_2$-$C_4$, hydroxycarbonyle, alcoxycarbonyle

contenant 1 à 4 atomes de carbone dans la partie alkyle à chaîne droite ou ramifiée, carbamoyle, alkyl- ou dialkyl-carbamoyle contenant 1 à 4 atomes de carbone dans chaque partie alkyle, et les groupes phényle ou phénylalkyle contenant 1 à 4 atomes de carbone dans la partie alkyle à chaîne droite ou ramifiée et portant chacun le cas échéant 1 ou 2 substituants identiques ou différents choisis parmi les halogènes et les groupes alkyle et alcoxy à chaîne droite ou ramifiée contenant chacun 1 à 4 atomes de carbone,

$R^I$ représente l'hydrogène ou un groupe alkyle à chaîne droite ou ramifiée en $C_1$-$C_4$,

$R^{II}$ représente l'hydrogène, un groupe alkyle à chaîne droite ou ramifiée en $C_1$-$C_4$, un groupe benzyle ou phénéthyle portant chacun le cas échéant 1 à 3 substituants identiques ou différents, les substituants du noyau phényle consistant en : le fluor, le chlore, les groupes méthyle, méthoxy et trifluorométhyle ; $R^{II}$ peut en outre représenter un groupe alcoxycarbonylaklyle contenant 1 à 4 atomes de carbone dans la partie alcoxy et 1 ou 2 atomes de carbone dans la partie alkyle, un groupe carbamoylalkyle contenant 1 ou 2 atomes de carbone dans la partie alkyle, un groupe alkylcarbamoylalkyle et dialkylcarbamoylalkyle contenant chacun 1 ou 2 atomes de carbone dans chaque partie aklyle, ou un groupe cycloalkyle en $C_3$-$C_6$ portant éventuellement 1 à 3 substituants identiques ou différents choisis parmi les groupes méthyle et éthyle,

$R^{III}$ a les significations de $R^{II}$,

$R^{IV}$ représente l'hydrogène, un groupe alkyle à chaîne droite ou ramifiée en $C_1$-$C_4$, un groupe benzyle ou phénéthyle portant chacun éventuellement 1 à 3 substituants identiques ou différents, les substituants du noyau phényle étant : le fluor, le chlore, les groupes méthyle, méthoxy et trifluorométhyle ; $R^{IV}$ peut en outre représenter un groupe acyle en $C_2$-$C_9$, et

n est égal à 0, 1 ou 2,

leurs isomères et mélanges d'isomères géométriques et optiques.

**4.** Procédé selon la revendication 1, pour lequel, dans la formule (I) :

R représente un groupe méthyle, éthyle, cyanométhyle, cyanéthyle, allyle, propargyle, un groupe cyclopropyle, cyclohexyle, cyclopropylméthyle ou cyclohexylméthyle portant chacun 1 à 3 substituants méthyle, ou un groupe benzyle ou phénéthyle portant chacun le cas échéant 1 à 3 substituants identiques ou différents choisis parmi les halogènes et les groupes méthyle,

$R^1$ représente le groupement $-B-CO-NR^3R^4$ dans lequel

B représente les groupements $-CH_2-$, $-CH(CH_3)-$ ou $-CH_2CH_2-$,

$R^3$ représente un groupe alkyle en $C_1$-$C_3$ portant 1 ou 2 substituants consistant en le chlore, des groupes hydroxy, alkylcarbonyloxy contenant 1 à 4 atomes de carbone dans la partie alkyle à chaîne droite ou ramifiée, alkylcarbonyle contenant 1 à 4 atomes de carbone dans la partie alkyle à chaîne droite ou ramifiée, alcoxy ou alkylthio contenant 1 ou 2 atomes de carbone, phényle, 2-furyle, 2-pyridyle, 1-morpholino, cyclopropyle et cyclohexyle portant chacun éventuellement 1 à 3 substituants identiques ou différents choisis parmi le chlore et les groupes méthyle ; $R^3$ peut en outre représenter de manière particulièrement avantageuse un groupe allyle ou propargyle, un groupe 1-cyclohexényle ou un groupe cyclohexyle portant 1 à 3 substituants identiques ou différents, les substituants consistant en : le chlore, les groupes méthyle, éthyle, méthoxy, cyano, amino, alkyl- et dialkylamino contenant chacun 1 ou 2 atomes de carbone dans chaque partie alkyle, carbamoyle, alkyl- et dialkylcarbamoyle contenant chacun 1 ou 2 atomes de carbone dans chaque partie alkyle, cyclohexyle, cyclohexylalkyle contenant chacun 1 ou 2 atomes de carbone dans la partie alkyle et 1-pyrrolidine-2-one ;

$R^3$ peut en outre représenter un groupe phényle portant éventuellement 1 à 3 substituants identiques ou différents choisis parmi le chlore et les groupes méthyle, un groupe 2-pyridyle ou 2-pyrimidinyle portant chacun éventuellement 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadiazole-5-yle éventuellement substitué par un groupe phényle, un groupe 1,3,4-thiadiazole-5-yle portant éventuellement 1 à 3 substituants identiques ou différents choisis parmi les groupes mercapto, les groupes alkyle à chaîne droite ou ramifiée en $C_1$-$C_4$, les groupes alkylthio, alkylsulfiny-

le et alkylsulfonyle à chaîne droite ou ramifiée contenant chacun 1 à 4 atomes de carbone, ou le groupement :

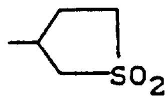

$R^4$ représente un groupe alkyle en $C_1$-$C_3$ portant 1 ou 2 substituants consistant en : le chlore, les groupes hydroxy, alkylcarbonyloxy contenant chacun 1 à 4 atomes de carbone dans la partie alkyle à chaîne droite ou ramifiée, alkylcarbonyle contenant 1 à 4 atomes de carbone dans la partie alkyle à chaîne droite ou ramifiée, alcoxy et alkylthio contenant chacun 1 ou 2 atomes de carbone, les groupes phényle, 2-furyle, 2-pyridyle, 1-morpholino, cyclopropyle et cyclohexyle portant le cas échéant 1 ou 3 substituants identiques ou différents choisis parmi le chlore et les groupes méthyle ; $R^4$ peut en outre représenter de manière particulièrement avantageuse un groupe allyle ou propargyle, un groupe 1-cyclohexényle ou un groupe cyclohexyle portant 1 à 3 substituants identiques ou différents consistant en : le chlore, des groupes méthyle, éthyle, méthoxy, cyano, amino, alkyl- ou dialkylamino contenant chacun 1 ou 2 atomes de carbone dans chaque partie alkyle, les groupes carbamoyle, alkyl- et dialkylcarbamoyle contenant chacun 1 ou 2 atomes de carbone dans la partie alkyle, les groupes cyclohexyle, cyclohexylalkyle contenant 1 ou 2 atomes de carbone dans la partie alkyle, et 1-pyrrolidine-2-one ;

$R^4$ peut en outre représenter un groupe phényle portant éventuellement 1 à 3 substituants identiques ou différents choisis parmi le chlore et les groupes méthyle, un groupe 2-pyridyle ou 2-pyrimidinyle portant chacun le cas échéant 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadiazole-5-yle éventuellement substitué par un groupe phényle, un groupe 1,3,4-thiadiazole-5-yle portant éventuellement 1 à 3 substituants identiques ou différents choisis parmi les groupes mercapto, les groupes alkyle à chaîne droite ou ramifiée en $C_1$-$C_4$, les groupes alkylthio, les groupes alkylsulfinyle ou alkylsulfonyle à chaîne droite ou ramifiée contenant 1 à 4 atomes de carbone, ou le groupement

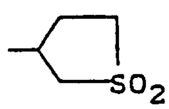

$R^4$ peut en outre représenter un groupe hydroxy, alcoxy en $C_1$-$C_2$, un groupe benzyloxy portant éventuellement 1 à 3 substituants identiques ou différents choisis parmi le chlore et les groupes méthyle, un groupe 1-adamantyle, 2-norbornyle, 1- ou 2-décalyle ou 1- ou 2-tétralyle.

**5.** Procédé selon la revendication 1, pour lequel, dans la formule (I) :

R représente un groupe méthyle, éthyle, cyanométhyle, cyanéthyle, allyle, propargyle, un groupe cylcopropyle, cyclohexyle, cyclopropylméthyle ou cyclohexylméthyle portant chacun 1 à 3 substituants méthyle, ou un groupe benzyle ou phénéthyle, portant chacun 1 ou 2 substituants identiques ou différents choisis parmi les halogènes et les groupes méthyle,

$R^1$ représente le groupement $-B-CO-NR^3R^4$,

B représente les groupements $-CH_2-$, $-CH(CH_3)-$ ou $-CH_2CH_2-$,

$R^3$ représente l'hydrogène ou un groupe alkyle à chaîne droite ou ramifiée en $C_1$-$C_4$,

$R^4$ représente un groupe aklyle substitué en $C_1$-$C_3$, les substituants consistant en : des groupes hydroxy, alkylcarbonyloxy contenant 1 à 4 atomes de carbone dans la partie alkyle à chaîne droite ou ramifiée, des groupes alkylcarbonyle contenant 1 à 4 atomes de carbone dans la partie alkyle, alkylthio en $C_1$ ou $C_2$, 2-furyle, 2-pyridyle, 1-morpholino, cyclopropyle et

cyclohexyle ; $R^4$ peut en outre représenter un groupe 1-cyclohexényle, un groupe 2-pyridyle ou 2-pyrimidinyle, portant chacun le cas échéant 1 à 3 substituants méthyle, un groupe 2-thiazolyle éventuellement substitué par un groupe phényle, un groupe 2-benzothiazolyle portant éventuellement 1 à 3 substituants identiques ou différents choisis parmi le chlore, les groupes méthyle et méthoxy, un groupe 1,2,4-triazole-3-yle, un groupe 1,2,4-thiadiazole-5-yle éventuellement substitué par un groupe phényle, un groupe 1,3,4-thiadiazole-5-yle portant éventuellement 1 à 3 substituants identiques ou différents choisis parmi les groupes mercapto, et les groupes alkyle, alkylthio, alkylsulfinyle et alkylsulfonyle contenant chacun 1 à 4 atomes de carbone, le groupement

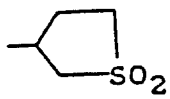 ,

un groupe 1-adamantyle, 2-norbornyle, 1- ou 2-décalyle ou 1- ou 2-tétralyle ; $R^4$ peut en outre représenter un groupe cyclohexyle portant 1 à 3 substituants identiques ou différents, les substituants consistant en : des groupes phényle, cyano, hydroxy, alcoxy en $C_1$ ou $C_2$, alkylamino et dialkylamino contenant chacun 1 ou 2 atomes de carbone dans chaque partie alkyle, alkylcarbamoyle et dialkylcarbamoyle contenant chacun 1 ou 2 atomes de carbone dans chaque partie alkyle, alcoxycarbonylamino contenant 1 ou 2 atomes de carbone dans la partie alkyle, cyclohexyle, cyclohexylalkyle contenant 1 ou 2 atomes de carbone dans la partie alkyle, et 1-pyrrolidine-2-one, et en outre les halogènes ou les groupes alkyle en $C_1$ ou $C_2$.

**6.** Procédé selon la revendication 1, pour lequel, dans la formule (I) :

| | |
|---|---|
| R | représente un groupe méthyle, éthyle, cyanométhyle, cyanéthyle, allyle, propargyle, un groupe cyclopropyle, cyclohexyle, cyclopropylméthyle ou cyclohexylméthyle portant chacun 1 à 3 substituants méthyle, ou un groupe benzyle ou phénéthyle portant chacun le cas échéant 1 à 3 substituants identiques ou différents choisis parmi les halogènes et les groupes méthyle, |
| $R^1$ | représente le groupement $-B-CO-NR^3R^4$, |
| B | représente les groupements $-CH_2-$, $-CH(CH_3)-$ ou $-CH_2CH_2-$, |
| $R^3$ et $R^4$ | forment ensemble et avec l'atome d'azote auquel ils sont reliés les hétérocycles ou spirohétérocycles mono-, bi- ou tri-cycliques suivants : |

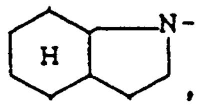 , 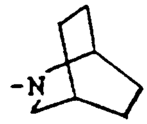 , 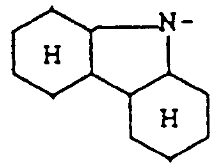 ,

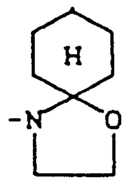 , 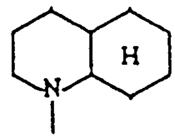 ,

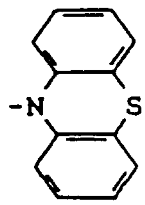 , 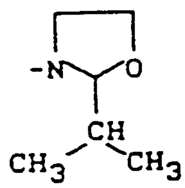 , 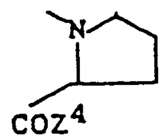

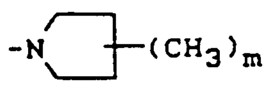 , 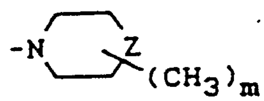

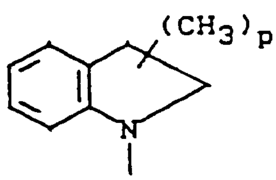 , 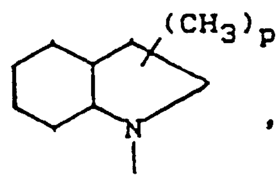 ,

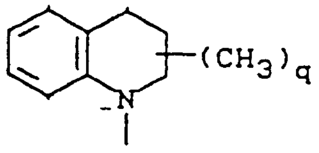 , 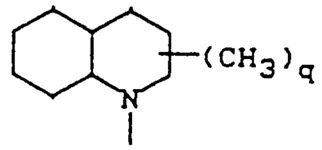 ,

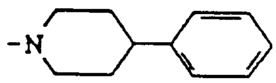 , 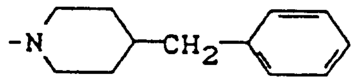 ,

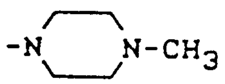 , 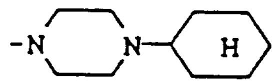 , 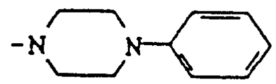 ,

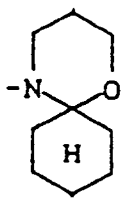 , 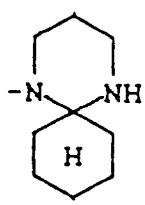 , 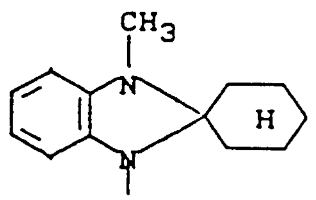 ,

dans lesquels

Z représente l'oxygène ou le groupe $CH_2$,

$Z^4$ représente un groupe hydroxy, méthoxy, éthoxy, amino, méthylamino, éthylamino, diméthylamino ou éthylméthylamino,

m est égal à 1, 2, 3 ou 4,

p est égal à 0, 1 ou 2,

q est égal à 0, 1, 2 ou 3.

**7.** Produit parasiticide, caractérisé en ce qu'il contient au moins un dérivé de (2-cyano-2-oximino-acétyl)-aminoacide de formule (I) selon les revendications 1 et 6.

**8.** Utilisation des dérivés de (2-cyano-2-oximino-acétyl)-aminoacides de formule (I) selon les revendications 1 et 6 pour la lutte contre les parasites.

**9.** Procédé pour combattre les parasites, caractérisé en ce que l'on fait agir sur ces parasites et/ou leur habitat des dérivés de (2-cyano-2-oximino-acétyl)-aminoacides de formule (I) selon les revendications 1 et 6.

**10.** Procédé de préparation de produits parasiticides, caractérisé en ce que l'on mélange des dérivés de (2-cyano-2-oximino-acétyl)-aminoacides de formule (I) selon les revendications 1 et 6, avec des diluants et/ou des agents tensioactifs.